# EUROPEAN PATENT APPLICATION

(11) **EP 2 336 215 A1**
(43) Date of publication of application: **22.06.2011**
(21) Application number: 09819094.5
(22) Date of filing: 25.09.2009
(51) Int. Cl.: C08G 61/00, C07D 251/24, C08L 65/00, C09K 11/06, H01L 51/50

(54) **POLYMER COMPOUND CONTAINING NITROGEN-CONTAINING HETEROCYCLIC STRUCTURE, AND COMPOSITION, SOLUTION, THIN FILM AND POLYMER LIGHT-EMITTING ELEMENT EACH CONTAINING SAME**

(30) Priority: 06.10.2008 JP 2008259473
(71) Applicant: Sumitomo Chemical Company, Limited, Tokyo 104-8260 (JP); Sumation Co., Ltd., Chuo-ku Tokyo 104-8260 (JP)
(72) Inventor: ISHII Yusuke, Toda-shi Saitama 335-0023 (JP); KOBAYASHI Shigeya, Tsukuba-shi Ibaraki 305-0005 (JP); ANRYU Makoto, Tsukuba-shi Ibaraki 305-0821 (JP); MIKAMI Satoshi, Takarazuka-shi Hyogo 665-0871 (JP)
(74) Representative: Duckworth, Timothy John
(86) International application number: PCT/JP2009/066645
(87) International publication number: WO 2010/041559

(57) **Abstract**

A polymer compound having a repeating unit represented by formula (1-0): 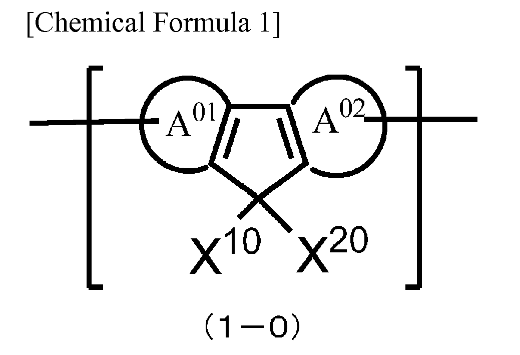 wherein in formula (1-0), ring A⁰¹ and ring A⁰² are the same or different and each represents an aromatic hydrocarbon ring that is optionally substituted; and X¹⁰ and X²⁰ are the same or different and each represents a hydrogen atom or a substituent, provided that at least one of X¹⁰ and X²⁰ is a group represented by formula (2-0): 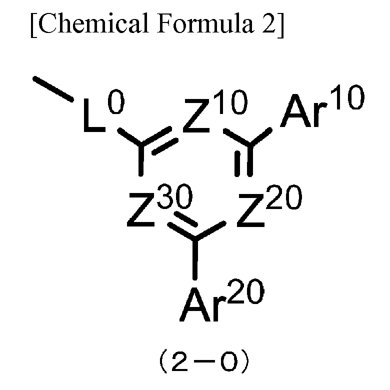 wherein in formula (2-0), Z¹⁰, Z²⁰ and Z³⁰ are the same or different and each represents -N= or -CH=, provided that at least two of Z¹⁰, Z²⁰ and Z³⁰ are -N=; L⁰ represents an arylene group or a single bond; and Ar¹⁰ and Ar²⁰ are the same or different and each represents an aryl group, provided that the total carbon number of the groups represented by Ar¹⁰, Ar²⁰ and L⁰ is 24 or more.

## Description

### Technical Field

The present invention relates to a polymer compound containing a nitrogen-containing heterocyclic structure, and a composition, a solution, a thin film and a polymer light-emitting device, each containing the same.

### Background Art

An organic electroluminescent device comprises an organic layer such as a light-emitting layer or a charge transport layer between a pair of electrodes, and an organic electroluminescent display using this has recently been attracting attention as the next generation display. Especially, when a polymer compound soluble in a solvent is used for an organic layer, the organic layer can be produced using an application method, and therefore, it is more advantageous when the area of an organic electroluminescent display is increased, than a case where the organic layer is produced using a vacuum evaporation method using a low-molecular compound. Therefore, various polymer compounds for use in the above organic layers have been developed, and a polymer compound having a fluorenediyl group in which two alkyl chains are bound to the carbon atom at position 9 of the fluorene is proposed as the polymer compound (Non Patent Literature 1).

### Citation List

### Non Patent Literature

Non Patent Literature 1: Advanced Materials, Vol. 12 (2000), pp. 362-365

### Summary of Invention

### Technical Problem

However, an organic electroluminescent device produced using the above polymer compound has not had a sufficiently low emission starting voltage.

Therefore, an object of the present invention is to provide a polymer compound having a low emission starting voltage when used for an organic electroluminescent device.

### Solution to Problem

More specifically, the present invention provides, in a first aspect, a polymer compound having a repeating unit represented by formula (1-0).

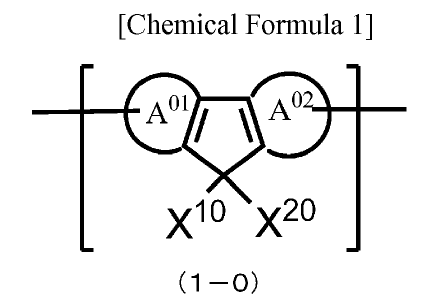

(In formula (1-0), ring A⁰¹ and ring A⁰² are the same or different and each represents an aromatic hydrocarbon ring that is optionally substituted; and X¹⁰ and X²⁰ are the same or different and each represents a hydrogen atom or a substituent, provided that at least one of X¹⁰ and X²⁰ is a group represented by formula (2-0).)

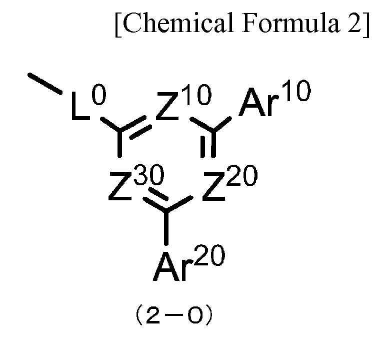

(In formula (2-0), Z¹⁰, Z²⁰ and Z³⁰ are the same or different and each represents -N= or -CH=, provided that at least two of Z¹⁰, Z²⁰ and Z³⁰ are -N=; L⁰ represents an arylene group or a single bond; and Ar¹⁰ and Ar²⁰ are the same or different and each represents an aryl group, provided that the total carbon number of the groups represented by Ar¹⁰, Ar²⁰ and L⁰ is 24 or more.)

The present invention provides, in a second aspect, the above polymer compound, wherein the repeating unit represented by formula (1-0) is a repeating unit represented by formula (1).

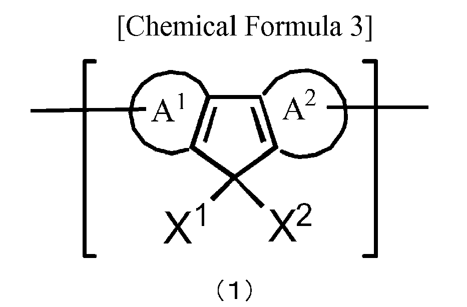

(In formula (1), ring A¹ and ring A² are the same or different and each represents an aromatic hydrocarbon ring that is optionally substituted; and X¹ and X² are the same or different and each represents a hydrogen atom or a substituent, provided that at least one of X¹ and X² is a group represented by formula (2).)

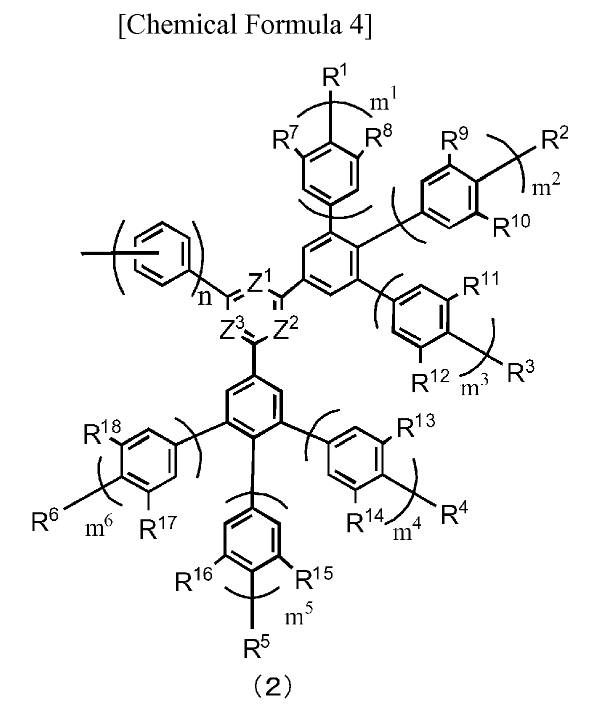

(In formula (2), Z¹, Z² and Z³ are the same or different and each represents -N= or -CH=, provided that at least two of Z¹, Z² and Z³ are -N=; n represents an integer of 0 or more; m¹ to m⁶ are the same or different and each represents an integer of 0 or more, provided that m¹ + m² + m³ ≥ 1 and m⁴ + m⁵ + m⁶ ≥ 1; R¹ to R⁶ are the same or different and each represents a hydrogen atom, a halogeno group, an alkyl group, an alkenyl group, an alkynyl group, an alkoxy group, an alkylthio group, an alkylsilyl group, or a group represented by formula (3); R⁷ to R¹⁸ are the same or different and each represents a hydrogen atom, a halogeno group, an alkyl group, an alkenyl group, an alkynyl group, an alkoxy group, an alkylthio group, an aryl group, an alkylsilyl group, or a group represented by formula (3); and when a plurality of each R⁷ to R¹⁸ exist, they may be the same or different.)

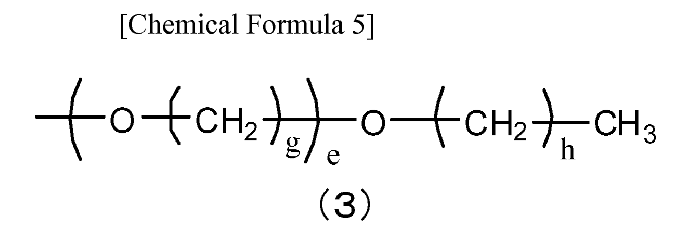

(In formula (3), e represents an integer of 1 to 6, g represents an integer of 1 to 6, and h represents an integer of 0 to 5; and when a plurality of g exist, they may be the same or different.)

The present invention provides, in a third aspect, a compound represented by formula (6).

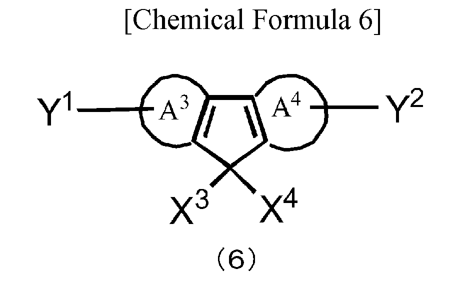

(In formula (6), ring A³ and ring A⁴ are the same or different and each represents an aromatic hydrocarbon ring that is optionally substituted; Y¹ and Y² are the same or different and each represents a hydrogen atom or a polymerizable reactive group; and X³ and X⁴ are the same or different and each represents a hydrogen atom or a substituent, provided that at least one of X³ and X⁴ is a group represented by formula (7).)

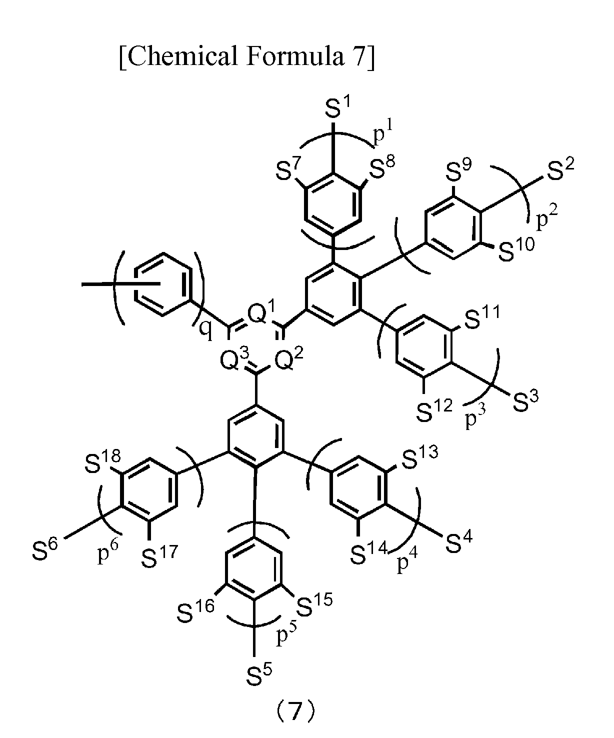

(In formula (7), Q¹, Q² and Q³ are the same or different and each represents -N= or -CH=, provided that at least two of Q¹, Q² and Q³ are -N=; q represents an integer of 0 or more; p¹ to p⁶ are the same or different and each represents an integer of 0 or more, provided that p¹ + p² + p³ ≥ 1 and p⁴ + p⁵ + p⁶ ≥ 1; S¹ to S⁶ are the same or different and each represents a hydrogen atom, a halogeno group, an alkyl group, an alkenyl group, an alkynyl group, an alkoxy group, an alkylthio group, an alkylsilyl group, or a group represented by formula (8); S⁷ to S¹⁸ are the same or different and each represents a hydrogen atom, a halogeno group, an alkyl group, an alkenyl group, an alkynyl group, an alkoxy group, an alkylthio group, an aryl group, an alkylsilyl group, or a group represented by formula (8); and when a plurality of each S⁷ to S¹⁸ exist, they may be the same or different.)

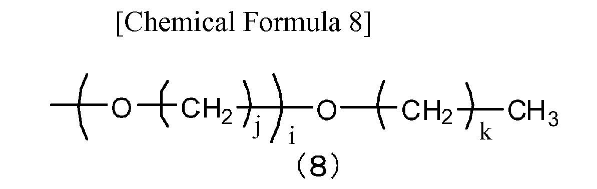

(In formula (8), i represents an integer of 1 to 6, j represents an integer of 1 to 6, and k represents an integer of 0 to 5; and when a plurality of j exist, they may be the same or different.)

The present invention provides, in a fourth aspect, a composition comprising at least one material selected from the group consisting of a hole transport material, an electron transport material, and a light-emitting material, and the above polymer compound.

The present invention provides, in a fifth aspect, a solution comprising the above polymer compound and a solvent.

The present invention provides, in a sixth aspect, a thin film comprising the above polymer compound.

The present invention provides, in a seventh aspect, a polymer light-emitting device having an organic layer (for example, a light-emitting layer and a charge transport layer) between electrodes consisting of an anode and a cathode, the organic layer including the above polymer compound or the above composition.

### Advantageous Effects of Invention

An organic electroluminescent device produced using the polymer compound of the present invention has a low emission starting voltage. Therefore, the polymer compound of the present invention can be suitably used as a material of light-emitting layer of an organic electroluminescent device and is industrially very useful.

### Description of Embodiments

Hereinafter, the embodiment for carrying out the present invention is described in detail. However, the present invention is not limited to the following embodiments. In the present specification, i-represents iso, s- represents sec-, and t- represents tert-.

The polymer compound of the present invention has a repeating unit represented by the above formula (1-0).

In formula (1-0), the aromatic hydrocarbon rings represented by ring A⁰¹ and ring A⁰² are preferably a single benzene ring or a ring into which a plurality of benzene rings are condensed, and examples thereof include a benzene ring, a naphthalene ring, an anthracene ring, a tetracene ring, a pentacene ring, a pyrene ring, a phenanthrene ring, and the like. As the combination of ring A⁰¹ and ring A⁰² combinations of a benzene ring and a benzene ring, a benzene ring and a naphthalene ring, a benzene ring and an anthracene ring, a naphthalene ring and a naphthalene ring, and a naphthalene ring and an anthracene ring are preferable, and a combination of a benzene ring and a benzene ring is more preferable.

When the aromatic hydrocarbon ring is substituted, the substituent may be one or a plurality of substituents, and when a plurality of substituents exist, they may be the same or different. Examples of the substituent include a halogeno group, an alkyl group, an alkenyl group, an alkynyl group, an alkoxy group, a group represented by the above formula (3), an alkylthio group, an aryl group, an aryloxy group, and an alkylsilyl group.

Examples of the halogeno group include a fluoro group, a chloro group, a bromo group, and an iodine group.

The alkyl group may be linear or branched, and may be a cycloalkyl group. The alkyl group is optionally substituted, and the carbon number of the alkyl group excluding a substituent is generally from 1 to 20. Examples of the alkyl group include a methyl group, an ethyl group, a propyl group, an i-propyl group, a butyl group, an i-butyl group, an s-butyl group, a t-butyl group, a pentyl group, a hexyl group, a cyclohexyl group, a heptyl group, an octyl group, a 2-ethylhexyl group, a nonyl group, a decyl group, a 3,7-dimethyloctyl group, and a dodecyl group. As the substituent which the alkyl group may have, an alkoxy group, an aryl group, an aryloxy group, a halogeno group, and a cyano group are preferable from the viewpoint of solubility, fluorescent properties, ease of synthesizing a monomer, and properties when made into a device.

The alkenyl group is optionally substituted, and the carbon number of the alkenyl group excluding a substituent is generally from 2 to 20. Examples of the alkenyl group include a vinyl group, a 1-propenyl group, a 2-propenyl group, a butenyl group, a pentenyl group, a hexenyl group, a heptenyl group, an octenyl group, and a cyclohexenyl group. Moreover, examples of the alkenyl group also include alkadienyl groups such as a 1,3-butadienyl group. As the substituent which the alkenyl group may have, an alkoxy group, an aryl group, an aryloxy group, a halogeno group, and a cyano group are preferable from the viewpoint of solubility, fluorescent properties, ease of synthesis, and properties when made into a device.

The alkynyl group is optionally substituted, and the carbon number of the alkynyl group excluding a substituent is generally from 2 to 20. Examples of the alkynyl group include an ethynyl group, a 1-propynyl group, a 2-propynyl group, a butynyl group, a pentynyl group, a hexynyl group, a heptenyl group, an octynyl group, and a cyclohexylethynyl group. Moreover, examples of the alkynyl group also include alkydienyl groups such as a 1,3-butadiynyl group, and groups having both a double bond and a triple bond such as a 2-pentene-4-ynyl group. As the substituent which the alkynyl group may have, an alkoxy group, an aryl group, an aryloxy group, a halogeno group, and a cyano group are preferable from the viewpoint of solubility, fluorescent properties, ease of synthesis, and properties when made into a device.

The alkoxy group may be linear or branched, and may be a cycloalkyloxy group. The alkoxy group is optionally substituted, and the carbon number of a portion excluding the substituent is generally from 1 to 20. Examples of the alkoxy group include a methoxy group, an ethoxy group, a propyloxy group, an i-propyloxy group, a butoxy group, an i-butoxy group, an s-butoxy group, a t-butoxy group, a pentyloxy group, a hexyloxy group, a cyclohexyloxy group, a heptyloxy group, an octyloxy group, a 2-ethylhexyloxy group, a nonyloxy group, a decyloxy group, a 3,7-dimethyloctyloxy group, a dodecyloxy group, a methoxymethyloxy group, and a 2-methoxyethyloxy group. As the substituent which the alkoxy group may have, an alkenyl group, an alkynyl group, an alkoxy group, an aryl group, an aryloxy group, a halogeno group, and a cyano group are preferable from the viewpoint of solubility, fluorescent properties, ease of synthesis, and properties when made into a device.

The alkylthio group may be linear or branched, and may be a cycloalkylthio group. The alkylthio group is optionally substituted, and the carbon number of the alkylthio group excluding a substituent is generally from 1 to 20. Examples of the alkylthio group include a methylthio group, an ethylthio group, a propylthio group, an i-propylthio group, a butylthio group, an i-butylthio group, an s-butylthio group, a t-butylthio group, a pentylthio group, a hexylthio group, a cyclohexylthio group, a heptylthio group, an octylthio group, a 2-ethylhexylthio group, a nonylthio group, a decylthio group, a 3,7-dimethyloctylthio group, and a dodecylthio group. As the substituent which the alkylthio group may have, an alkenyl group, an alkynyl group, an alkoxy group, an aryl group, an aryloxy group, a halogeno group, and a cyano group are preferable from the viewpoint of solubility, fluorescent properties, ease of synthesis, and properties when made into a device.

The aryl group is an atomic group obtained by removing one hydrogen atom from an aromatic hydrocarbon, and also includes groups having a condensed ring, and groups in which two or more of either or both independent benzene rings or/and condensed rings are bonded directly or via a vinylene group or the like. The aryl group is optionally substituted, and the carbon number of a portion excluding the substituent is generally about 6 to 60. Examples of the aryl group include a phenyl group, C₁-C₁₂ alkylphenyl groups (C₁-C₁₂ indicates that the carbon number of is 1 to 12. The same applies hereinafter.), C₁-C₁₂ alkoxyphenyl groups, a 1-naphthyl group, a 2-naphthyl group, a 1-anthracenyl group, a 2-anthracenyl group, a 9-anthracenyl group, a 1-pyrenyl group, a 2-pyrenyl group, and a 4-pyrenyl group. Examples of the C₁-C₁₂ alkylphenyl groups include a methylphenyl group, a dimethylphenyl group, a trimethylphenyl group, an ethylphenyl group, a diethylphenyl group, a triethylphenyl group, a propylphenyl group, a dipropylphenyl group, an i-propylphenyl group, a di(i-propyl)phenyl group, a butylphenyl group, a dibutylphenyl group, an i-butylphenyl group, a di(i-butyl)phenyl group, an s-butylphenyl group, a di(s-butyl)phenyl group, a t-butylphenyl group, a di(t-butyl)phenyl group, a pentylphenyl group, a hexylphenyl group, a cyclohexylphenyl group, a heptylphenyl group, an octylphenyl group, a 2-ethylhexylphenyl group, a nonylphenyl group, a decylphenyl group, a 3,7-dimethyloctylphenyl group, and a dodecylphenyl group. Examples of the C₁-C₁₂ alkoxyphenyl groups include a methoxyphenyl group, a dimethoxyphenyl group, a trimethoxyphenyl group, an ethoxyphenyl group, a diethoxyphenyl group, a triethoxyphenyl group, a propyloxyphenyl group, a dipropyloxyphenyl group, an i-propyloxyphenyl group, a di(i-propyloxy)phenyl group, a butyloxyphenyl group, a dibutyloxyphenyl group, an i-butyloxyphenyl group, a di(i-butyloxy)phenyl group, an s-butyloxyphenyl group, a di(s-butyloxy)phenyl group, a t-butyloxyphenyl group, a di(t-butyloxy)phenyl group, a pentyloxyphenyl group, a hexyloxyphenyl group, a cyclohexyloxyphenyl group, a heptyloxyphenyl group, an octyloxyphenyl group, a 2-ethylhexyloxyphenyl group, a nonyloxyphenyl group, a decyloxyphenyl group, a 3,7-dimethyloctyloxyphenyl group, and a dodecyloxyphenyl group. As the substituent which the aryl group may have, an alkyl group, an alkenyl group, an alkynyl group, an alkoxy group, an aryloxy group, a halogeno group, and a cyano group are preferable from the viewpoint of solubility, fluorescent properties, ease of synthesis, and properties when made into a device.

The aryloxy group is a substituent represented by -OAr^{x}, and Ar^{x} represents an aryl group. The aryl group is the same as described above. The aryloxy group is optionally substituted, and the carbon number of a portion excluding the substituent is generally about 6 to 60. Examples of the aryloxy group include a phenyloxy group, C₁-C₁₂ alkylphenyloxy groups, C₁-C₁₂ alkoxyphenyloxy groups, a 1-naphthyloxy group, a 2-naphthyloxy group, a 1-anthracenyloxy group, a 2-anthracenyloxy group, a 9-anthracenyloxy group, a 1-pyrenyloxy group, a 2-pyrenyloxy group, and a 4-pyrenyloxy group. Examples of the C₁-C₁₂ alkylphenyloxy groups include a methylphenyloxy group, a dimethylphenyloxy group, a trimethylphenyloxy group, an ethylphenyloxy group, a diethylphenyloxy group, a triethylphenyloxy group, a propylphenyloxy group, a dipropylphenyloxy group, an i-propylphenyloxy group, a di(i-propyl)phenyloxy group, a di(i-butyl)phenyloxy group, an s-butylphenyloxy group, a di(s-butyl)phenyloxy group, a t-butylphenyloxy group, a di(t-butyl)phenyloxy group, a pentylphenyloxy group, a hexylphenyloxy group, a cyclohexylphenyloxy group, a heptylphenyloxy group, an octylphenyloxy group, a 2-ethylhexylphenyloxy group, nonylphenyloxy group, a decylphenyloxy group, a 3,7-dimethyloctylphenyloxy group, and a dodecylphenyloxy group. Examples of the C₁-C₁₂ alkoxyphenyl groups include a methoxyphenyloxy group, a dimethoxyphenyloxy group, a trimethoxyphenyloxy group, an ethoxyphenyloxy group, a diethoxyphenyloxy group, a triethoxyphenyloxy group, a propyloxyphenyloxy group, a dipropyloxyphenyloxy group, an i-propyloxyphenyloxy group, a di(i-propyloxy)phenyloxy group, a butyloxyphenyloxy group, a dibutyloxyphenyloxy group, an i-butyloxyphenyloxy group, a di(i-butyloxy)phenyloxy group, an s-butyloxyphenyloxy group, a di(s-butyloxy)phenyloxy group, a t-butyloxyphenyloxy group, a di(t-butyloxy)phenyloxy group, a pentyloxyphenyloxy group, a hexyloxyphenyloxy group, a cyclohexyloxyphenyloxy group, a heptyloxyphenyloxy group, an octyloxyphenyloxy group, a 2-ethylhexyloxyphenyloxy group, a nonyloxyphenyloxy group, a decyloxyphenyloxy group, a 3,7-dimethyloctyloxyphenyloxy group, and a dodecyloxyphenyloxy group. As the substituent which the aryloxy group may have, an alkyl group, an alkenyl group, an alkynyl group, an alkoxy group, a halogeno group, and a cyano group are preferable from the viewpoint of solubility, fluorescent properties, ease of synthesis, and properties when made into a device.

The alkylsilyl group may be linear or branched, and may be a cycloalkylsilyl group. The alkylsilyl group is optionally substituted, and the carbon number of the alkylsilyl group excluding a substituent is generally from 1 to 20. Examples of the alkylsilyl group include a methylsilyl group, an ethylsilyl group, a propylsilyl group, an i-propylsilyl group, a butylsilyl group, an i-butylsilyl group, an s-butylsilyl group, a t-butylsilyl group, a pentylsilyl group, a hexylsilyl group, a cyclohexylsilyl group, a heptylsilyl group, an octylsilyl group, a 2-ethylhexylsilyl group, a nonylsilyl group, a decylsilyl group, a 3,7-dimethyloctylsilyl group, and a dodecylsilyl group. As the substituent which the alkylsilyl group may have, an alkyl group, an alkenyl group, an alkynyl group, an alkoxy group, a halogeno group, and a cyano group are preferable from the viewpoint of solubility, fluorescent properties, ease of synthesis, and properties when made into a device.

In formula (1-0), X¹⁰ and X²⁰ are the same or different and each represents a hydrogen atom or a substituent, provided that at least one of X¹ and X² is a group represented by the above formula (2-0).

Examples of a substituent other than the groups represented by formula (2-0), from the viewpoint of light-emitting properties when made into a device, include an alkyl group, an aryl group, an alkenyl group, an alkynyl group, an alkoxy group, an alkylthio group, an alkylsilyl group, an aryloxy group, and a group represented by formula (3), and an alkyl group, an aryl group, an alkenyl group, and an alkynyl group are preferable. Examples of these substituents include the same groups as the examples of the alkyl group, the aryl group, the alkenyl group, the alkynyl group, the alkoxy group, the alkylthio group, the alkylsilyl group, and the aryloxy group, which are substituents which the above ring A⁰¹ may have.

When X¹⁰ is a group represented by formula (2-0), from the viewpoint of light-emitting properties when made into a device, X²⁰ is preferably a hydrogen atom, an alkyl group, an aryl group, an alkenyl group, an alkynyl group, an alkoxy group, an alkylthio group, an alkylsilyl group, an aryloxy group, a group represented by formula (2-0), or a group represented by formula (3), more preferably a hydrogen atom, an alkyl group, an aryl group, an alkenyl group, an alkynyl group, or a group represented by formula (2-0), and further preferably an alkyl group, an aryl group, an alkenyl group, or an alkynyl group.

When both X¹⁰ and X²⁰ are a group represented by formula (2-0), X¹⁰ and X²⁰ are preferably the same, from the viewpoint of ease of monomer synthesis.

In formula (2-0), Z¹⁰, Z²⁰ and Z³⁰ are the same or different and each represents -N= or -CH=, provided that at least two of Z¹⁰, Z²⁰ and Z³⁰ are -N=. From the viewpoint of the emission starting voltage of the device and driving voltage at practical luminance, Z¹⁰, Z²⁰ and Z³⁰ are preferably all -N=.

In formula (2-0), L⁰ represents an arylene group or a single bond, and is preferably an arylene group. The arylene group is an atomic group obtained by removing two hydrogen atoms from an aromatic hydrocarbon and also includes groups having a condensed ring, and groups in which two or more of either or both independent benzene rings or/and condensed rings are bonded directly or via a vinylene group or the like. The arylene group is optionally substituted.

When L⁰ is an arylene group, the carbon number of a portion excluding the substituent is generally from 6 to 60, preferably from 6 to 18, more preferably from 6 to 12, and further preferably 12, and the total carbon number including the substituent is generally from 6 to 100.

When L⁰ is an arylene group, it is preferable not to be substituted from the viewpoint of monomer synthesis, and it is preferable to be substituted from the viewpoint of solvent solubility of monomer and the resulting polymer compound. In the case of being substituted, an alkyl group, an alkenyl group, an alkynyl group, an alkoxy group, an alkylthio group, an alkylsilyl group, an aryloxy group, and a group represented by formula (3) are preferable for the substituent, and an alkyl group is more preferable for the substituent. Examples of these substituents include the same groups as the examples of the alkyl group, the alkenyl group, the alkynyl group, the alkoxy group, the alkylthio group, the alkylsilyl group, and the aryloxy group, which are substituents which the above ring A⁰¹ may have.

When L⁰ is an arylene group, the structure of L⁰ of a portion excluding the substituent is represented, for example, by the following formulae L1 to L18.

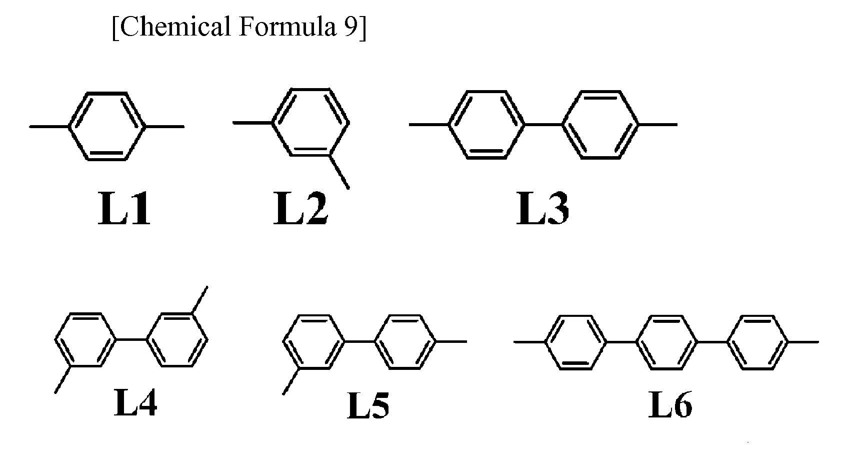

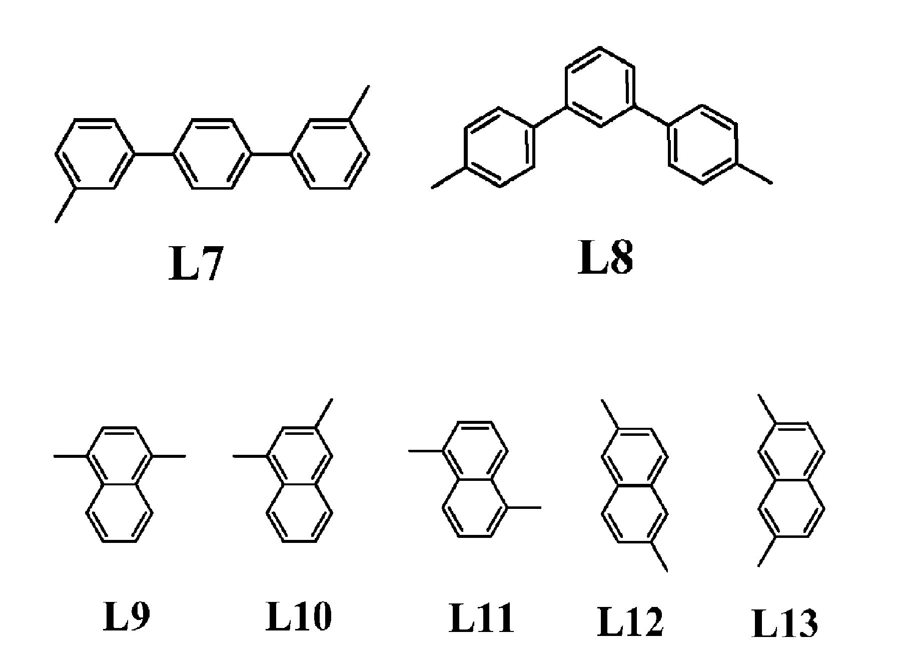

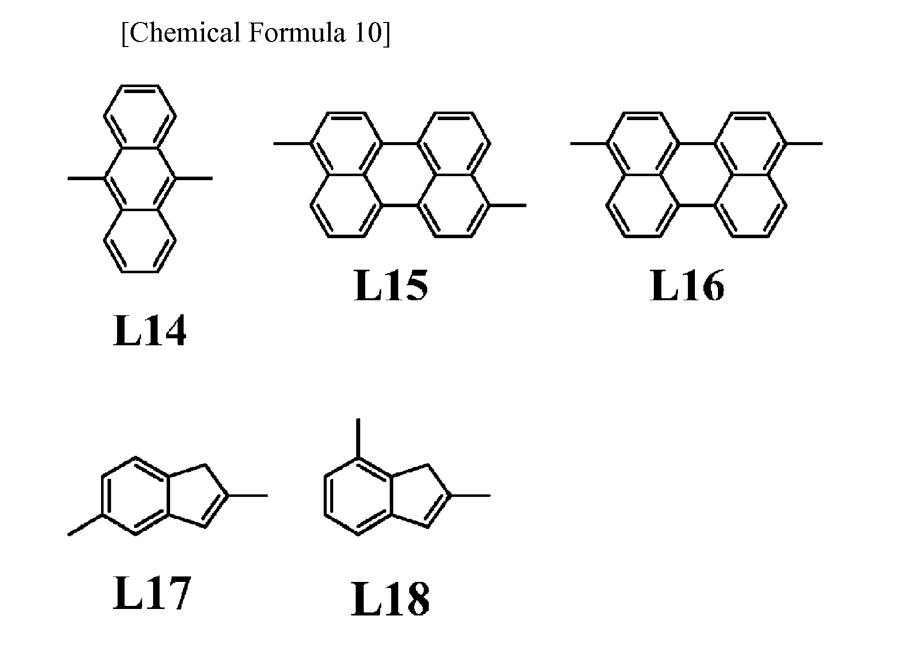

In formula (2-0), Ar¹⁰ and Ar²⁰ are the same or different and each represents an aryl group. Examples of the aryl group include the same groups as the examples of the aryl group that is the substituents which the above ring A⁰¹ may have. Ar¹⁰ and Ar²⁰ are preferably the same, from the viewpoint of monomer synthesis.

In formula (2-0), the total carbon number of the groups represented by Ar¹⁰, Ar²⁰ and L⁰ is 24 or more. When the groups represented by Ar¹⁰, Ar²⁰ and L⁰ are substituted, the total carbon number of the groups represented by Ar¹⁰, Ar²⁰ and L⁰ includes the carbon number which the substituent has.

Examples of the group represented by formula (2-0) include groups represented by formula (2-0-1) to the following formula (2-0-10), and groups represented by formula (2-1) to formula (2-19) described later.

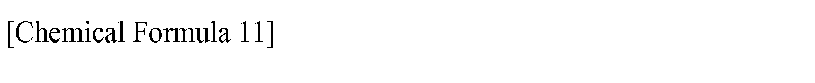

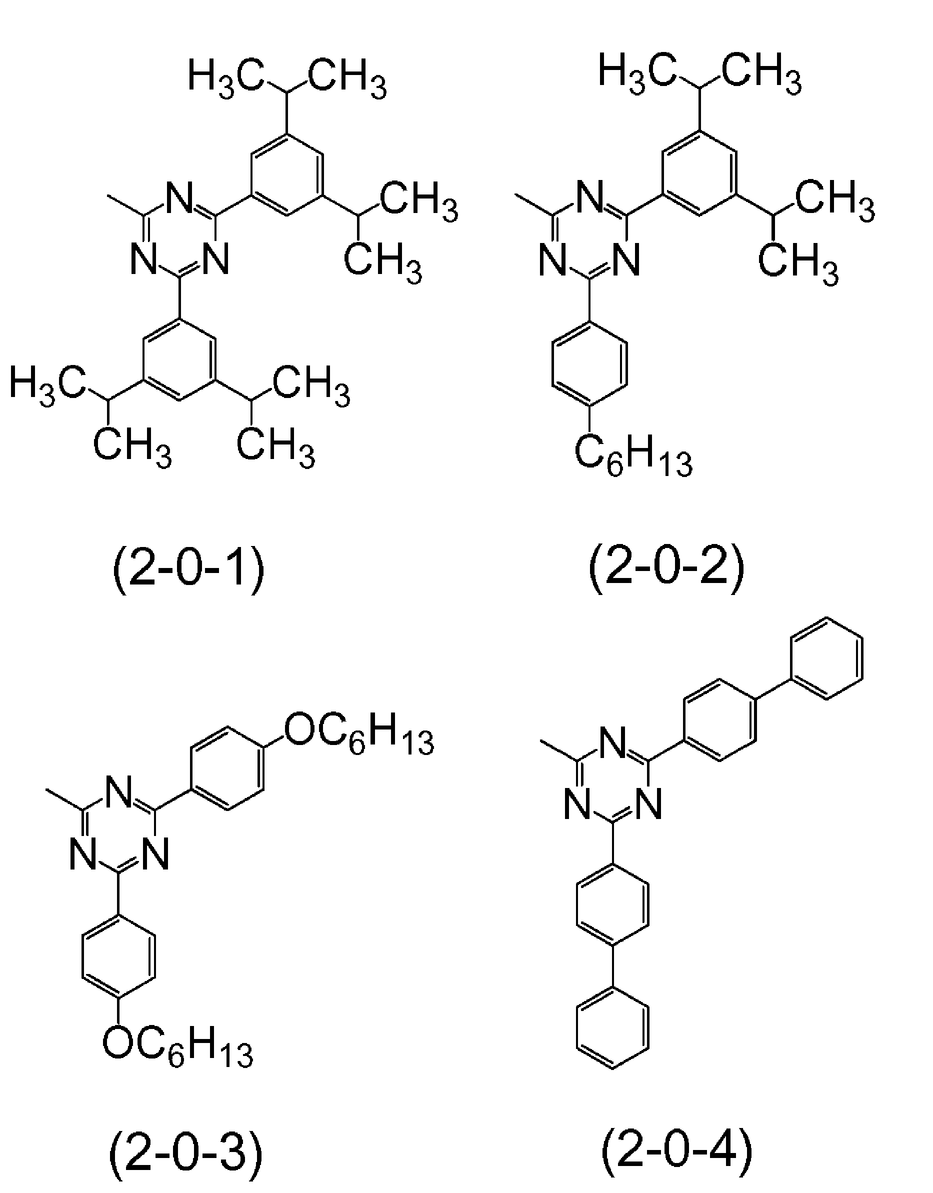

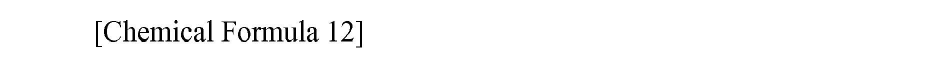

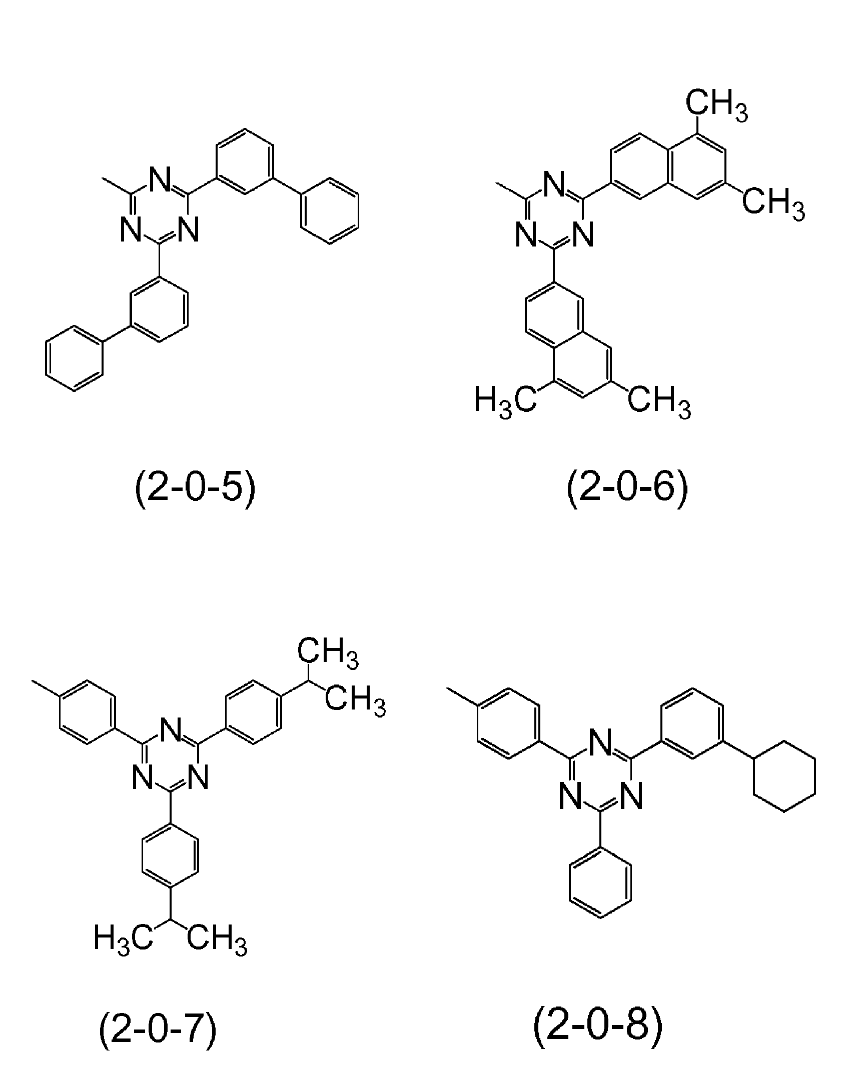

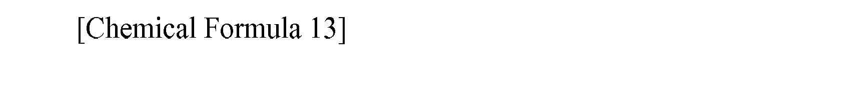

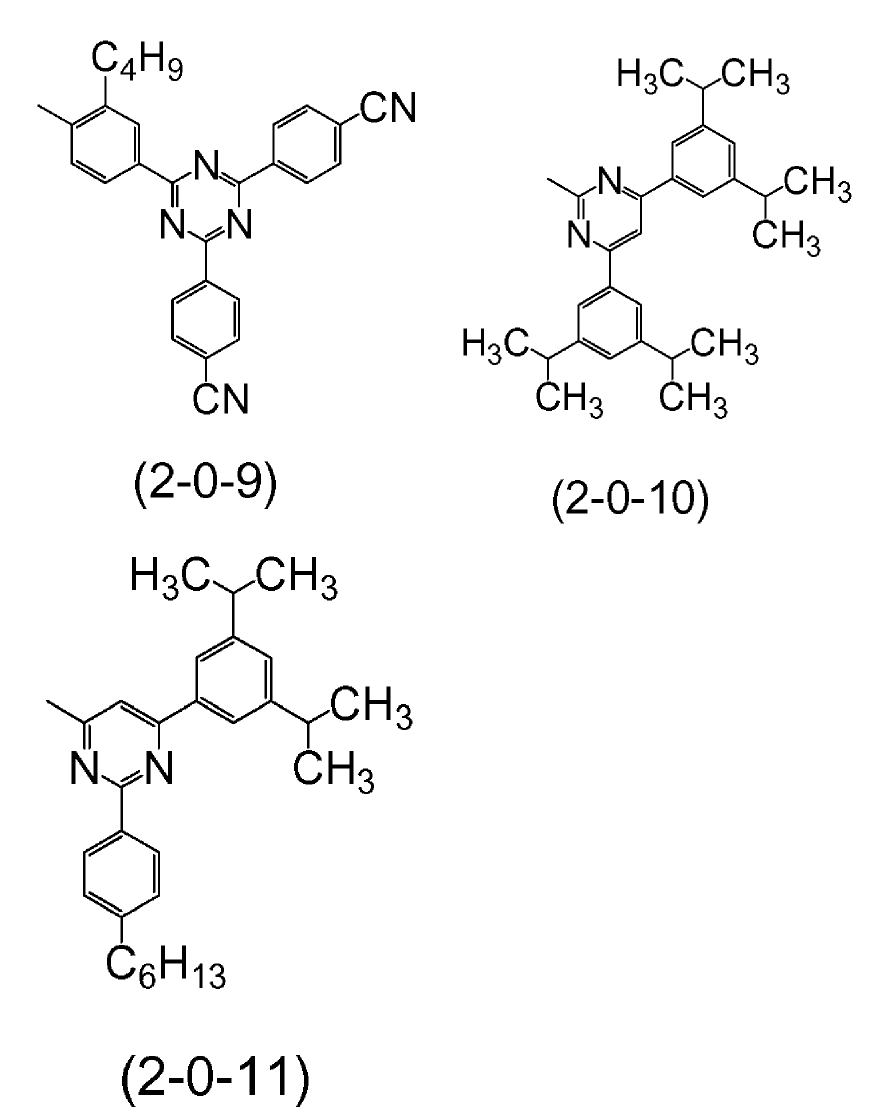

The repeating unit represented by formula (1-0) is preferably a repeating unit represented by formula (1), from the viewpoint of light-emitting properties when made into a device.

In formula (1), the aromatic hydrocarbon rings represented by ring A¹ and ring A² are preferably a single benzene ring or a ring into which a plurality of benzene rings are condensed, and examples thereof include a benzene ring, a naphthalene ring, an anthracene ring, a tetracene ring, a pentacene ring, a pyrene ring, and a phenanthrene ring. As the combination of ring A¹ and ring A², combinations of a benzene ring and a benzene ring, a benzene ring and a naphthalene ring, a benzene ring and an anthracene ring, and a naphthalene ring and an anthracene ring are preferable, and a combination of a benzene ring and a benzene ring is more preferable.

When the aromatic hydrocarbon ring is substituted, the substituent may be one or more, and when a plurality of substituents exist, they may be the same or different. Examples of the substituent include a halogeno group, an alkyl group, an alkenyl group, an alkynyl group, an alkoxy group, a group represented by formula (3), an alkylthio group, an aryl group, and an alkylsilyl group.

Examples of the halogeno group include a fluoro group, a chloro group, a bromo group, and an iodine group.

The alkyl group may be linear or branched, and may be a cycloalkyl group. The alkyl group is optionally substituted, and the carbon number of the alkyl group excluding a substituent is generally from 1 to 20. Examples of the alkyl group include a methyl group, an ethyl group, a propyl group, an i-propyl group, a butyl group, an i-butyl group, an s-butyl group, a t-butyl group, a pentyl group, a hexyl group, a cyclohexyl group, a heptyl group, an octyl group, a 2-ethylhexyl group, a nonyl group, a decyl group, a 3,7-dimethyloctyl group, and a dodecyl group. As the substituent which the alkyl group may have, an alkoxy group, an aryl group, a halogeno group, and a cyano group are preferable from the viewpoint of solubility, fluorescent properties, ease of synthesizing a monomer, and properties when made into a device.

The alkenyl group is optionally substituted, and the carbon number of the alkenyl group excluding a substituent is generally from 2 to 20. Examples of the alkenyl group include a vinyl group, a 1-propenyl group, a 2-propenyl group, a butenyl group, a pentenyl group, a hexenyl group, a heptenyl group, an octenyl group, and a cyclohexenyl group. Moreover, examples of the alkenyl group also include alkadienyl groups such as a 1,3-butadienyl group. As the substituent which the alkenyl group may have, an alkoxy group, an aryl group, a halogeno group, and a cyano group are preferable from the viewpoint of solubility, fluorescent properties, ease of synthesizing a monomer, and properties when made into a device.

The alkynyl group is optionally substituted, and the carbon number of the alkynyl group excluding a substituent is generally from 2 to 20. Examples of the alkynyl group include an ethynyl group, a 1-propynyl group, a 2-propynyl group, a butynyl group, a pentynyl group, a hexynyl group, a heptynyl group, an octynyl group, and a cyclohexylethynyl group. Moreover, examples of the alkynyl group also include alkydienyl groups such as a 1,3-butadiynyl group, and groups having both a double bond and a triple bond such as a 2-pentene-4-ynyl group. As the substituent which the alkynyl group may have, an alkoxy group, an aryl group, a halogeno group, and a cyano group are preferable from the viewpoint of solubility, fluorescent properties, ease of synthesis, and properties when made into a device, and the like.

The alkoxy group may be linear or branched, and may be a cycloalkyloxy group. The alkoxy group is optionally substituted, and the carbon number of a portion excluding the substituent is generally from 1 to 20. Examples of the alkoxy group include a methoxy group, an ethoxy group, a propyloxy group, an i-propyloxy group, a butoxy group, an i-butoxy group, an s-butoxy group, a t-butoxy group, a pentyloxy group, a hexyloxy group, a cyclohexyloxy group, a heptyloxy group, an octyloxy group, a 2-ethylhexyloxy group, a nonyloxy group, a decyloxy group, a 3,7-dimethyloctyloxy group, a dodecyloxy group, a methoxymethyloxy group, and a 2-methoxyethyloxy group. As the substituent which the alkoxy group may have, an alkenyl group, an alkynyl group, an alkoxy group, an aryl group, a halogeno group, and a cyano group are preferable from the viewpoint of solubility, fluorescent properties, ease of synthesis, and properties when made into a device.

The alkylthio group may be linear or branched, and may be a cycloalkylthio group. The alkylthio group is optionally substituted, and the carbon number of the alkylthio group excluding a substituent is generally from 1 to 20. Examples of the alkylthio group include a methylthio group, an ethylthio group, a propylthio group, an i-propylthio group, a butylthio group, an i-butylthio group, an s-butylthio group, a t-butylthio group, a pentylthio group, a hexylthio group, a cyclohexylthio group, a heptylthio group, an octylthio group, a 2-ethylhexylthio group, a nonylthio group, a decylthio group, a 3,7-dimethyloctylthio group, and a dodecylthio group. As the substituent which the alkylthio group may have, an alkenyl group, an alkynyl group, an alkoxy group, an aryl group, a halogeno group, and a cyano group are preferable from the viewpoint of solubility, fluorescent properties, ease of synthesis, and properties when made into a device.

The aryl group is an atomic group obtained by removing one hydrogen atom from an aromatic hydrocarbon, and also includes groups having a condensed ring, and groups in which two or more of either or both independent benzene rings or/and condensed rings are bonded directly or via a vinylene group or the like. The aryl group is optionally substituted, and the carbon number of a portion excluding the substituent is generally from 6 to 60. Examples of the aryl group include a phenyl group, C₁-C₁₂ alkylphenyl groups (C₁-C₁₂ indicates that the carbon number of is 1 to 12. The same applies hereinafter.), C₁-C₁₂ alkoxyphenyl groups, a 1-naphthyl group, a 2-naphthyl group, a 1-anthracenyl group, a 2-anthracenyl group, a 9-anthracenyl group, a 1-pyrenyl group, a 2-pyrenyl group, and a 4-pyrenyl group. Examples of the C₁-C₁₂ alkylphenyl groups include a methylphenyl group, a dimethylphenyl group, a trimethylphenyl group, an ethylphenyl group, a diethylphenyl group, a triethylphenyl group, a propylphenyl group, a dipropylphenyl group, an i-propylphenyl group, a di(i-propyl)phenyl group, a butylphenyl group, a dibutylphenyl group, an i-butylphenyl group, a di(i-butyl)phenyl group, an s-butylphenyl group, a di(s-butyl)phenyl group, a t-butylphenyl group, a di(t-butyl)phenyl group, a pentylphenyl group, a hexylphenyl group, a cyclohexylphenyl group, a heptylphenyl group, an octylphenyl group, a 2-ethylhexylphenyl group, a nonylphenyl group, a decylphenyl group, a 3,7-dimethyloctylphenyl group, and a dodecylphenyl group. Examples of the C₁-C₁₂ alkoxyphenyl groups include, specifically, a methoxyphenyl group, a dimethoxyphenyl group, a trimethoxyphenyl group, an ethoxyphenyl group, a diethoxyphenyl group, a triethoxyphenyl group, a propyloxyphenyl group, a dipropyloxyphenyl group, an i-propyloxyphenyl group, a di(i-propyloxy)phenyl group, a butyloxyphenyl group, a dibutyloxyphenyl group, an i-butyloxyphenyl group, a di(i-butyloxy)phenyl group, an s-butyloxyphenyl group, a di(s-butyloxy)phenyl group, a t-butyloxyphenyl group, a di(t-butyloxy)phenyl group, a pentyloxyphenyl group, a hexyloxyphenyl group, a cyclohexyloxyphenyl group, a heptyloxyphenyl group, an octyloxyphenyl group, a 2-ethylhexyloxyphenyl group, a nonyloxyphenyl group, a decyloxyphenyl group, a 3,7-dimethyloctyloxyphenyl group, and a dodecyloxyphenyl group. As the substituent which the aryl group may have, an alkyl group, an alkenyl group, an alkynyl group, an alkoxy group, a halogeno group, and a cyano group are preferable from the viewpoint of solubility, fluorescent properties, ease of synthesis, and properties when made into a device.

The alkylsilyl group may be linear or branched, and may be a cycloalkylsilyl group. The alkylsilyl group is optionally substituted, and the carbon number of the alkylsilyl group excluding a substituent is generally from 1 to 20. Examples of the alkylsilyl group include a methylsilyl group, an ethylsilyl group, a propylsilyl group, an i-propylsilyl group, a butylsilyl group, an i-butylsilyl group, an s-butylsilyl group, a t-butylsilyl group, a pentylsilyl group, a hexylsilyl group, a cyclohexylsilyl group, a heptylsilyl group, an octylsilyl group, a 2-ethylhexylsilyl group, a nonylsilyl group, a decylsilyl group, a 3,7-dimethyloctylsilyl group, and a dodecylsilyl group. As the substituent which the alkylsilyl group may have, an alkyl group, an alkenyl group, an alkynyl group, an alkoxy group, a halogeno group, and a cyano group are preferable from the viewpoint of solubility, fluorescent properties, ease of synthesis, and properties when made into a device.

In formula (3), e represents an integer of 1 to 6, g represents an integer of 1 to 6, and h represents an integer of 0 to 5. When a plurality of g exist, they may be the same or different. From the viewpoint of monomer synthesis, e is preferably an integer of 1 to 3, g is preferably 1 or 2, and h is preferably 0 or 1.

In formula (1), X¹ and X² are the same or different and each represents a hydrogen atom or a substituent, provided that at least one of X¹ and X² is a group represented by formula (2).

As a substituent other than the groups represented by formula (2), from the viewpoint of light-emitting properties when made into a device, an alkyl group, an aryl group, an alkenyl group, an alkynyl group, an alkoxy group, an alkylthio group, an alkylsilyl group, and a group represented by formula (3) are preferable, and an alkyl group, an aryl group, an alkenyl group, and an alkynyl group are more preferable. Examples of these substituents include the same groups as the examples of the alkyl group, the aryl group, the alkenyl group, the alkynyl group, the alkoxy group, the alkylthio group, and the alkylsilyl group, which are substituents which the above ring A¹ may have.

When X¹ is a group represented by formula (2), from the viewpoint of light-emitting properties when made into a device, X² is preferably a hydrogen atom, an alkyl group, an aryl group, an alkenyl group, an alkynyl group, an alkoxy group, an alkylthio group, an alkylsilyl group, a group represented by formula (2), or a group represented by formula (3), more preferably a hydrogen atom, an alkyl group, an aryl group, an alkenyl group, an alkynyl group, or a group represented by formula (2), and further preferably an alkyl group, an aryl group, an alkenyl group, or an alkynyl group.

When both X¹ and X² are a group represented by formula (2), X¹ and X² are preferably the same, from the viewpoint of ease of monomer synthesis.

In formula (2), Z¹, Z² and Z³ are the same or different and each represents -N= or -CH=, provided that at least two of Z¹, Z² and Z³ are -N=. From the viewpoint of the emission starting voltage of the device and driving voltage at practical luminance, Z¹, Z² and Z³ are preferably all -N=. Examples of the group represented by formula (2) include groups represented by the following formula (2-1) to formula (2-4), and among them, a group represented by formula (2-4) in which Z¹, Z² and Z³ are all -N= is preferable.

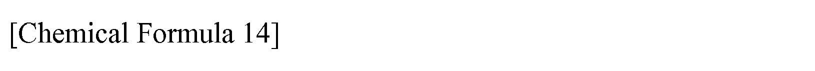

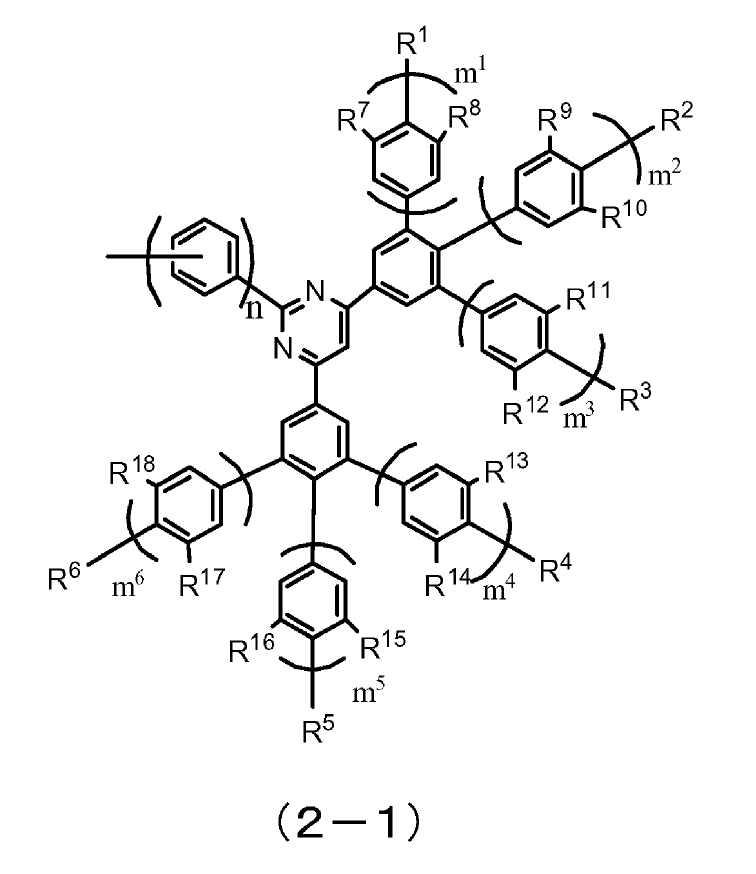

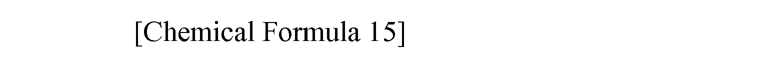

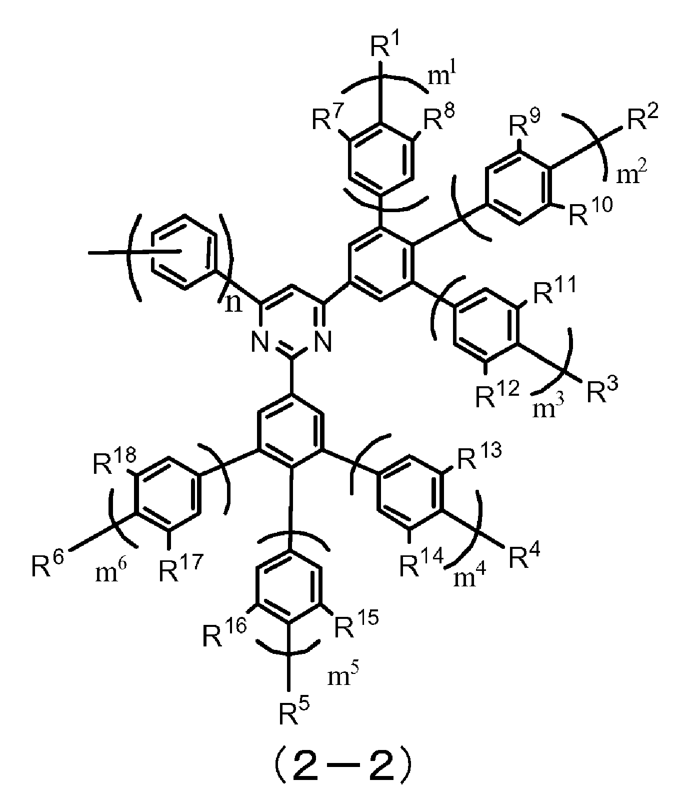

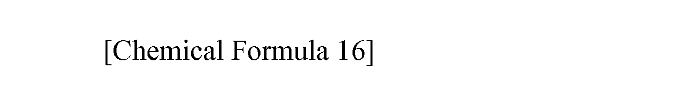

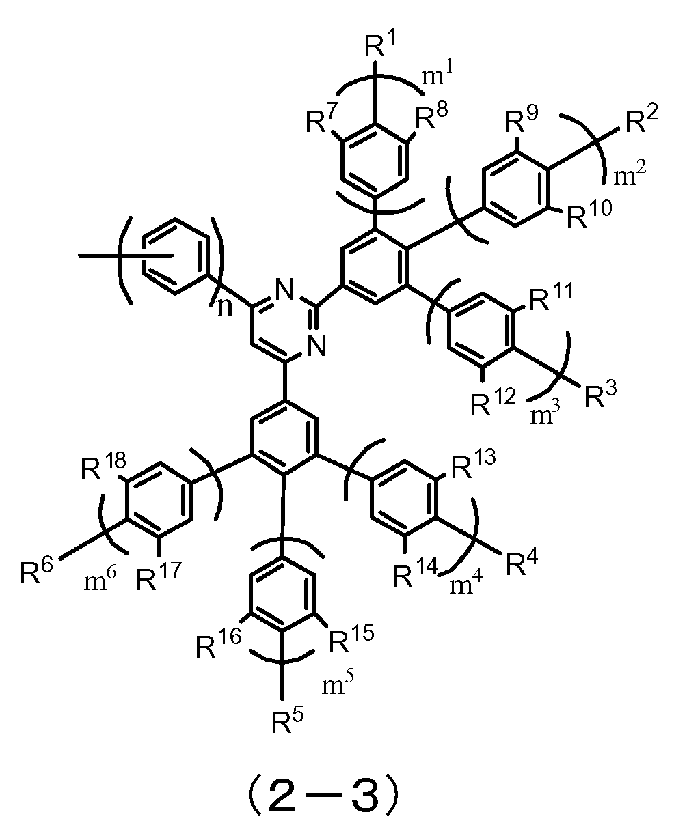

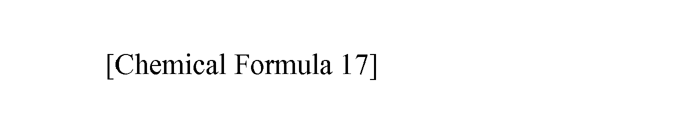

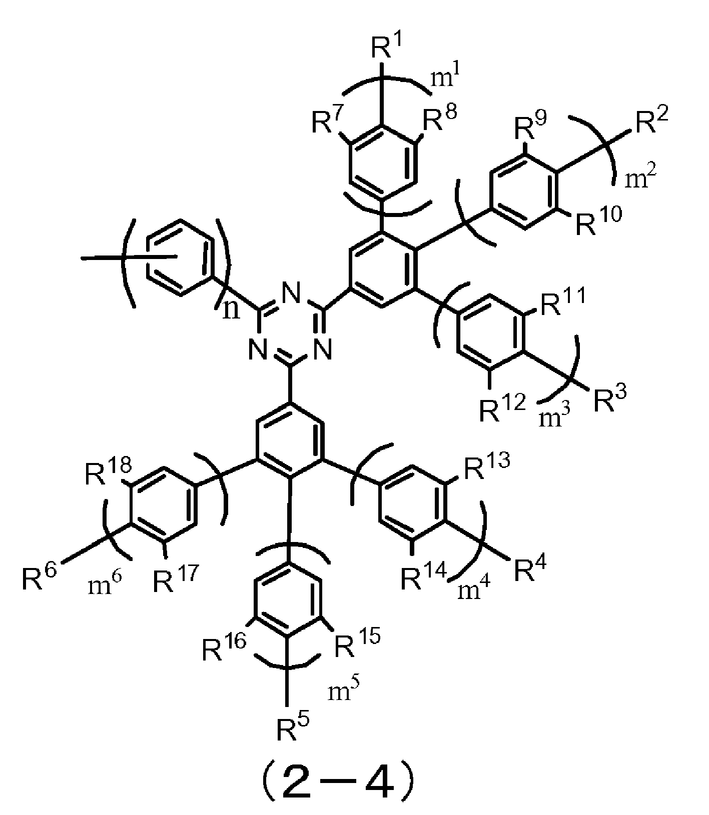

(In formula (2-1) to formula (2-4), n, m¹ to m⁶ and R¹ to R¹⁸ represent the same meanings as described above.)

In formula (2), n represents an integer of 0 or more, and is preferably an integer of 0 to 3, more preferably 1 or 2, and further preferably 2.

In formula (2), m¹ to m⁶ are the same or different and each represents an integer of 0 or more, provided that m¹ + m² + m³ ≥ 1 and m⁴ + m⁵ + m⁶ ≥ 1. m¹ to m⁶ are preferably 0, 1 or 2, and more preferably 0 or 1.

When m¹ to m⁶ are 0 or 1, the group represented by formula (2) is represented by the following formula (2-5) to formula (2-19). Among these groups, from the viewpoint of charge transporting properties and properties when made into a device, the groups represented by formula (2-10), formula (2-11), formula (2-13), formula (2-16), and formula (2-19) are preferable, and the group represented by formula (2-10) is more preferable. In addition, among these groups, from the viewpoint of ease of monomer synthesis, the groups represented by the following formula (2-5), formula (2-10), formula (2-14), formula (2-17), and formula (2-19) are preferable, and the groups represented by formula (2-5), formula (2-10) and formula (2-17) are more preferable.

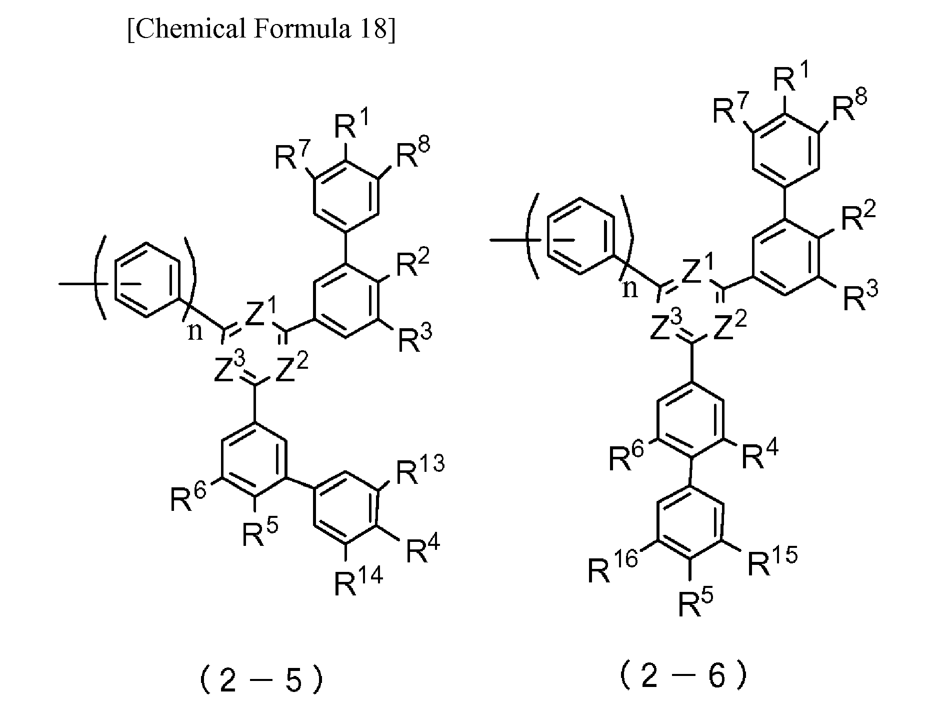

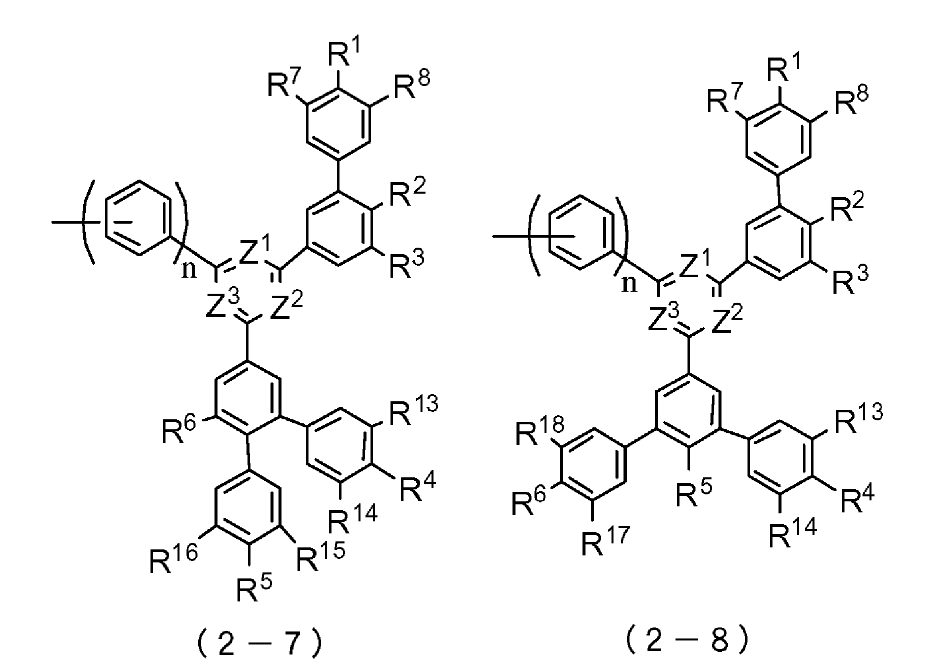

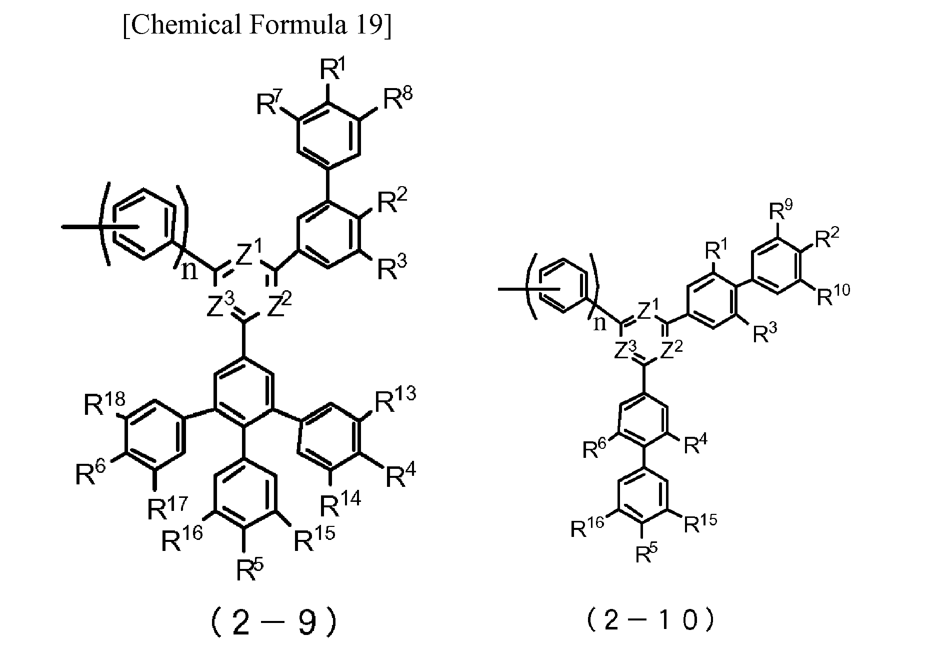

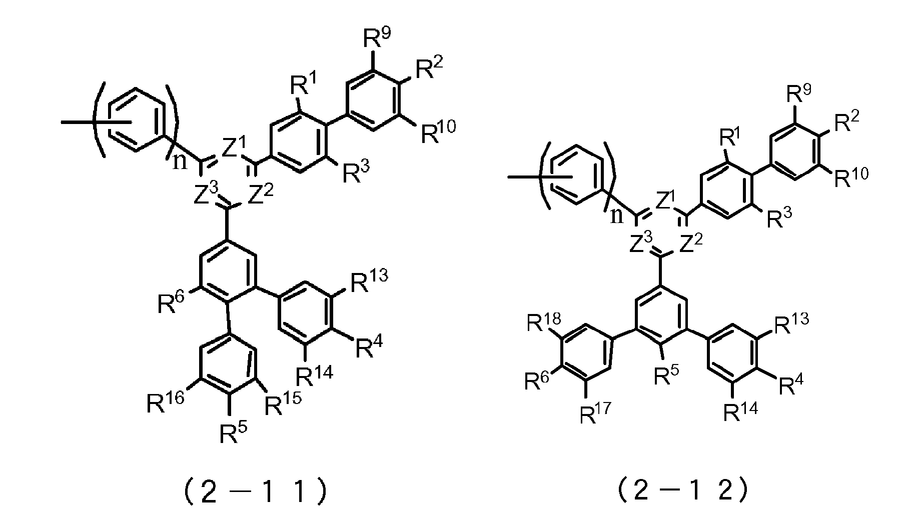

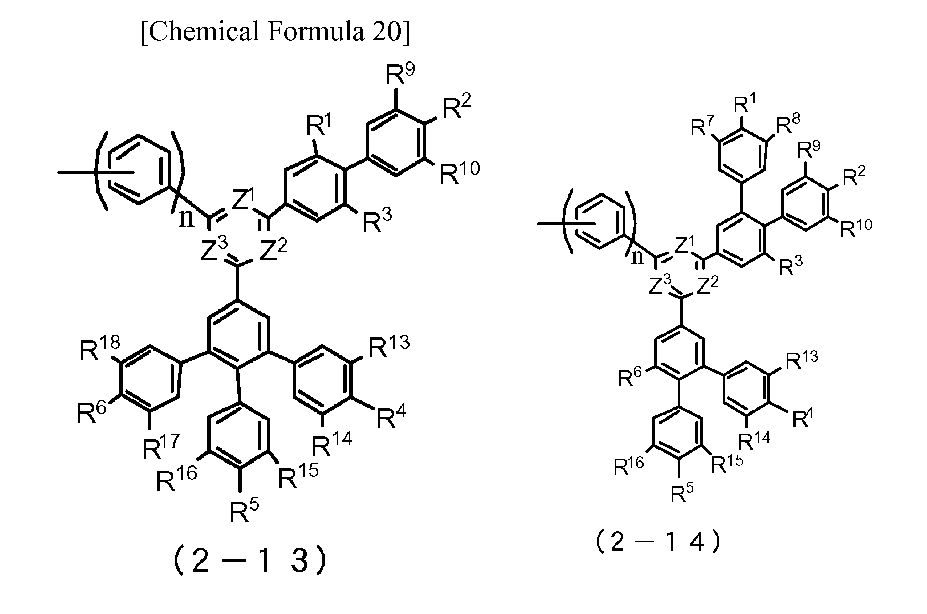

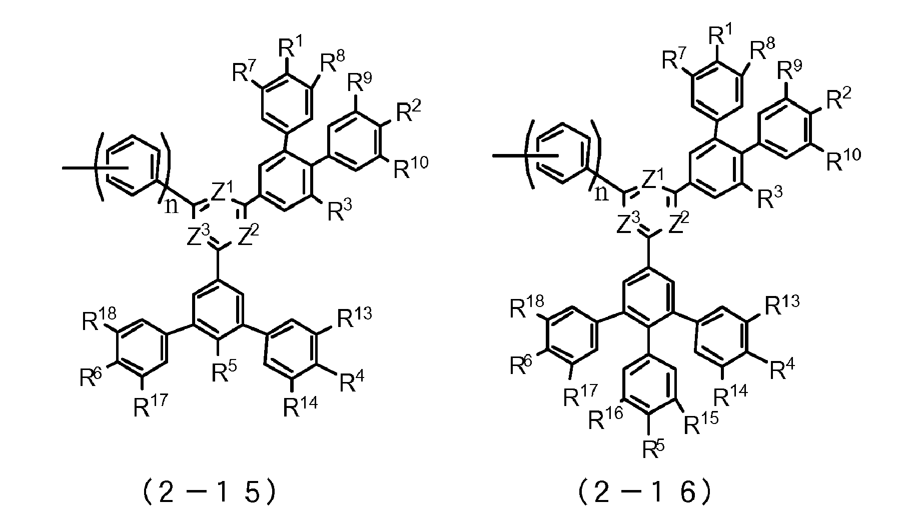

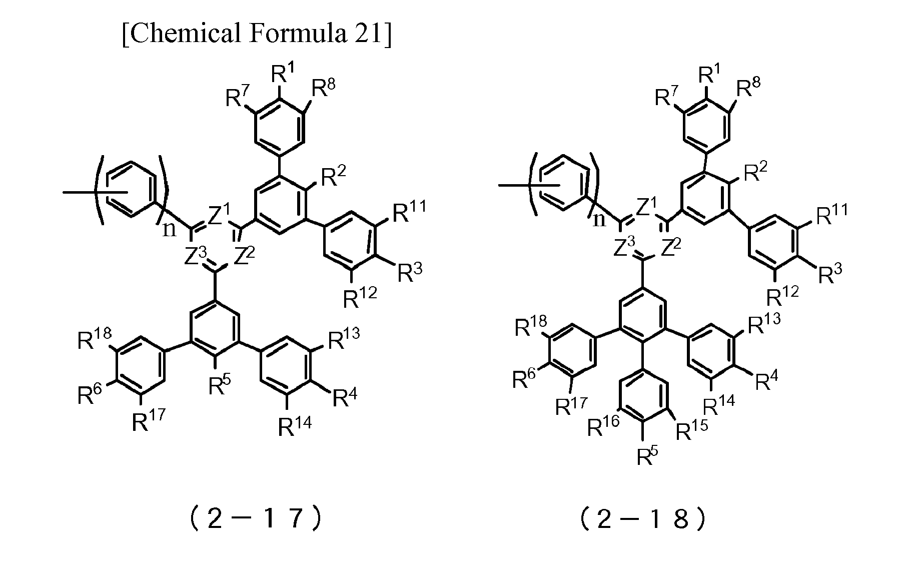

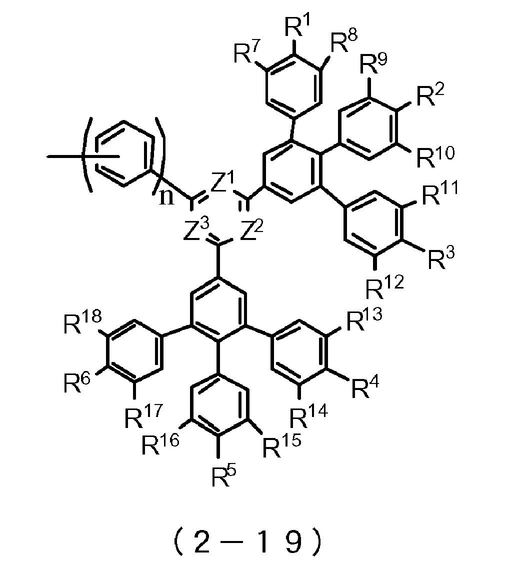

(In formula (2-5) to formula (2-19), n, R¹ to R¹⁸ and Z¹ to Z³ represent the same meanings as described above.)

In formula (2), R¹ to R⁶ are the same or different and each represents a hydrogen atom, a halogeno group, an alkyl group, an alkenyl group, an alkynyl group, an alkoxy group, a group represented by formula (3), an alkylthio group, or an alkylsilyl group, and are preferably a hydrogen atom, an alkyl group, an alkenyl group, or an alkynyl group, and more preferably a hydrogen atom and an alkyl group.

In formula (2), R⁷ to R¹⁸ are the same or different and each represents a hydrogen atom, a halogeno group, an alkyl group, an alkenyl group, an alkynyl group, an alkoxy group, a group represented by formula (3), an alkylthio group, an aryl group, or an alkylsilyl group, and when a plurality of each R⁷ to R¹⁸ exist, they may be the same or different and are preferably a hydrogen atom, an alkyl group, an alkenyl group, an alkynyl group, or aryl group, and more preferably a hydrogen atom and an alkyl group.

Examples of the halogeno group include a fluoro group, a chloro group, a bromo group, and an iodine group.

Examples of the alkyl group, the aryl group, the alkenyl group, the alkynyl group, the alkoxy group, the alkylthio group, and the alkylsilyl group include the same groups as the examples of the alkyl group, the aryl group, the alkenyl group, the alkynyl group, the alkoxy group, the alkylthio group, and the alkylsilyl group, which are substituents which the above ring A¹ may have.

From the viewpoint of light-emitting properties when made into a device, m¹, m³, m⁴ and m⁶ are preferably 0.

Examples of one of the preferred embodiments of the group represented by formula (2) include a group represented by formula (4).

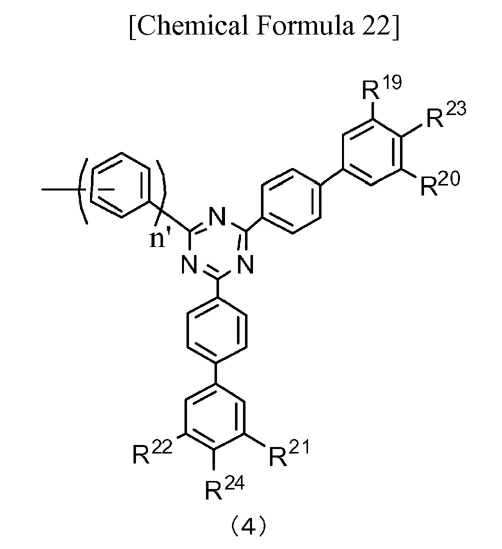

(In formula (4), n' represents 1 or 2; and R¹⁹ to R²⁴ are the same or different and each represents a hydrogen atom, an alkyl group, an alkenyl group, an alkoxy group, or a group represented by formula (3).)

In formula (4), n' represents 1 or 2, and is preferably 2.

In formula (4), R¹⁹ to R²⁴ are the same or different and each represents a hydrogen atom, an alkyl group, an alkenyl group, an alkoxy group, or a group represented by formula (3). Examples of the alkyl group, the alkenyl group, and the alkoxy group include the same groups as the examples of the alkyl group, the alkenyl group, and the alkoxy group, which are substituents which the above ring A¹ may have. R¹⁹ to R²⁴ are preferably a hydrogen atom, an alkyl group, an alkenyl group, and an alkynyl group, and more preferably a hydrogen atom and an alkyl group.

As the group represented by formula (4), from the viewpoint of ease of monomer synthesis, groups represented by the following formulae (4-1) to (4-8) are preferable, and the groups represented by formulae (4-1), (4-2), (4-7) and (4-8) are more preferable, and the group represented by formula (4-1) or formula (4-2) is further preferable.

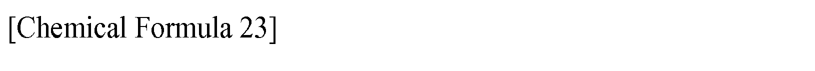

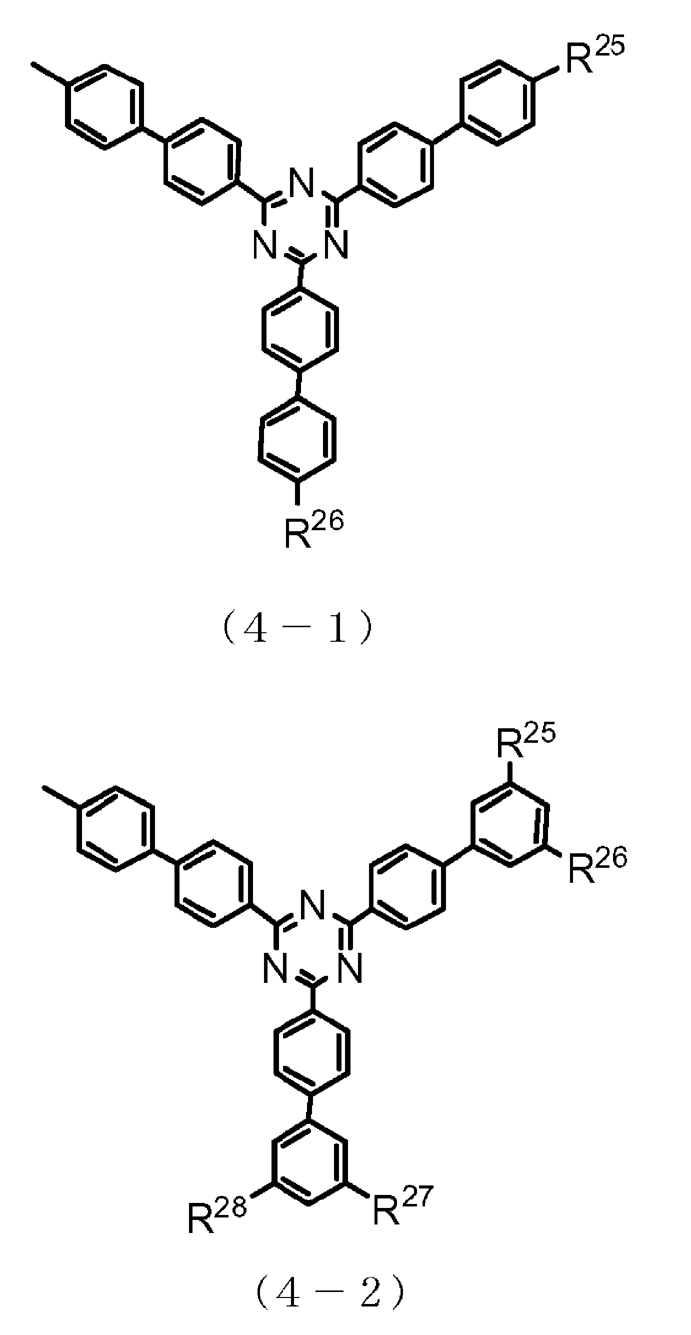

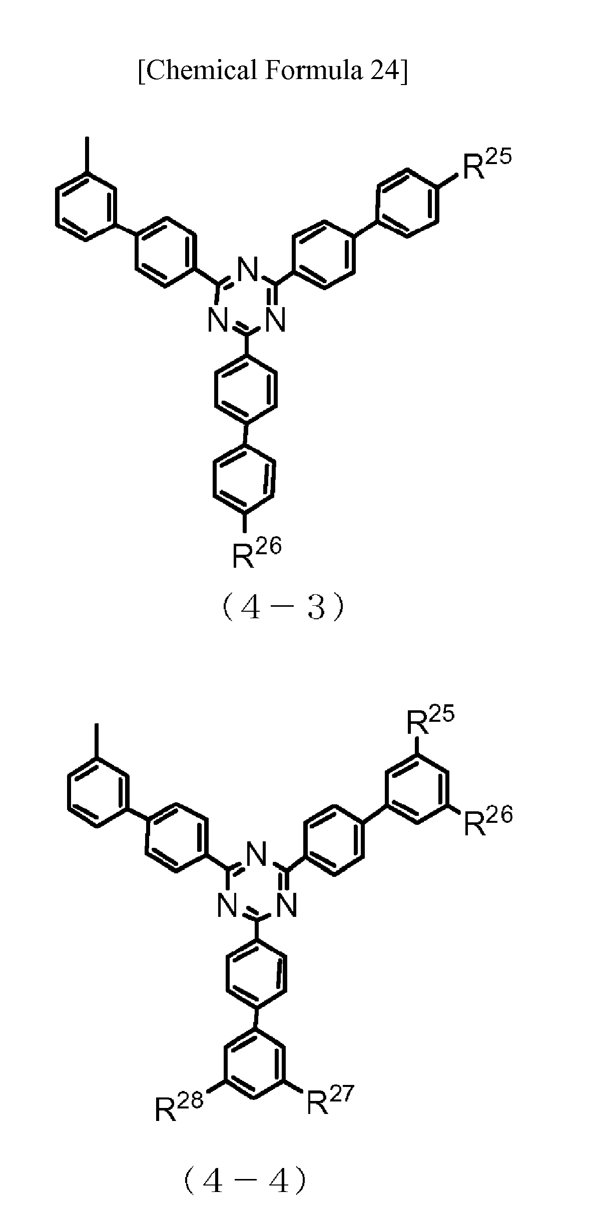

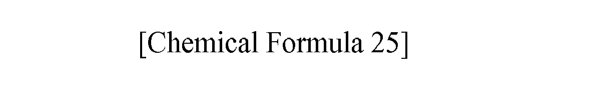

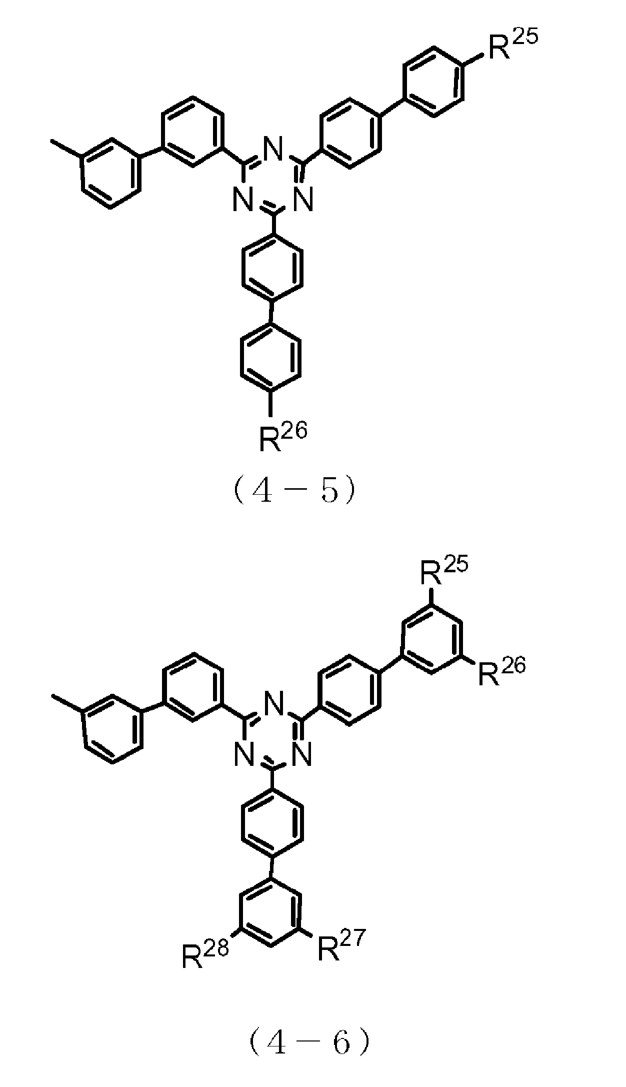

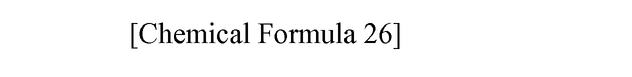

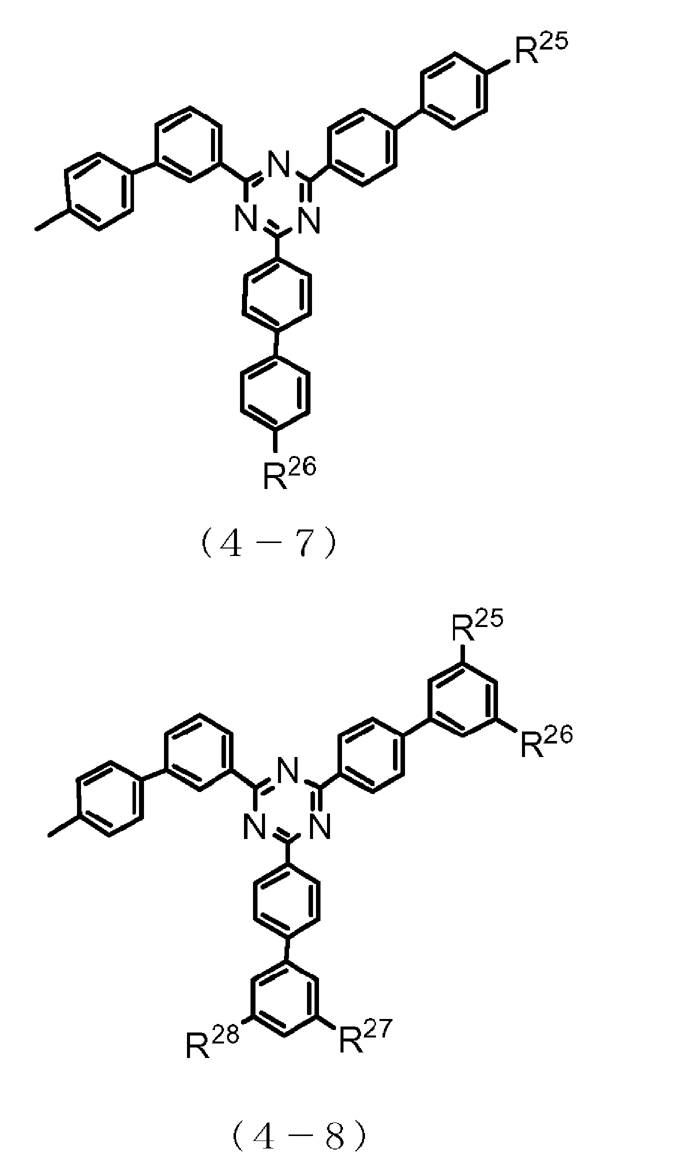

(In formula (4-1) to formula (4-8), R²⁵ to R²⁸ are the same or different and each represents an alkyl group, an alkenyl group, an alkoxy group, or a group represented by formula (3)).

(In formula (4-1) to formula (4-8), R²⁵ to R²⁸ are the same or different and are an alkyl group, an alkenyl group, an alkoxy group, or a group represented by formula (3). Examples of the alkyl group, the alkenyl group, and the alkoxy group include the same groups as the examples of the alkyl group, the alkenyl group, and the alkoxy group, which are substituents which the above ring A¹ may have.

R²⁵ to R²⁸ are preferably an alkyl group, and from the viewpoint of solubility and synthesis, an i-propyl group, a butyl group, an i-butyl group, an s-butyl group, a t-butyl group, a pentyl group, a hexyl group, a cyclohexyl group, a heptyl group, an octyl group, a 2-ethylhexyl group, a nonyl group, and a decyl group are more preferable, an i-propyl group, a butyl group, a t-butyl group, a pentyl group, a hexyl group, a heptyl group, and an octyl group are particularly preferable.

The repeating unit represented by formula (1) is preferably a repeating unit represented by formula (1-1) or a repeating unit represented by formula (1-2), from the viewpoint of ease of monomer synthesis or light-emitting properties when made into a device.

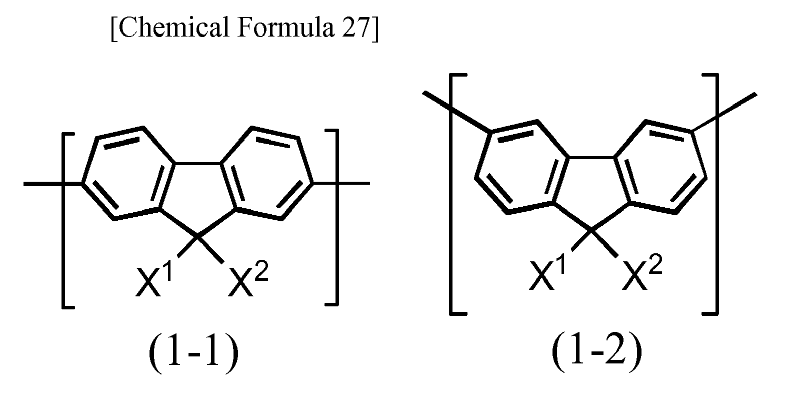

(The benzene rings in formula (1-1) and formula (1-2) are optionally substituted. X¹ and X² represent the same meanings as described above.)

Since the polymer compound of the present invention has a repeating unit represented by formula (1-0) (preferably formula (1)), an organic electroluminescent device produced using the polymer compound has a low emission starting voltage. In addition, the secondary effects of lowering the driving voltage at practical luminance and the driving voltage at maximum luminance can be expected.

### <Other Repeating Units>

From the viewpoint of charge transporting properties and light-emitting properties of a polymer compound, the polymer compound of the present invention further preferably has a repeating unit represented by the following formula (5) that is different from repeating units represented by formula (1-0) and formula (1), and more preferably has two or more types of repeating units represented by the following formula (5).

-Ar- (5)

(In formula (5), Ar represents an arylene group, a divalent heterocyclic group or a divalent aromatic amine residue.)

The arylene group is an atomic group obtained by removing two hydrogen atoms from an aromatic hydrocarbon and also includes groups having a condensed ring, and groups in which two or more of either or both independent benzene rings or/and condensed rings are bonded directly or via a vinylene group or the like. The arylene group is optionally substituted. As the above substituent, an alkyl group, an alkenyl group, an alkynyl group, an alkoxy group, an aryl group, a halogeno group, and a cyano group are preferable from the viewpoint of solubility, fluorescent properties, ease of synthesis, and properties when made into a device. Examples of the alkyl group, the alkenyl group, the alkynyl group, the alkoxy group, the aryl group, and a halogeno group include the same groups as the examples of the alkyl group, the alkenyl group, the alkynyl group, the alkoxy group, the aryl group, and the halogeno group, which are substituents which the above ring A¹ may have.

In the above arylene group, the carbon number of a portion excluding the substituent is generally from 6 to 60, and preferably from 6 to 20, and the total carbon number including the substituent is generally from 6 to 100.

Examples of the above arylene group include phenylene groups: formulae A1 to A3, naphthalenediyl groups: formulae A4 to A13, anthracene-diyl groups: formulae A14 to A19, biphenyl-diyl groups: formulae A20 to A25, terphenyl-diyl groups: formulae A26 to A28, condensed ring compound groups: formulae A29 to A35, fluorene-diyl groups: formulae A36 to A38, formulae A38-1 to A38-6, and benzofluorene-diyl: formulae A39 to A46, formulae A46-1 to A46-13. The following groups are optionally substituted.

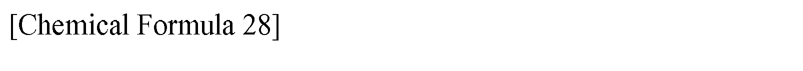

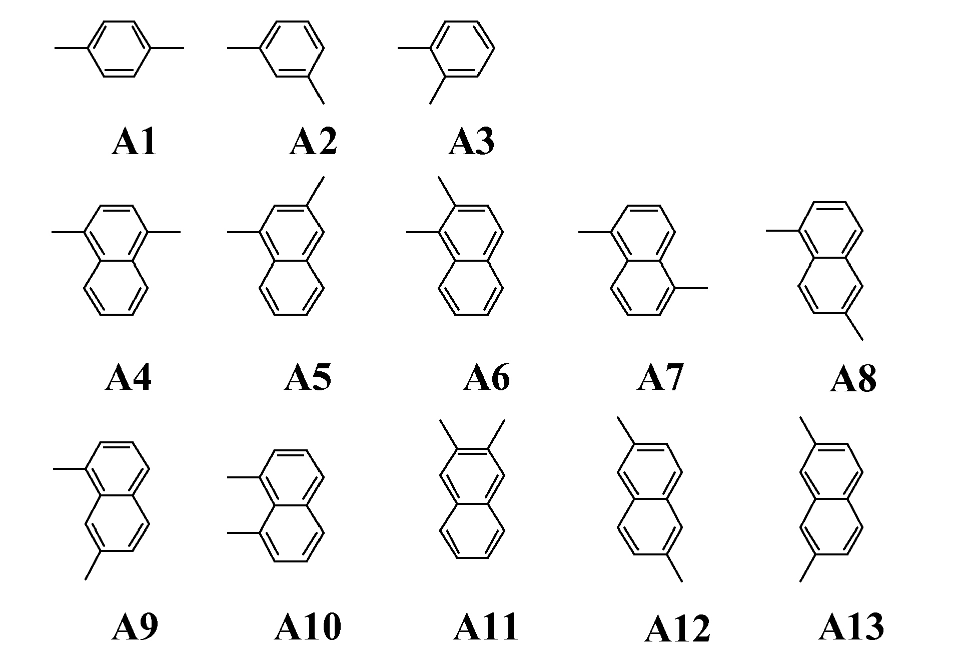

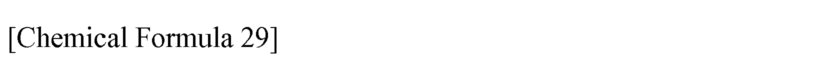

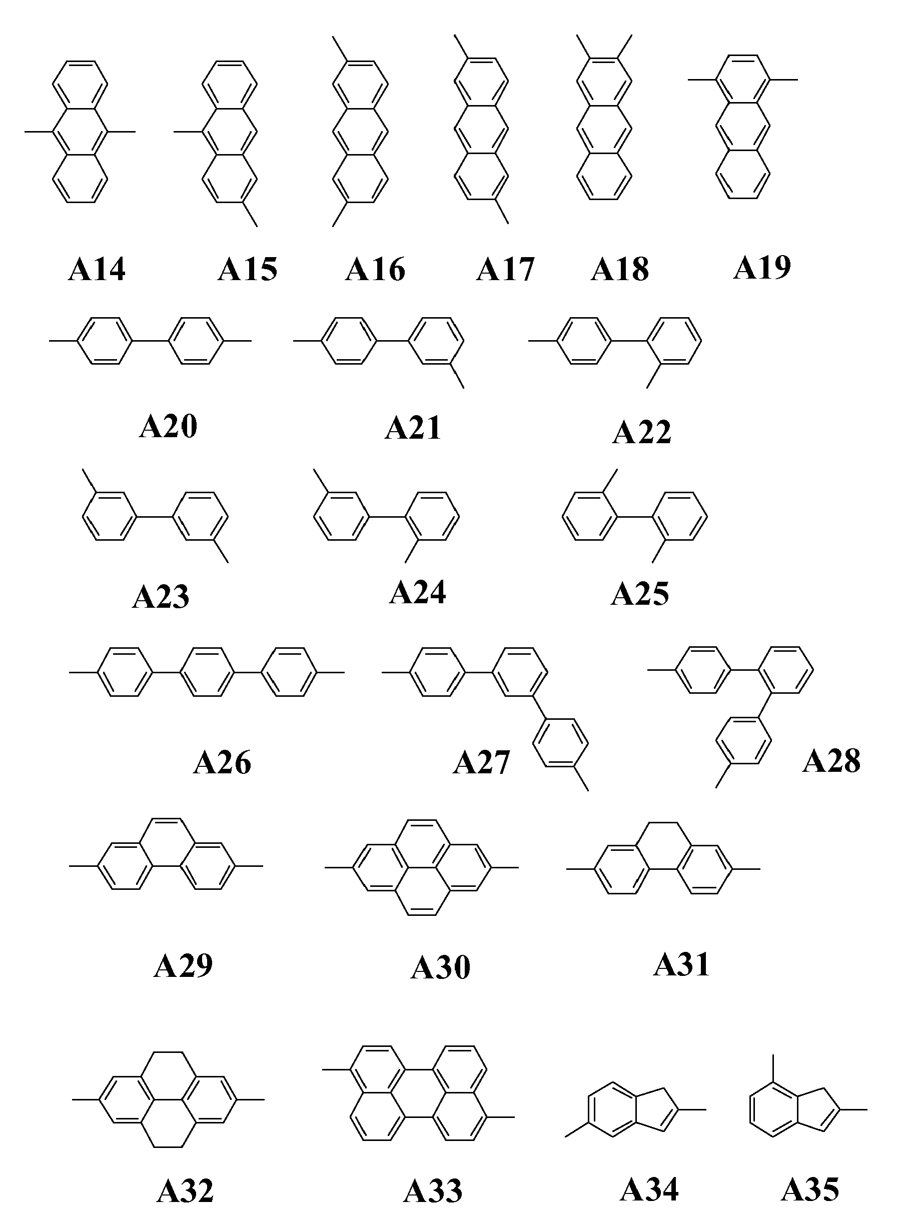

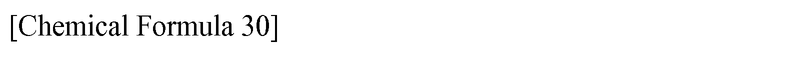

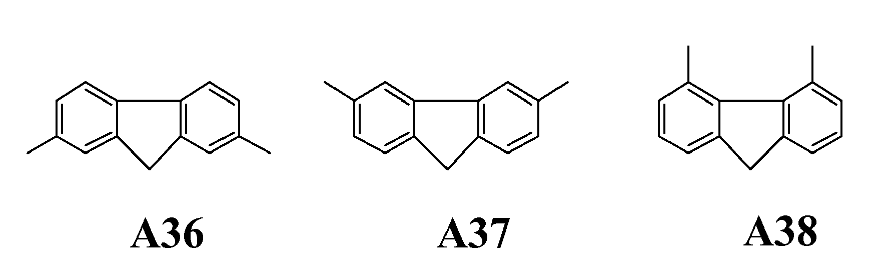

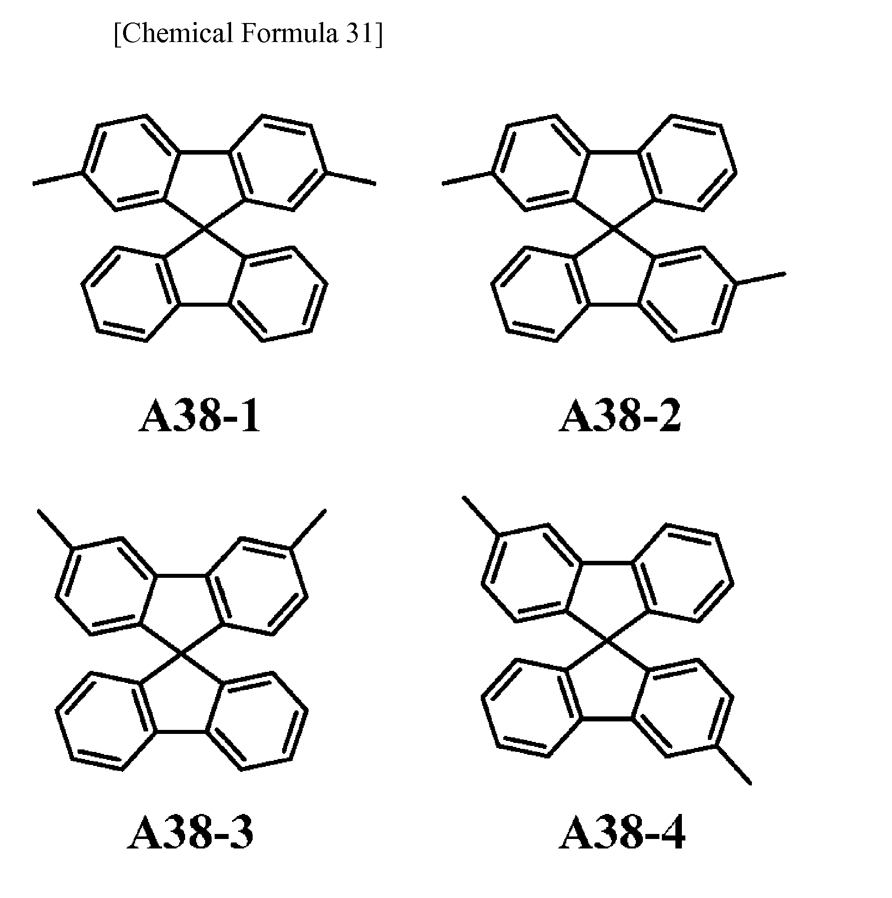

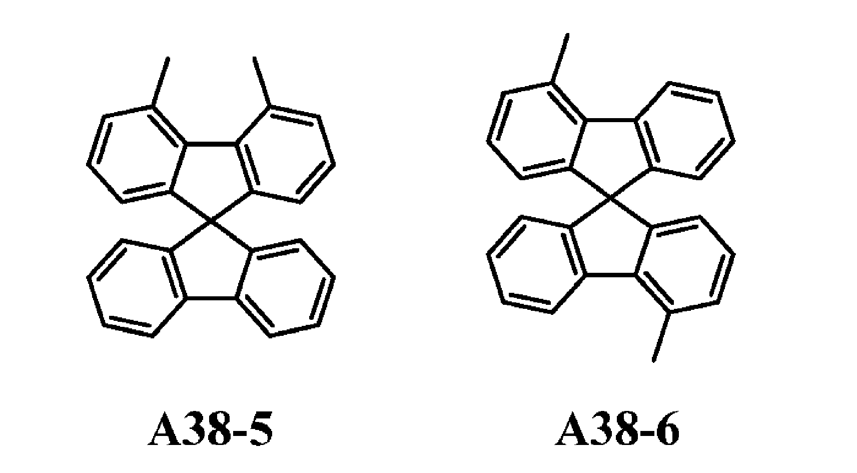

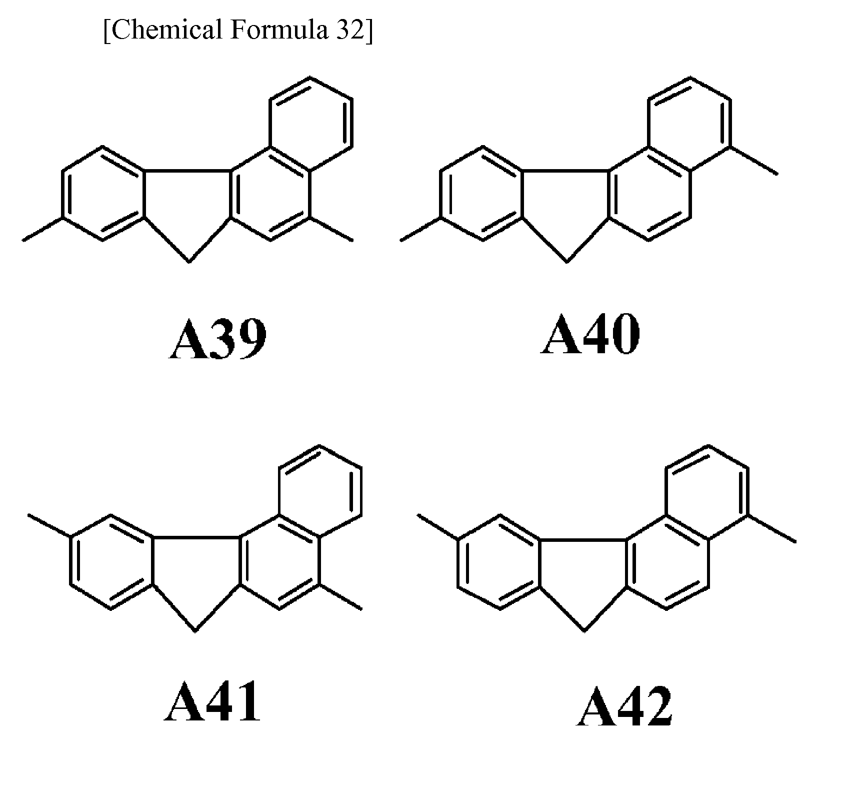

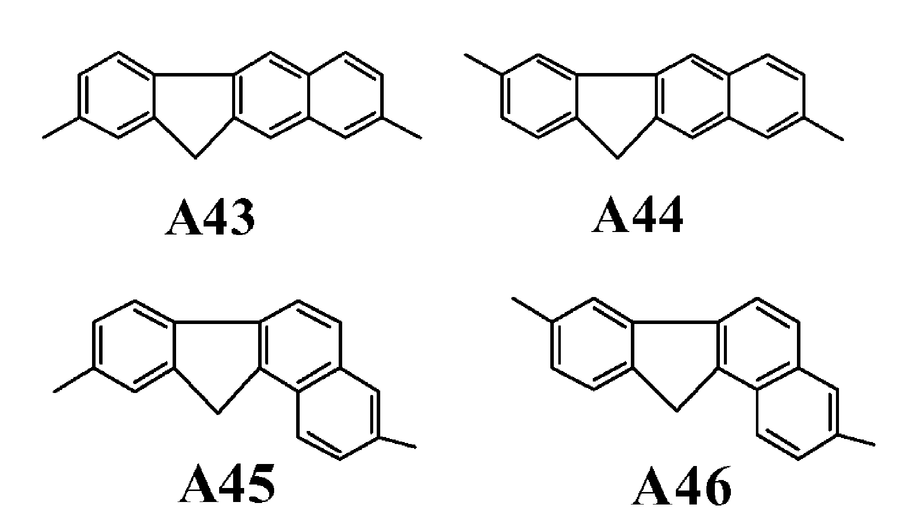

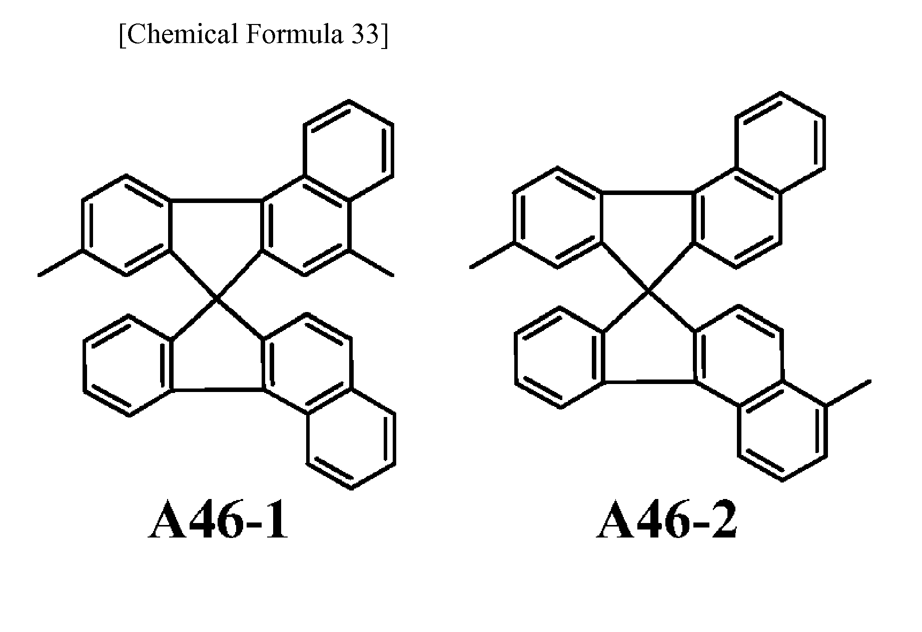

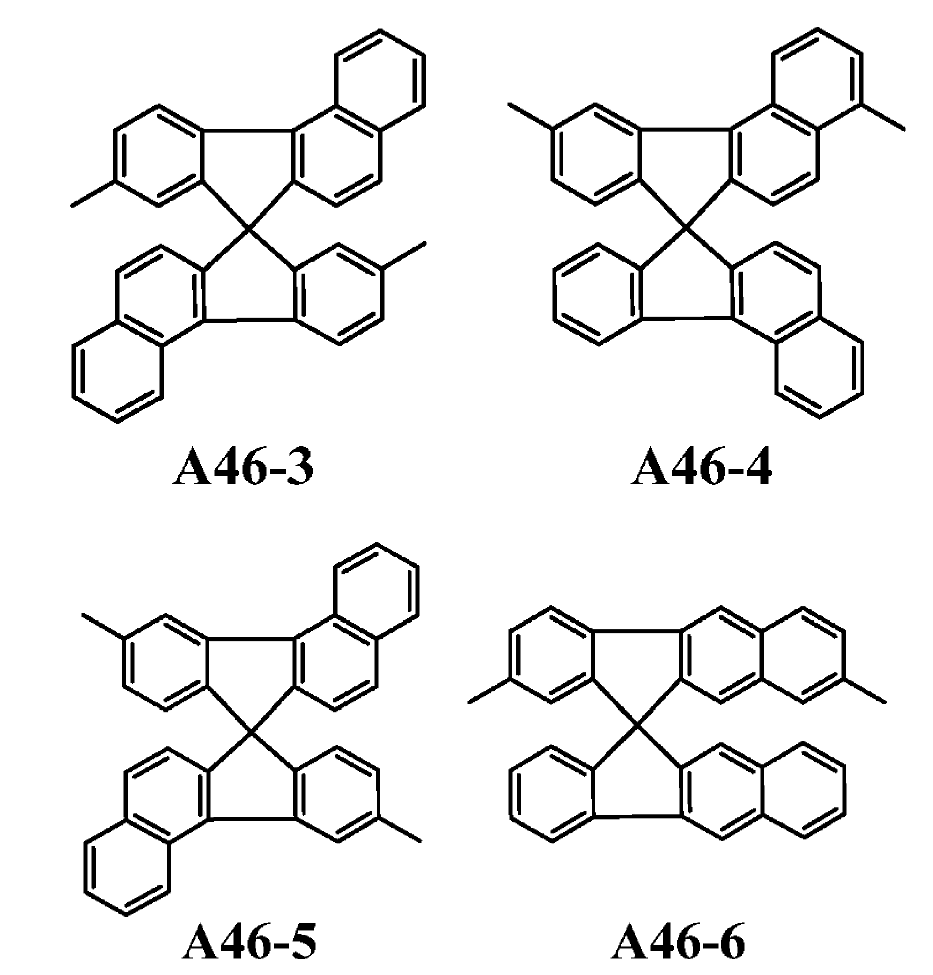

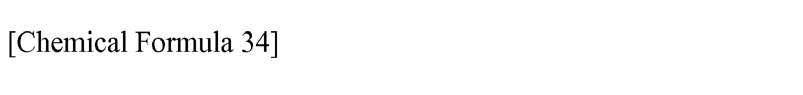

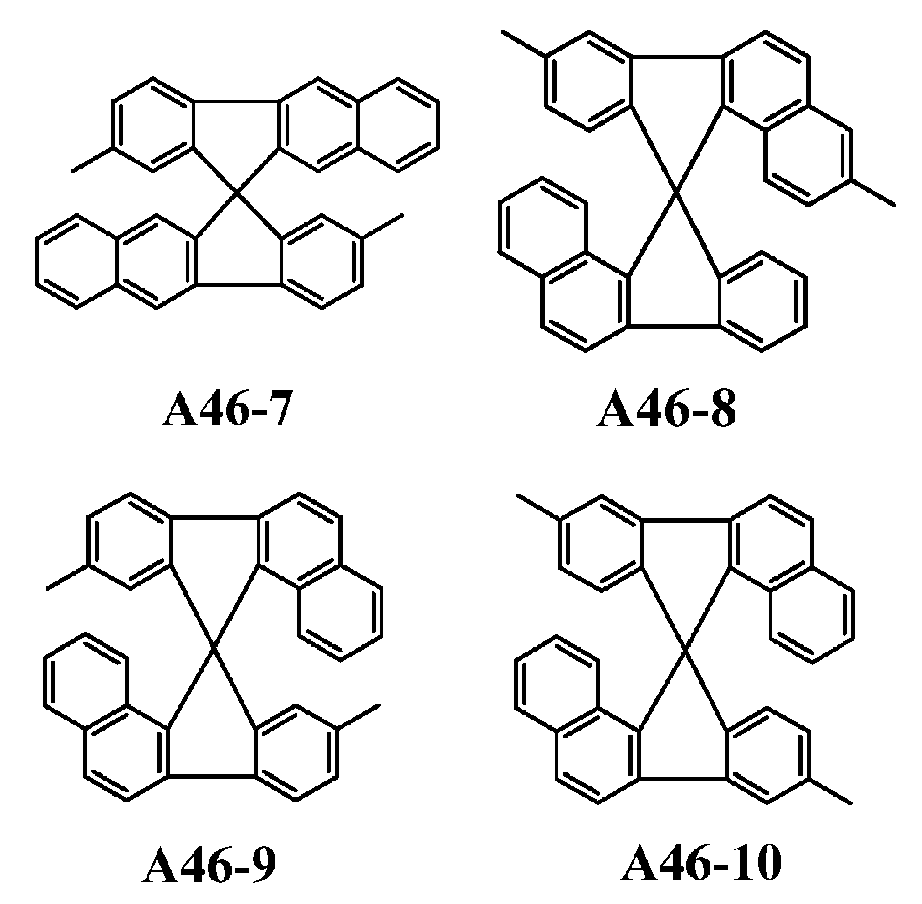

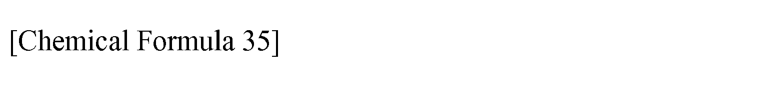

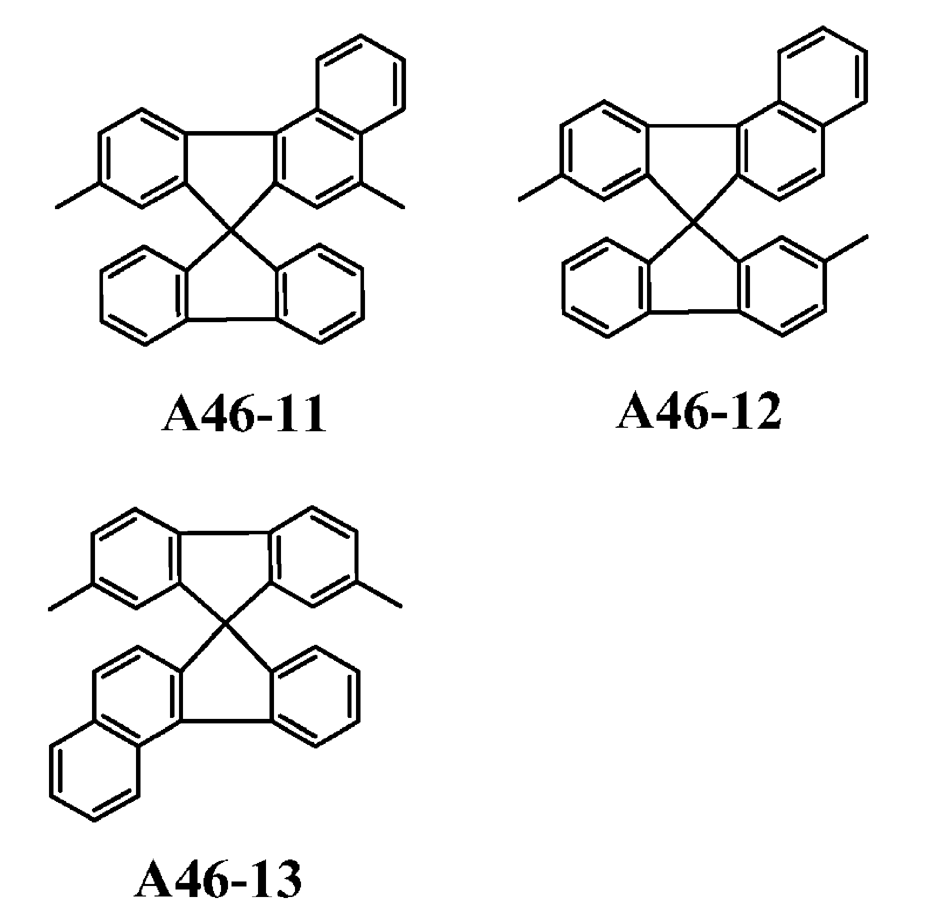

Among the arylene groups, from the viewpoint of polymer stability, the groups represented by the formulae A1 to A28 and the formulae A36 to A46 are preferable, the groups represented by the formulae A4 to A28 and the formulae A36 to A46 are more preferable, the groups represented by the formulae A4 to A19 and the formulae A36 to A38 are further preferable, and the groups represented by the formulae A36 to A46 are particularly preferable. In addition, from the viewpoint of ease of synthesis and light-emitting properties of the resulting compound, the groups represented by the formulae A1 to A3, the formulae A20 to A28 and the formulae A36 to A46 are preferable, the groups represented by the formula A1, the formula A2, the formula A20, the formula A21, the formula A23, the formula A26, the formula A27, the formula A3 6, and the formula A37 are more preferable, and the groups represented by the formula A1, the formula A2, the formula A3 6, and the formula A37 are further preferable. These arylene groups are optionally substituted, and as the substituent which the arylene group may have, an alkyl group, an alkenyl group, an alkynyl group, an alkoxy group, an aryl group, a halogeno group, and a cyano group are preferable, an alkyl group, an alkoxy group and an aryl group are more preferable, and an alkyl group is further preferable, from the viewpoint of solubility, fluorescent properties and ease of synthesis and properties when made into a device of the resulting compound.

The divalent heterocyclic group refers to an atomic group remaining after removing two hydrogen atoms from a heterocyclic compound, and is optionally substituted. The above heterocyclic compound refers to organic compounds having a cyclic structure in which elements constituting the ring include not only a carbon atom, but also a heteroatom such as an oxygen atom, a sulfur atom, a nitrogen atom, a phosphorus atom, a boron atom, and an arsenic atom, contained in the ring. As the divalent heterocyclic group, divalent aromatic heterocyclic groups are preferable. As the above substituent, an alkyl group, an alkenyl group, an alkynyl group, an alkoxy group, an aryl group, a halogeno group, and a cyano group are preferable from the viewpoint of solubility, fluorescent properties, ease of synthesis and properties when made into a device of the resulting compound. Specific examples of the alkyl group, the alkenyl group, the alkynyl group, the alkoxy group, the aryl group, and the halogeno group include the same groups as the examples of the alkyl group, the alkenyl group, the alkynyl group, the alkoxy group, the aryl group, and the halogeno group, which are substituents which the above ring A¹ may have.

The carbon number of the divalent heterocyclic group excluding a portion excluding the substituent is generally from 3 to 60, and the total carbon number including the substituent is generally from 3 to 100.

Examples of the divalent heterocyclic group include the following groups. The following groups are optionally substituted.

As a divalent heterocyclic group containing a nitrogen atom as a heteroatom, pyridine-diyl groups: formulae B1 to B4, diazaphenylene groups: formulae B5 to B8, triazine-diyl group: formula B9, quinoline-diyl groups: formulae B10 to B14, quinoxaline-diyl groups: formulae B15 to B19, acridinediyl groups: formulae B20 to B23, and phenanthrolinediyl groups: formulae B24 and B25.

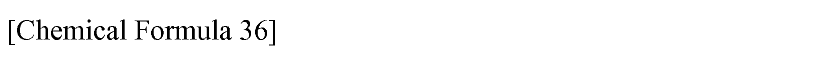

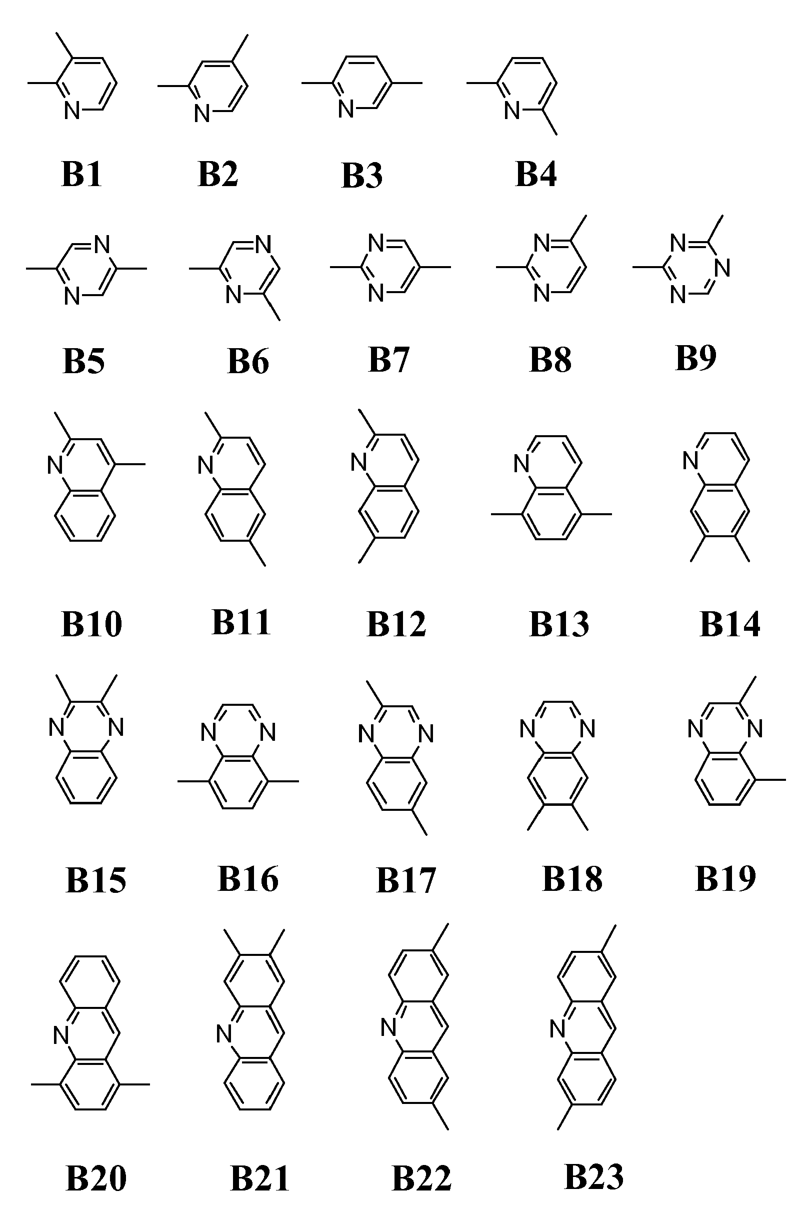

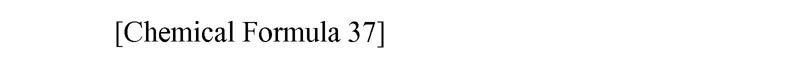

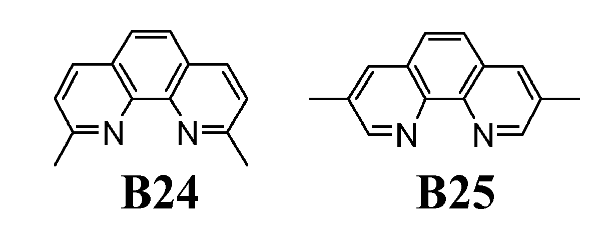

Groups containing an oxygen atom, a sulfur atom, a nitrogen atom, a silicon atom or the like as a heteroatom and having a fluorene structure: formulae B26 to B33.

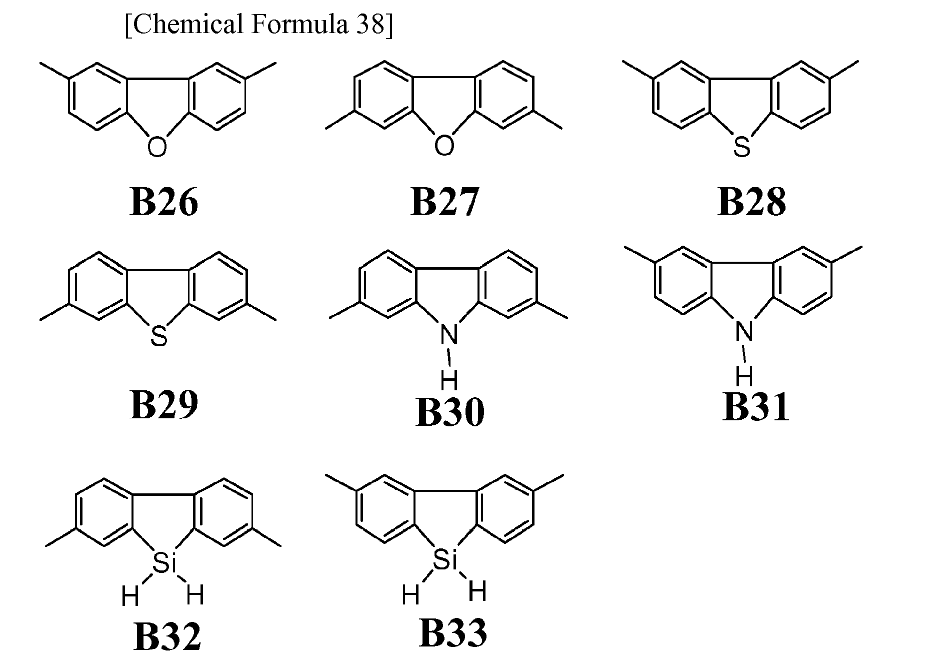

5-membered ring heterocyclic groups containing an oxygen atom, a sulfur atom, a nitrogen atom, a silicon atom or the like as a heteroatom: formulae B34 to B37.

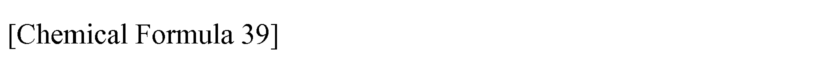

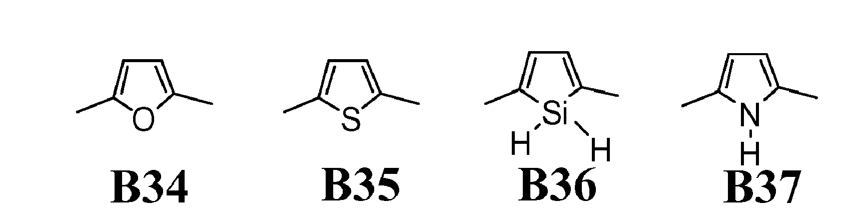

5-membered condensed heterocyclic groups containing an oxygen atom, a sulfur atom, a nitrogen atom, a silicon atom or the like as a heteroatom: formulae B38 to B47.

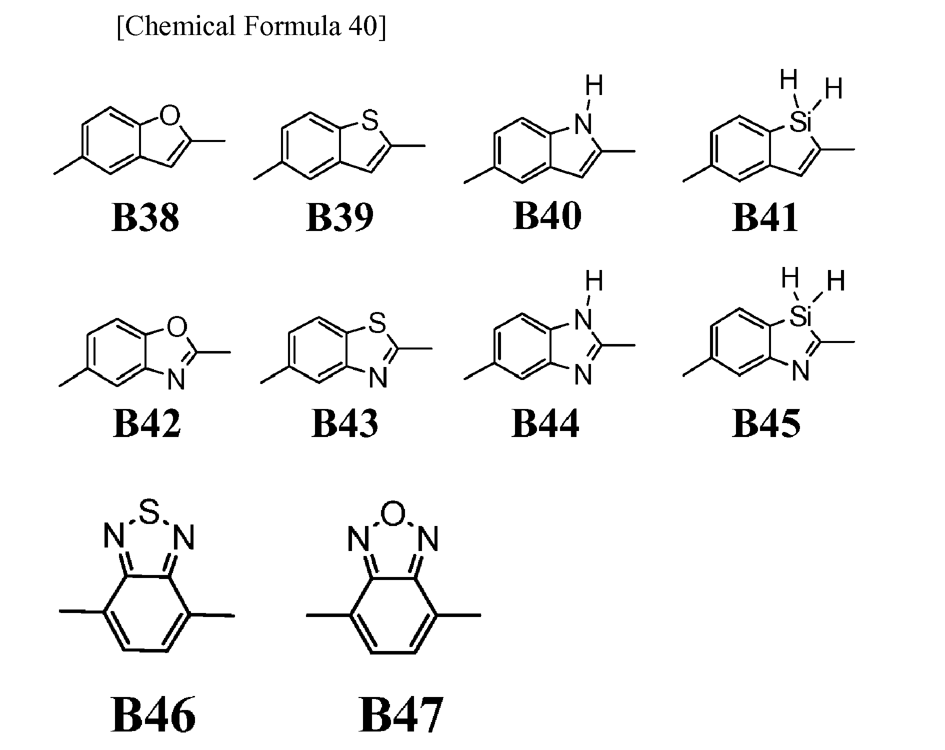

5-membered ring heterocyclic groups containing an oxygen atom, a sulfur atom, a nitrogen atom, a silicon atom or the like as a heteroatom, which is bonded at the α position of the heteroatom to form a dimer or an oligomer: formulae B48 and B49.

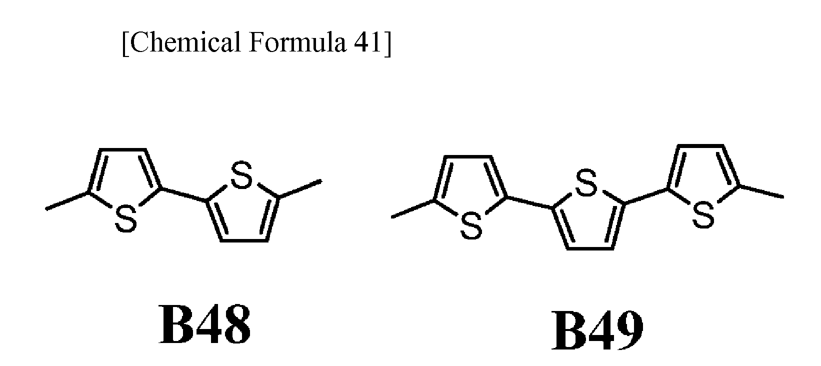

5-membered ring heterocyclic groups containing an oxygen atom, a sulfur atom, a nitrogen atom, a silicon atom or the like as a heteroatom, which is bonded to a phenyl group at the α position of the heteroatom: formulae B50 to B55.

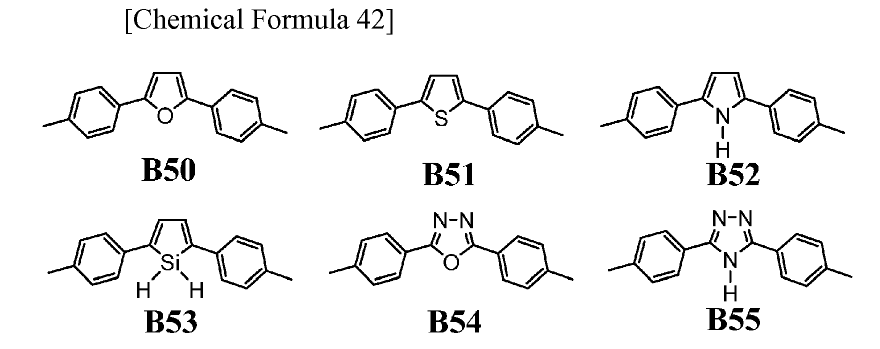

5-membered condensed heterocyclic groups containing an oxygen atom, a sulfur atom, a nitrogen atom, or the like as a heteroatom, and substituted by a phenyl group, a furyl group or a thienyl group: formulae B56 to B61.

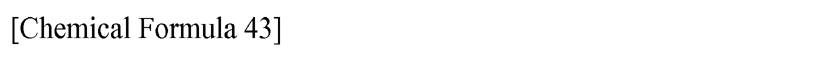

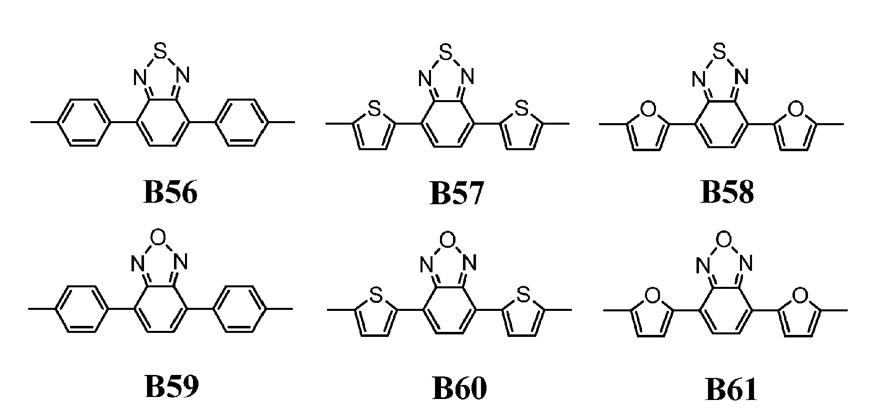

6-membered ring heterocyclic groups containing an oxygen atom, a nitrogen atom, or the like as a heteroatom: formulae B62 to B65.

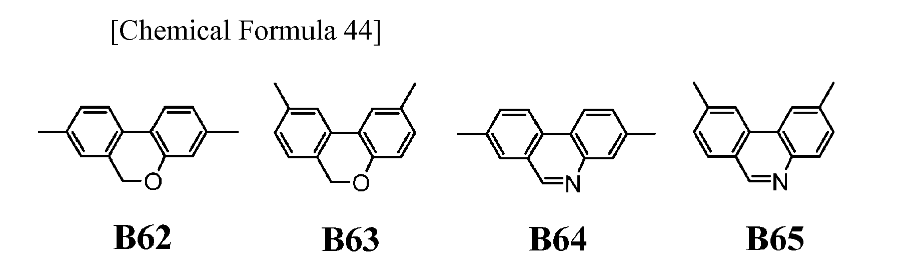

Among the divalent heterocyclic groups, from the viewpoint of charge transporting properties and light-emitting properties, the groups represented by the formulae B5 to B9, the formulae B24 to B37 and the formulae B42 to B61 are preferable, the groups represented by the formulae B5 to B9, the formulae B26 to B31, the formulae B42 to B44 and the formulae B50 to B54 are more preferable, the groups represented by the formulae B5 to B9, the formula B30, the formula 31, the formula B53, and the formula B54 are further preferable, and the groups represented by the formulae B5 to B9, the formula B30, and the formula B31 are particularly preferable. In addition, from the viewpoint of polymer synthesis, as the divalent heterocyclic groups, the groups represented by the formulae B1 to B9, the formulae B26 to B31, and the formulae B46 to B54 are preferable, and the groups represented by the formulae B3 to B9, the formula B30, and the formula B31 are more preferable.

The divalent aromatic amine residue refers to an atomic group remaining after removing two hydrogen atoms from an aromatic amine, and is optionally substituted, and the carbon number excluding a portion excluding the substituent is generally from 5 to 100, and preferably from 15 to 60. As the above substituent, an alkyl group, an alkenyl group, an alkynyl group, an alkoxy group, an aryl group, a halogeno group, and a cyano group are preferable from the viewpoint of solubility, fluorescent properties, ease of synthesis and properties when made into a device of the resulting compound. Examples of the alkyl group, the alkenyl group, the alkynyl group, the alkoxy group, the aryl group, and the halogeno group include the same groups as the examples of the alkyl group, the alkenyl group, the alkynyl group, the alkoxy group, the aryl group, and the halogeno group, which are substituents which the above ring A¹ may have.

Examples of the divalent aromatic amine residue include divalent groups represented by the following formulae C 1 to C31. The following groups are optionally substituted.

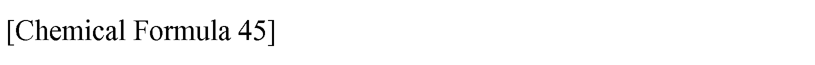

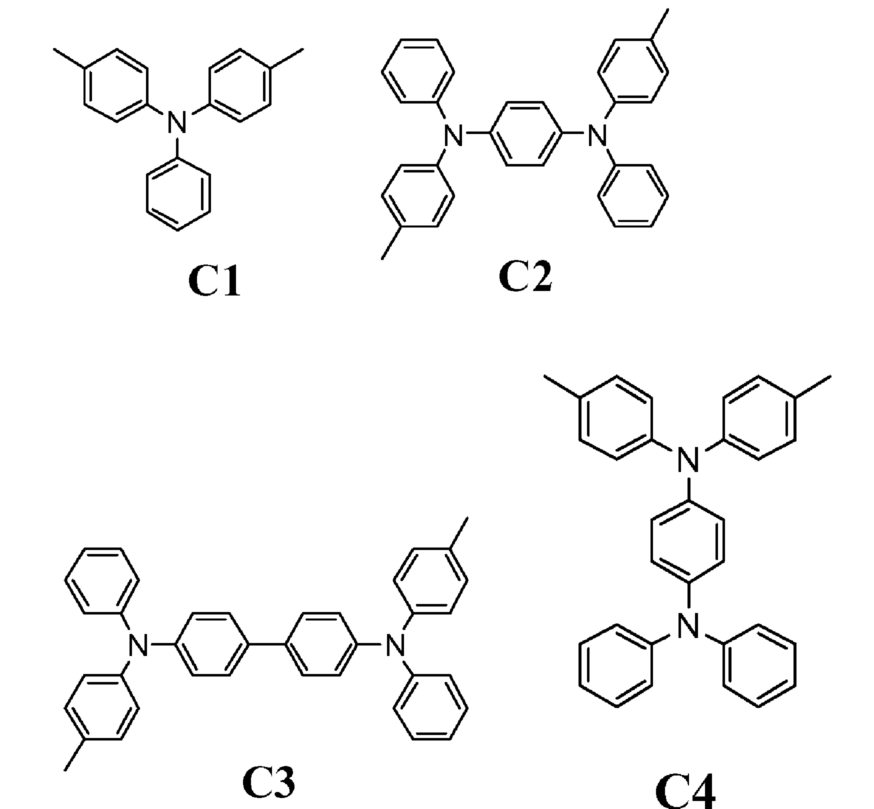

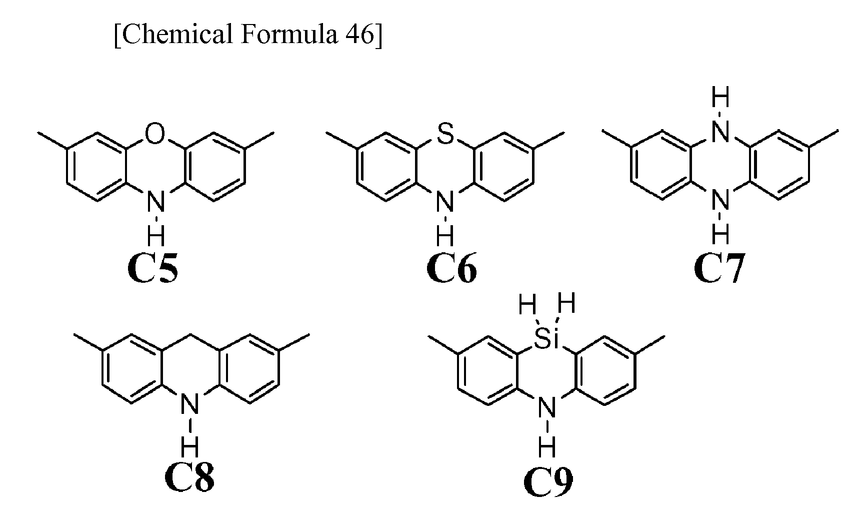

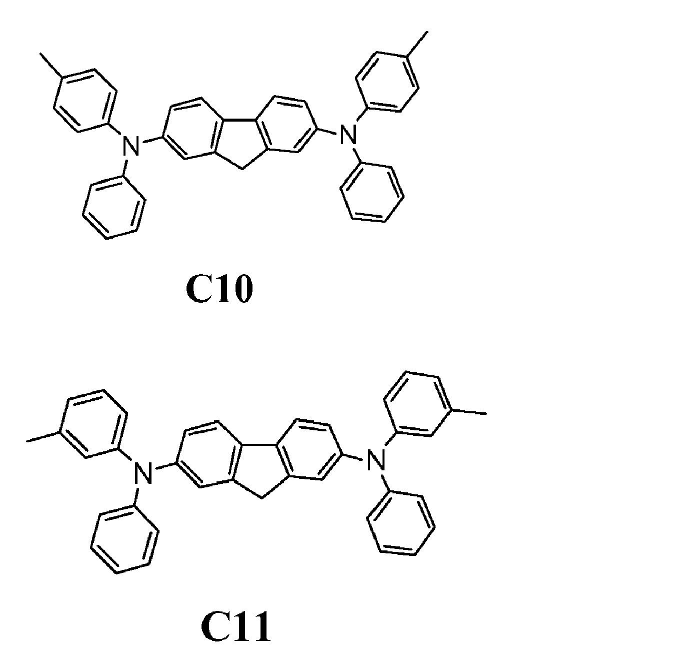

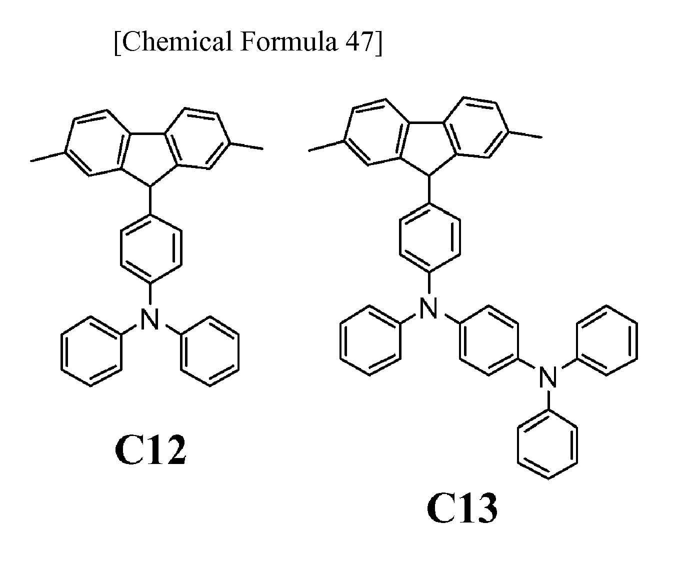

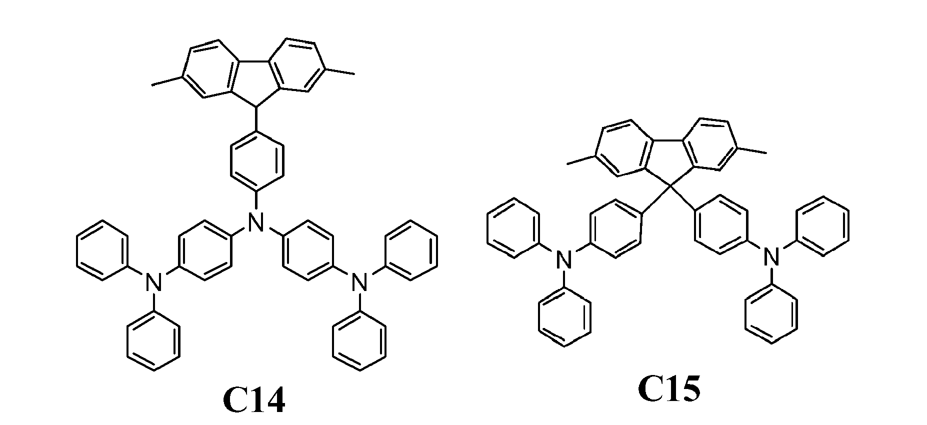

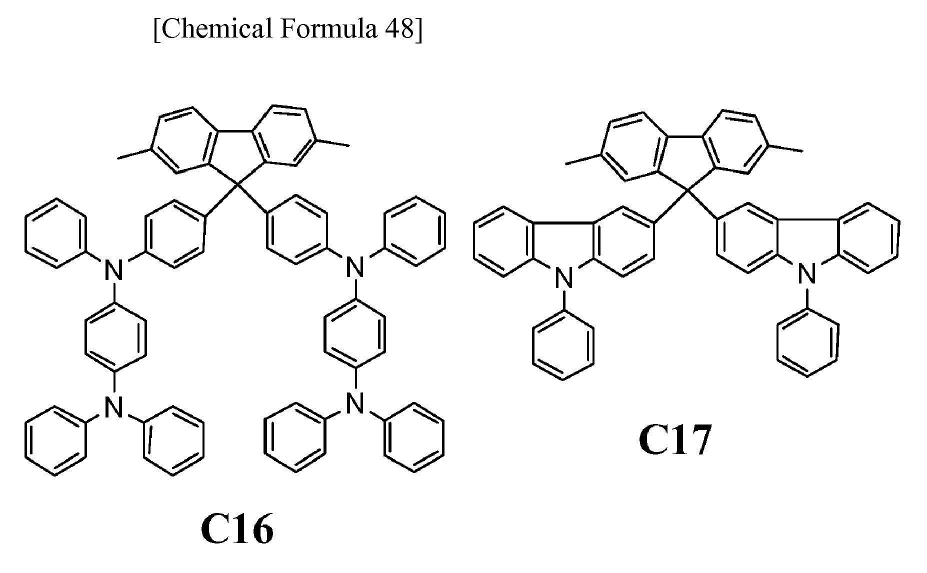

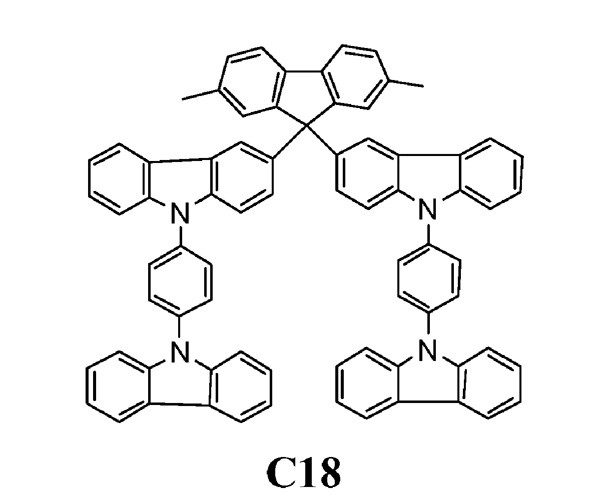

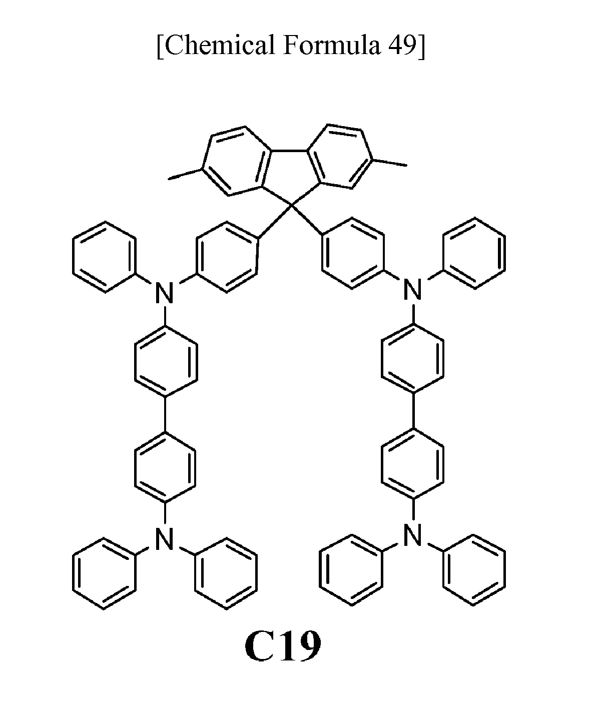

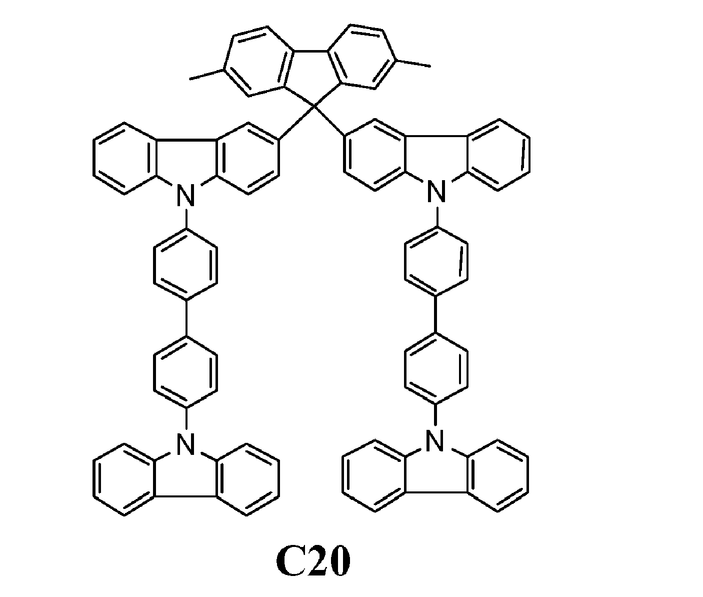

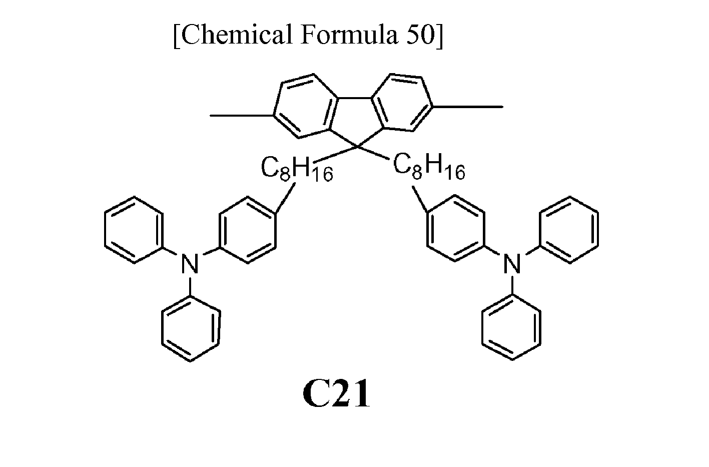

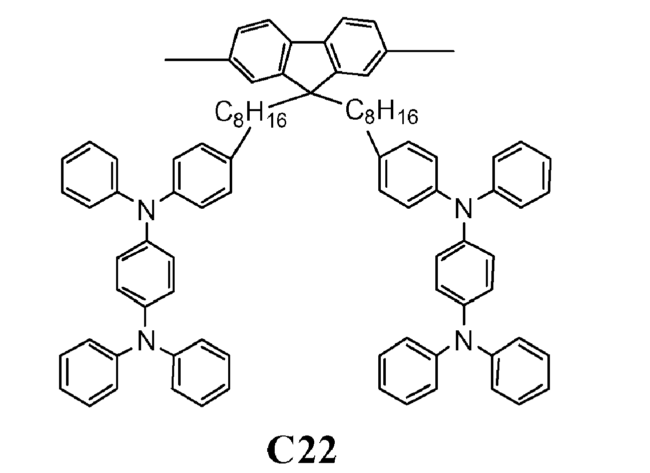

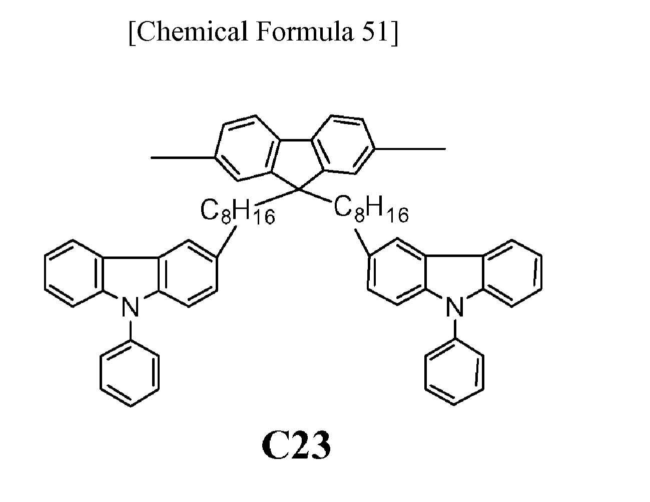

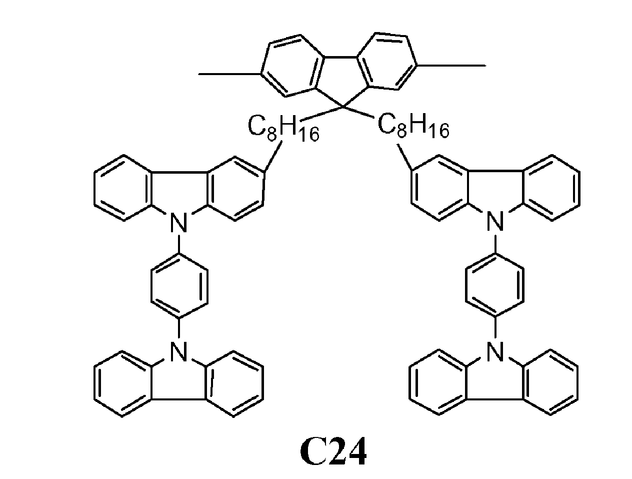

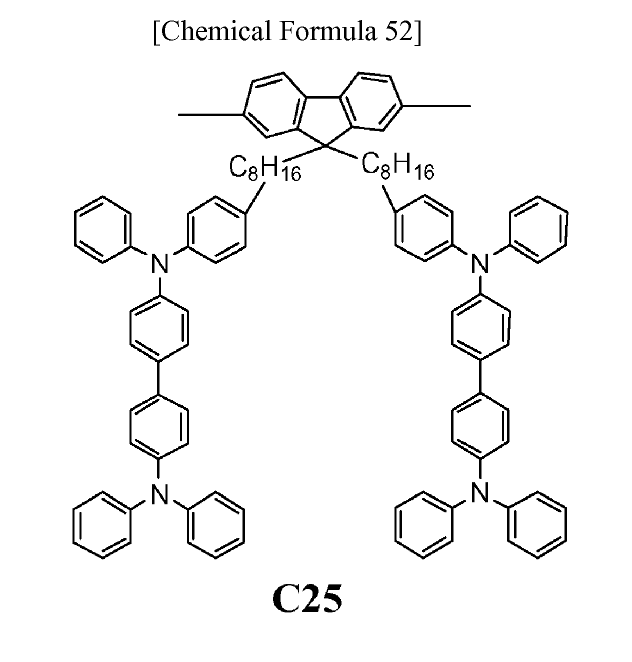

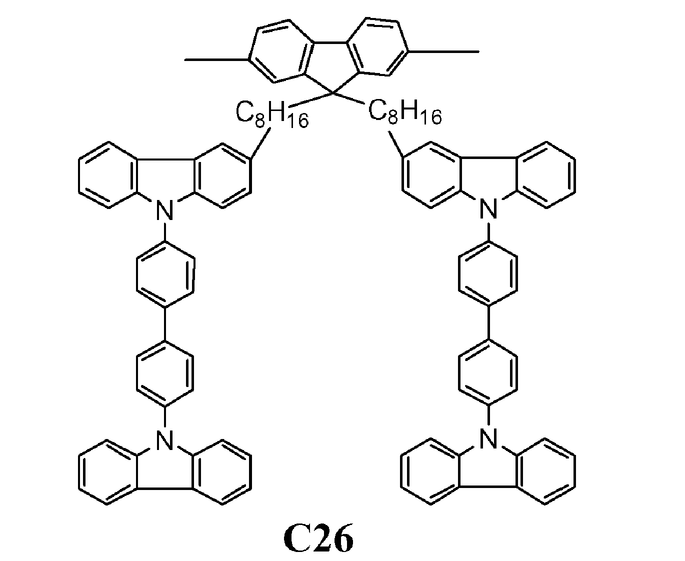

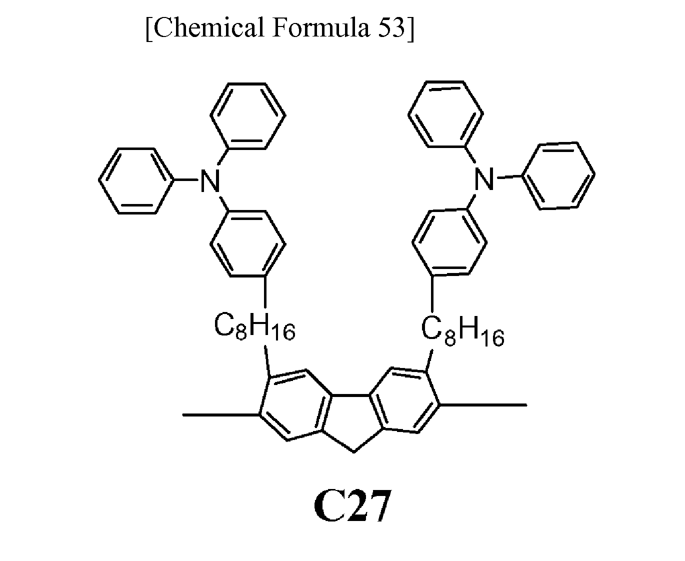

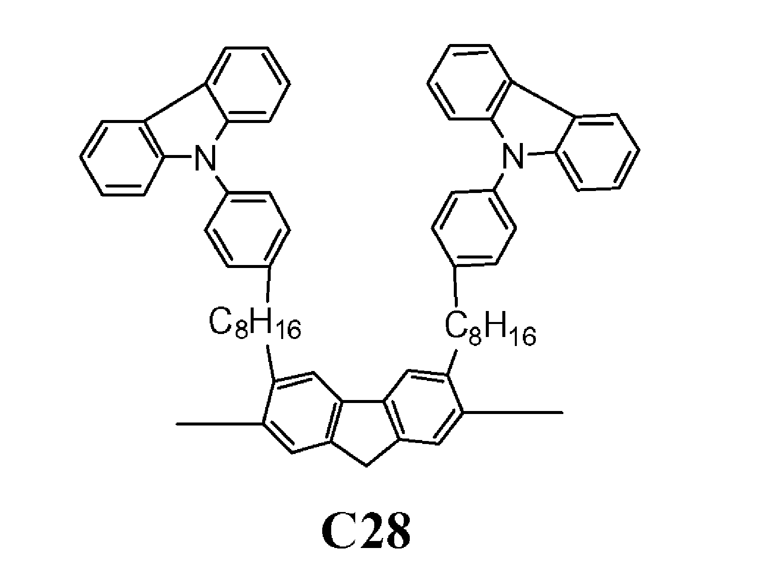

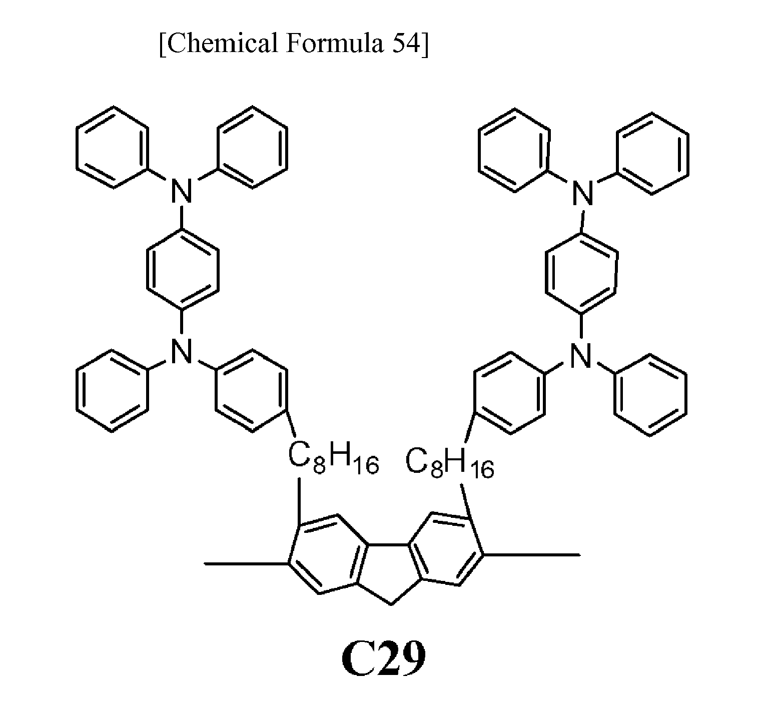

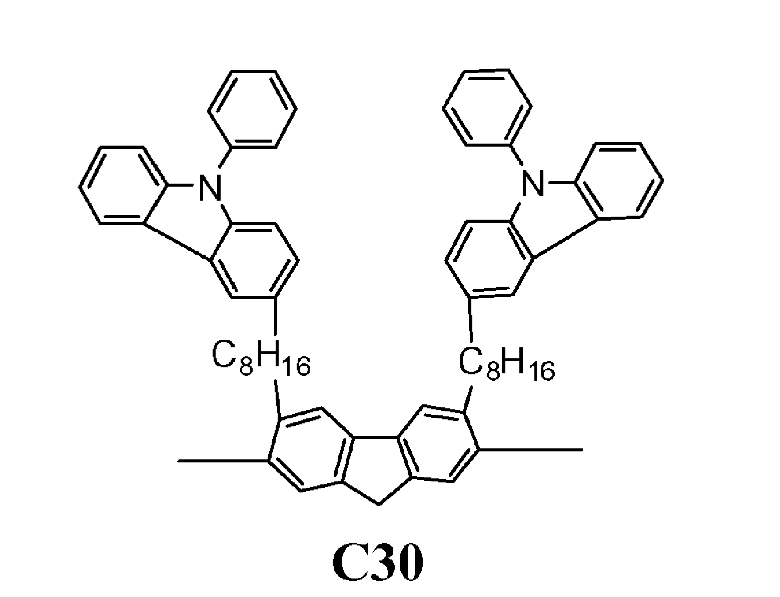

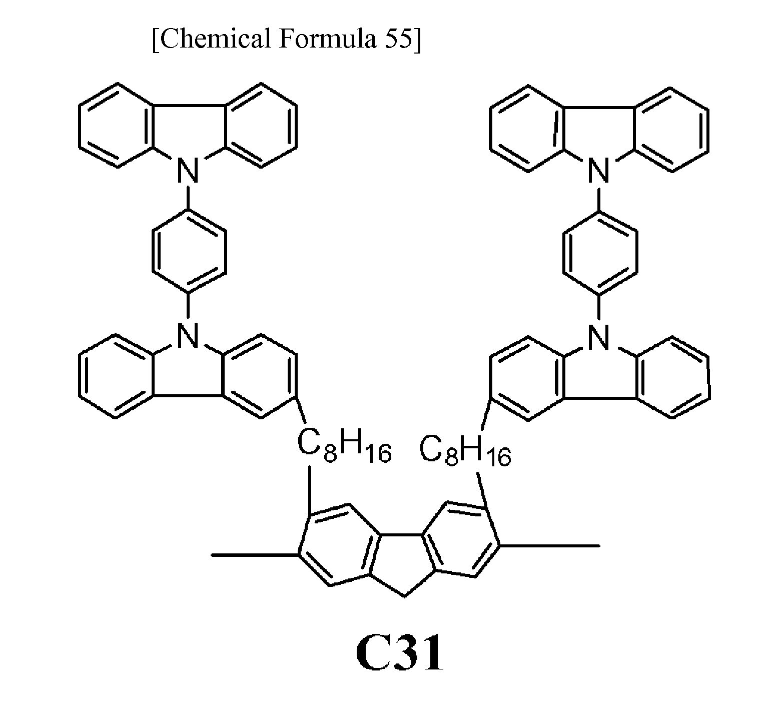

Among the divalent aromatic amine residues, the groups represented by the formulae C1 to C4 and the formulae C10 to C26 are preferable from the viewpoint of hole transporting properties, and the groups represented by the formulae C1 to C5, the formula C12, and the formulae C14 to C21 are preferable from the viewpoint of ease of synthesis of the resulting compound.

When the polymer compound of the present invention contains only 1 type of the repeating unit represented by formula (5), Ar is preferably an arylene group. When the polymer compound of the present invention contains 2 or more types of the repeating unit represented by formula (5), at least 1 type is preferably an arylene group, and more preferably a combination of only arylene groups or a combination of an arylene group and an aromatic amine residue.

The polymer compound of the present invention has a polystyrene-equivalent number average molecular weight of preferably 1 × 10³ to 1 × 10⁸ and more preferably 1 × 10³ to 1 × 10⁷, and a polystyrene-equivalent weight average molecular weight of preferably 1 × 10³ to 1 × 10⁸ and more preferably 1 × 10³ to 1 × 10⁷, from the viewpoint of life property of a light-emitting device when used in the production of the light-emitting device. The number average molecular weight and the weight average molecular weight can be determined using, for example, size exclusion chromatography (SEC).

The polymer compound of the present invention may be any of an alternating copolymer, a random copolymer, a block copolymer and a graft copolymer, and may also be a polymer compound having an intermediate structure between them, for example, a random copolymer having blocking properties. As the polymer compound of the present invention, from the viewpoint of fluorescent or phosphorescent quantum yield, a random copolymer having blocking properties, a block copolymer and a graft copolymer are more preferable than a complete random copolymer. The polymer compound of the present invention also includes a compound having a branched main chain and more than three terminals, and a dendrimer.

In an end group of the polymer compound of the present invention, if a polymerization active group remains intact, light-emitting properties and lifetime of the resulting light-emitting device may decrease when used in the production of the light-emitting device, and thus, the end group may be protected by a stable group. As the above end group, a group having a conjugation bond continuous with a conjugation structure of the main chain is preferable, and examples include groups bonding to an aryl group or monovalent heterocyclic group via a carbon-carbon bond and also include substituents described in Chemical Formula 10 in Japanese Patent Application Laid-Open Publication No. 9-45478.

Examples of the polymer compound of the present invention include the following copolymers <EX1> to <EX3>.

<EX1> An alternating copolymer comprising a repeating unit represented by 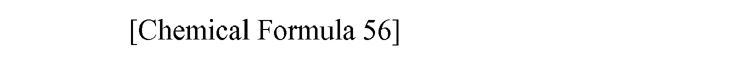 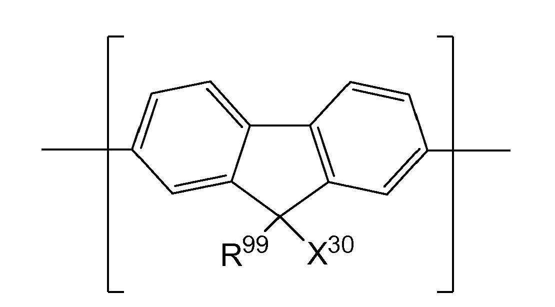 in an amount of 50% by mol as the stoichiometric ratio, comprising a repeating unit represented by 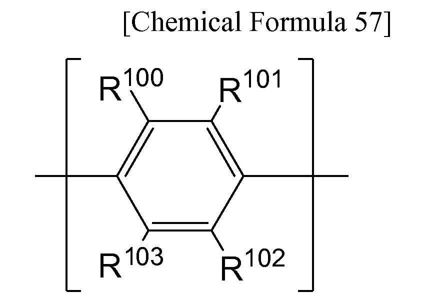 or a repeating unit represented by 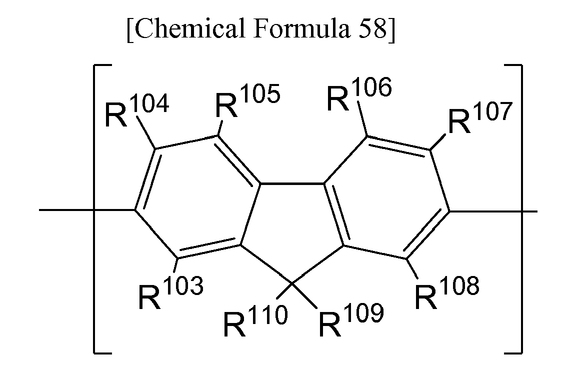 in an amount of 50% by mol as the stoichiometric ratio.

(In the formulae, X³⁰ represents a group represented by formula (2-0); and R⁹⁹ to R¹¹⁰ are the same or different and each represents a hydrogen atom, an alkyl group, or an aryl group.)

<EX2> A copolymer comprising a repeating unit represented by 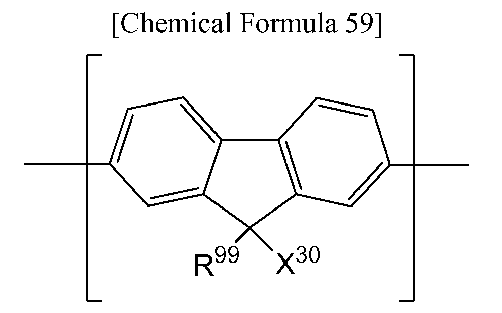 in an amount of 1 to 50% by mol as the stoichiometric ratio, comprising a repeating unit represented by 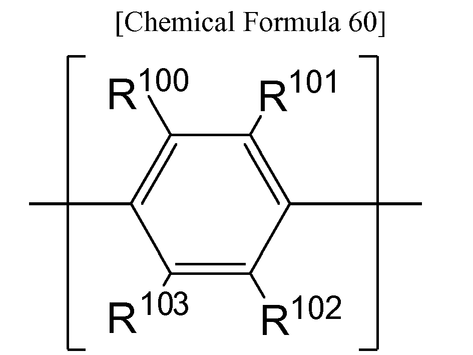 in an amount of 1 to 50% by mol as the stoichiometric ratio, and comprising a repeating unit represented by 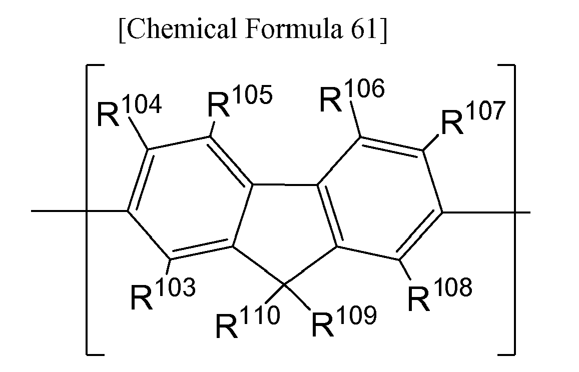 in an amount of 1 to 50% by mol as the stoichiometric ratio.

(In the formulae, X³⁰ and R⁹⁹ to R¹¹⁰ represent the same meanings as described above.)

<EX3> A copolymer comprising a repeating unit represented by 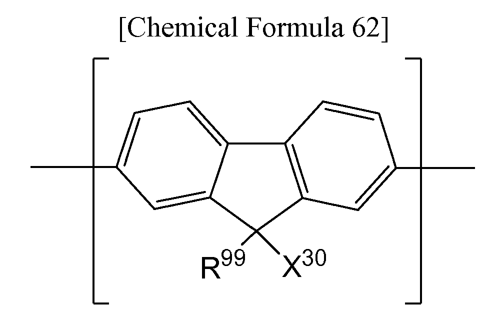 in an amount of 1 to 30% by mol as the stoichiometric ratio, comprising a repeating unit represented by 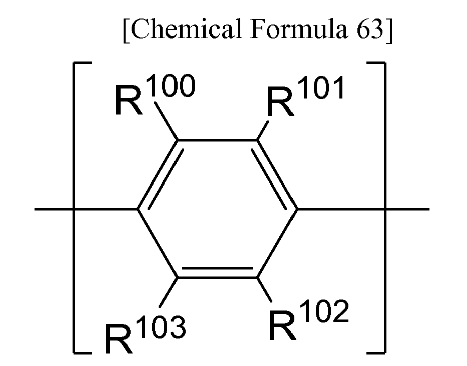 in an amount of 1 to 50% by mol as the stoichiometric ratio, comprising a repeating unit represented by 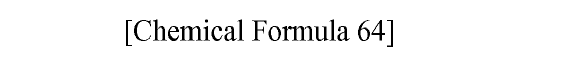 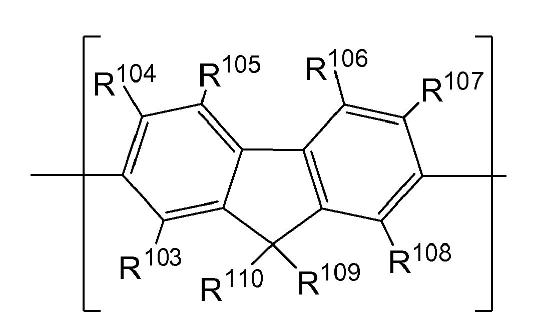 in an amount of 1 to 50% by mol as the stoichiometric ratio, comprising a repeating unit represented by 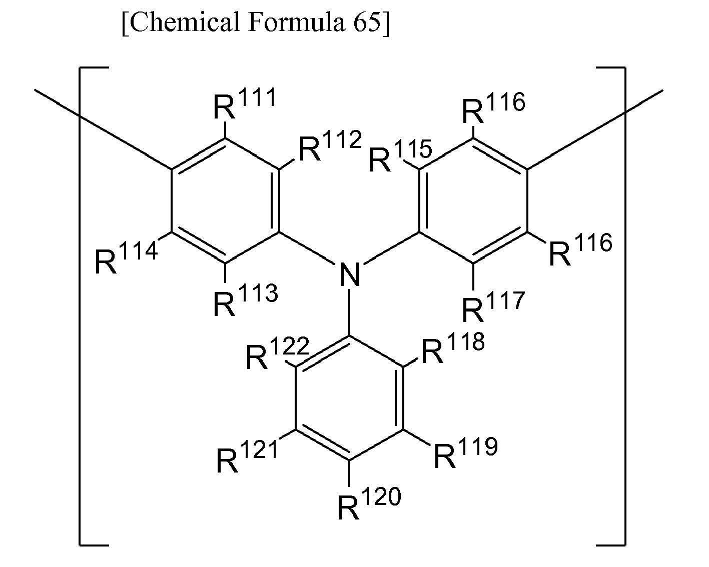 in an amount of 0.1 to 30% by mol as the stoichiometric ratio.

(In the formulae, X³⁰ and R⁹⁹ to R¹¹⁰ represent the same meanings as described above; and R¹¹¹ to R¹²² are the same or different and each represents a hydrogen atom, an alkyl group, or an aryl group.)

<Method for Producing Polymer Compound> Next, a method for producing the polymer compound of the present invention is described.

The polymer compound of the present invention may be produced by any method, and when it is described using a polymer compound having the repeating unit represented by formula (1) and the repeating unit represented by formula (5) as an example, the polymer compound can be produced by subjecting a compound shown by formula: Y³-W¹-Y⁴ and a compound shown by formula: Y⁵-W²-Y⁶ to condensation polymerization. In the formula, W¹ and W² represent the repeating unit represented by formula (1) or the repeating unit represented by formula (5). Y³, Y⁴, Y⁵ and Y⁶ are the same or different and each represents a polymerizable reactive group. In addition, when the polymer compound of the present invention has a repeating unit other than the above, a compound having the repeating unit other than the above may be subjected to condensation polymerization in coexistence with a compound having two polymerizable reactive groups.

Examples of the above polymerizable reactive group include a halogeno group, an alkylsulfonyloxy group, an arylsulfonyloxy group, an arylalkylsulfonyloxy group, a boric acid ester residue, a sulfonium methyl group, a phosphonium methyl group, a phosphonate methyl group, a monohalogenated methyl group, a boric acid residue (-B(OH)₂), a formyl group, a cyano group, a vinyl group, and the like.

Examples of the halogeno group that is the above polymerizable reactive group include a fluoro group, a chloro group, a bromo group, and an iodine group.

Examples of the alkylsulfonyloxy group that is the above polymerizable reactive group include a methanesulfonyloxy group, an ethanesulfonyloxy group, a trifluoromethanesulfonyloxy group, and the like.

Examples of the arylsulfonyloxy group that is the above polymerizable reactive group include a benzenesulfonyloxy group, a p-toluenesulfonyloxy group, and the like.

Examples of the arylalkylsulfonyloxy group that is the above polymerizable reactive group include a benzylsulfonyloxy group, and the like.

Examples of the boric acid ester residue that is the above polymerizable reactive group include groups shown by the following formulae.

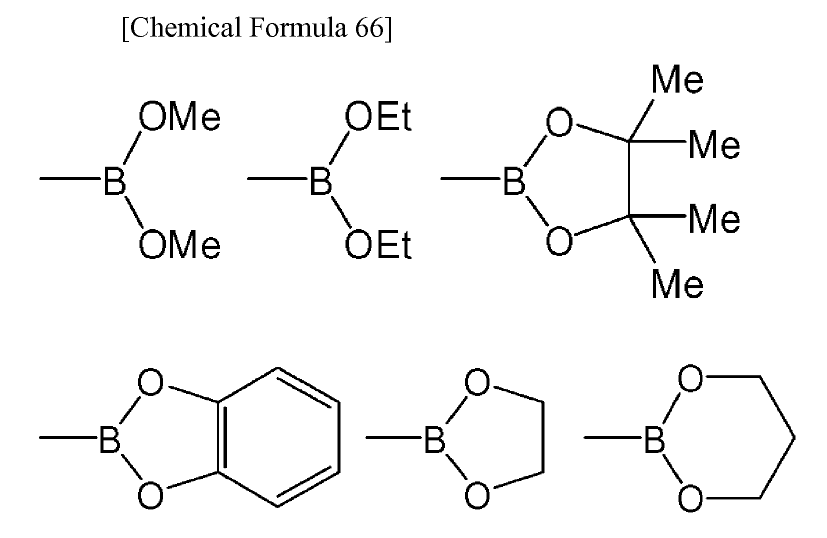

(In the formulae, Me represents a methyl group, Et represents an ethyl group. The same applies hereinafter.)

Examples of the sulfonium methyl group that is the above polymerizable reactive group include groups shown by the following formulae.

-CH₂S⁺Me₂X⁻

-CH₂S⁺Ph₂X⁻

(In the formulae, X- represents a halogenated ion. Ph represents a phenyl group. The same applies hereinafter.)

Examples of the phosphonium methyl group that is the above polymerizable reactive group include a group shown by the following formula.

-CH₂P⁺Ph₃X⁻

(In the formula, X- represents a halogenated ion.)

Examples of the phosphonate methyl group that is the above polymerizable reactive group include a group shown by the following formula.

-CH₂PO(OR')₂

(In the formula, R' represents an alkyl group or an aryl group. Examples of the alkyl group and the aryl group include the same groups as the examples of the alkyl group and the aryl group, which are substituents which the above ring A¹ may have. Two existing R' may be the same or different.)

Examples of the halogenated ion represented by the above X⁻ include a fluoride ion, a chloride ion, a bromide ion, and an iodide ion.
Examples of the monohalogenated methyl group that is the above polymerizable reactive group include a methyl fluoride group, a methyl chloride group, a methyl bromide group, and a methyl iodide group.

For example, in a case where a nickel zero-valent complex is used such as the Yamamoto coupling reaction, the above polymerizable reactive group is a halogeno group, an alkylsulfonyloxy group, an arylsulfonyloxy group, an arylalkylsulfonyloxy group, or the like, and when using a nickel catalyst or a palladium catalyst such as the Suzuki coupling reaction, the above polymerizable reactive group is an alkylsulfonyloxy group, a halogeno group, a boric acid ester residue, a boric acid residue, or the like.

The production of the polymer compound of the present invention can be carried out by dissolving a compound having a plurality of polymerizable reactive groups to be a monomer (hereinafter, may be referred to as "compound to be a raw material".) into an organic solvent, as necessary, using an alkali or an appropriate catalyst, at a temperature not less than the melting point and not more than the boiling point of the organic solvent. This is described in "Organic Reactions", Vol. 14, pp. 270-490, John Wiley & Sons, Inc., 1965; "Organic Syntheses", Collective Volume VI, pp. 407-411, John Wiley & Sons, Inc., 1988; Chem. Rev., Vol. 95, p. 2457, 1995; J. Organomet. Chem., Vol. 576, p. 147, 1999; Macromol. Chem., Macromol. Symp., Vol. 12, p. 229, 1987, and the like.

In the method for producing the polymer compound of the present invention, a known condensation reaction can be used depending on the type of the above polymerizable reactive group, and examples include a method of polymerizing corresponding monomers by the Suzuki coupling reaction, a polymerization method by the Grignard reaction, a polymerization method using a nickel zero-valent complex, a polymerization method using an oxidization agent such as FeCl₃, an electrochemical oxidization polymerization method, a method of decomposing an intermediate polymer having an appropriate leaving group, and the like. Of them, a method of polymerizing corresponding monomers by the Suzuki coupling reaction, a polymerization method by the Grignard reaction, and a polymerization method using a nickel zero-valent complex are preferable from the viewpoint of structural control.

Among the methods for producing the polymer compound of the present invention, a production method of subjecting the compound having polymerizable reactive group selected from the group consisting of halogeno groups, alkylsulfonyloxy groups, arylsulfonyloxy groups, and arylalkylsulfonyloxy groups to condensation polymerization in the presence of a nickel zero-valent complex is preferable.

Examples of the compound to be a raw material for the polymer compound of the present invention include dihalogenated compounds, bis(alkylsulfonate) compounds, bis(arylsulfonate) compounds, bis(arylalkyl sulfonate) compounds, halogen-alkylsulfonate compounds, halogen-arylsulfonate compounds, halogen-arylalkylsulfonate compounds, alkylsulfonate-arylsulfonate compounds, alkylsulfonate-arylalkylsulfonate compounds, arylsulfonate-arylalkylsulfonate compounds, and the like. In addition, when a polymer compound controlled in sequence is produced, it is preferable to use a halogen-alkylsulfonate compound, a halogen-arylsulfonate compound, a halogen-arylalkylsulfonate compound, an alkylsulfonate-arylsulfonate compound, an alkylsulfonate-arylalkylsulfonate compound, an arylsulfonate-arylalkylsulfonate compounds, or the like, as the compound to be a raw material.

As the method for producing the polymer compound of the present invention, from the viewpoint of ease of synthesizing a polymer compound, a production method of carrying out a condensation polymerization using a compound having one or more types of polymerizable reactive groups selected from the group consisting of halogeno groups, alkylsulfonyloxy groups, arylsulfonyloxy groups, arylalkylsulfonyloxy groups, a boric acid residue and a boric acid ester residue, and using a nickel catalyst or a palladium catalyst such that the ratio of the total number of moles (J) of the halogeno groups, the alkylsulfonyloxy groups, the arylsulfonyloxy groups, and the arylalkylsulfonyloxy groups, contained in the all compounds to be raw materials relative to the total number of moles (K) of the boric acid residue and the boric acid ester residue is substantially 1 (generally, K/J is in the range of 0.7 to 1.2) is preferable.

Examples of combinations of the above compounds to be raw materials (more specifically, the compound shown by the formula: Y³-W¹-Y⁴ and the compound shown by the formula: Y⁵-W²-Y⁶) include combinations of a dihalogenated compound, a bis(alkylsulfonate) compound, a bis(arylsulfonate) compound or a bis(arylalkyl sulfonate) compound, and a diboric acid compound or a diboric acid ester compound, and the like.

The organic solvent used for the above condensation polymerization is preferably sufficiently subjected to deoxidization treatment and dewatering treatment, in order to suppress a side reaction. However, dewatering treatment is not required in a case of a reaction in a two-phase system of the solvent and water such as in Suzuki coupling reaction.

Examples of the organic solvent to be used for the above condensation polymerization include saturated hydrocarbons such as pentane, hexane, heptane, octane, and cyclohexane; unsaturated hydrocarbons such as benzene, toluene, ethylbenzene, and xylene; halogenated saturated hydrocarbons such as carbon tetrachloride, chloroform, dichloromethane, chlorobutane, bromobutane, chloropentane, bromopentane, chlorohexane, bromohexane, chlorocyclohexane, and bromocyclohexane; halogenated unsaturated hydrocarbons such as chlorobenzene, dichlorobenzene, and trichlorobenzene; alcohols such as methanol, ethanol, propanol, isopropanol, butanol, and t-butyl alcohol; carboxylic acids such as formic acid, acetic acid, and propionic acid; ethers such as dimethyl ether, diethyl ether, methyl-t-butyl ether, tetrahydrofuran, tetrahydropyran, and dioxane; amines such as trimethylamine, triethylamine, N,N,N',N'-tetramethylethylenediamine, and pyridine; amides such as N,N-dimethylformamide, N,N-dimethylacetamide, N,N-diethylacetamide, and N-methylmorpholine oxide, and the like, ethers are preferable, and tetrahydrofuran and diethyl ether are particularly preferable. These organic solvents may be used singly or in combinations of two or more.

In the above condensation polymerization, an alkali or suitable catalyst may be added, in order to accelerate a reaction. As the alkali and catalyst, those sufficiently dissolved in the solvent used for the reaction are preferable. For mixing the alkali or catalyst with a reaction solution, a solution in which the alkali or catalyst is dissolved or dispersed may be slowly added to a reaction solution with stirring under an inert atmosphere such as argon or nitrogen, or conversely a reaction solution should be slowly added to a solution in which the alkali or catalyst is dissolved or dispersed.

When the polymer compound of the present invention is used in the production of a light-emitting device or the like, the purity thereof influences properties of light-emitting device such as light-emitting properties, and therefore, it is preferable to purify a compound to be a raw material before polymerization by a method such as distillation, sublimation purification, or recrystallization and then polymerize. Moreover, it is preferable to carry out a purification treatment such as reprecipitation purification or fractionation by chromatography for the obtained polymer compound after the polymerization.

(Compound Represented by Formula (6)) Next, the compound represented by formula (6) is described.

The compound represented by formula (6) is a compound to be a raw material, useful for synthesizing a polymer compound having the repeating unit represented by formula (1).

In formula (6), the aromatic hydrocarbon rings represented by ring A³ and ring A⁴ are preferably a single benzene ring or a ring into which a plurality of benzene rings are condensed, and examples thereof include a benzene ring, a naphthalene ring, an anthracene ring, a tetracene ring, a pentacene ring, a pyrene ring, a phenanthrene ring, and the like. As the combination of ring A³ and ring A⁴, combinations of a benzene ring and a benzene ring, a benzene ring and a naphthalene ring, a benzene ring and an anthracene ring, and a naphthalene ring and an anthracene ring are preferable, and a combination of a benzene ring and a benzene ring is more preferable.

When the combination of ring A³ and ring A⁴ is a benzene ring and a benzene ring, from the viewpoint of the synthesis of the resulting compound or the viewpoint of light-emitting properties as a device, as the compound represented by formula (6), a structure represented by formula (6-1) or formula (6-2) are preferable.

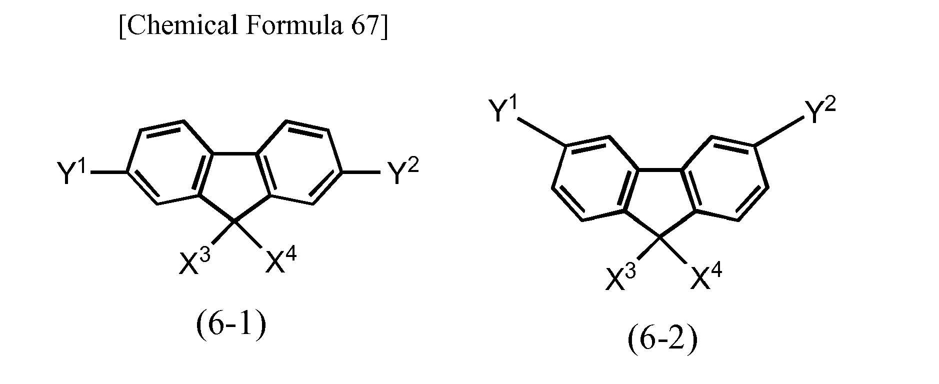

(The benzene rings in formula (6-1) and formula (6-2) are optionally substituted. X³, X⁴, Y¹ and Y² represent the same meanings as described above.)

When the aromatic hydrocarbon ring is substituted, the substituent may be single or plural, and when a plurality of substituents exist, they may be the same or different. Examples of the substituent include a halogeno group, an alkyl group, an alkenyl group, an alkynyl group, an alkoxy group, a group represented by formula (8), an alkylthio group, an aryl group, and an alkylsilyl group.

Examples of the alkyl group, the aryl group, the alkenyl group, the alkynyl group, the alkoxy group, the alkylthio group, and the alkylsilyl group include the same groups as the examples of the alkyl group, the aryl group, the alkenyl group, the alkynyl group, the alkoxy group, the alkylthio group, and the alkylsilyl group, which are substituents which the above ring A¹ may have.

In formula (6), Y¹ and Y² are the same or different, and represent a hydrogen atom or a polymerizable reactive group. A polymerizable reactive group refers to the same meaning as described above. When assuming that the compound represented by formula (6) is used for a polymerization using a nickel zero-valent complex such as the Yamamoto coupling reaction, the above polymerizable reactive group is preferably a halogeno group, an alkylsulfonyloxy group, an arylsulfonyloxy group, or an arylalkylsulfonyloxy group. Furthermore, as the halogeno group in this case, a chloro group, a bromo group, and an iodine group are preferable, and a bromo group and an iodine group are more preferable. In addition, when assuming that the compound represented by formula (6) is used for a polymerization using a nickel catalyst or a palladium catalyst such as the Suzuki coupling reaction, the above polymerizable reactive group is preferably an alkylsulfonyloxy group, a halogeno group, a boric acid ester residue, or a boric acid residue, and more preferably a halogeno group, a boric acid ester residue, or a boric acid residue. Furthermore, as the halogeno group in this case, a chloro group, a bromo group, and an iodine group are preferable, and a bromo group and an iodine group more preferable. From the viewpoint of synthesizing the compound represented by formula (6), Y¹ and Y² are preferably the same.

In formula (6), X³ and X⁴ are the same or different and each represents a hydrogen atom or a substituent, provided that at least one of X³ and X⁴ is a group represented by formula (7).

As a substituent other than the groups represented by formula (7), an alkyl group, an aryl group, an alkenyl group, an alkynyl group, an alkoxy group, an alkylthio group, an alkylsilyl group, and a group represented by formula (8) are preferable, and an alkyl group, an aryl group, an alkenyl group, and an alkynyl group are more preferable, from the viewpoint of light-emitting properties when made into a device.

Examples of the alkyl group, the aryl group, the alkenyl group, the alkynyl group, the alkoxy group, the alkylthio group, and the alkylsilyl group include the same groups as the examples of the alkyl group, the aryl group, the alkenyl group, the alkynyl group, the alkoxy group, the alkylthio group, and the alkylsilyl group, which are substituents which the above ring A¹ may have.

When X³ is a group represented by formula (7), X⁴ is preferably a hydrogen atom, an alkyl group, an aryl group, an alkenyl group, an alkynyl group, an alkoxy group, an alkylthio group, an alkylsilyl group, a group represented by formula (7), or a group represented by formula (8), more preferably a hydrogen atom, an alkyl group, an aryl group, an alkenyl group, an alkynyl group, or a group represented by formula (7), and further preferably an alkyl group, an aryl group, an alkenyl group, or an alkynyl group, from the viewpoint of light-emitting properties when made into a device.

When both X³ and X⁴ are a group represented by formula (7), X³ and X⁴ are preferably the same, from the viewpoint of ease of synthesizing the resulting compound.

In formula (7), Q¹, Q² and Q³ are the same or different and each represents -N= or -CH=, provided that at least two of Q¹, Q² and Q³ are -N=. From the viewpoint of the emission starting voltage and driving voltage at practical luminance of a device obtained when the polymer compound of the present invention is used in the production of the device, Q¹, Q² and Q³ are preferably all -N=.

In formula (7), q represents an integer of 0 or more, is preferably an integer of 0 to 3, more preferably 1 or 2, and further preferably 2.

In formula (7), p¹ to p⁶ are the same or different and each represents an integer of 0 or more, provided that p¹ + p² + p³ ≥ 1 and p⁴ + p⁵ + p⁶ ≥ 1. p¹ to p⁶ are preferably 0, 1 or 2, and more preferably 0 or 1. When p¹ to p⁶ are 0 or 1, the group represented by formula (7) is represented by formula (7-5) to (7-19) described later. Among these groups, from the viewpoint of charge transporting properties and properties when made into a device, the groups represented by formula (7-10), formula (7-11), formula (7-13), formula (7-16), and formula (7-19) are preferable, and the group represented by formula (7-10) is more preferable. In addition, among these groups, from the viewpoint of ease of monomer synthesis, the groups represented by formula (7-5), formula (7-10), formula (7-14), formula (7-17), and formula (7-19) are preferable, and the groups represented by formula (7-5), formula (7-10) and formula (7-17) are more preferable.

Examples of the group represented by formula (7) include groups represented by formula (7-1) to formula (7-4), and among them, a group represented by formula (7-4) in which Q¹, Q² and Q³ are all -N= is preferable.

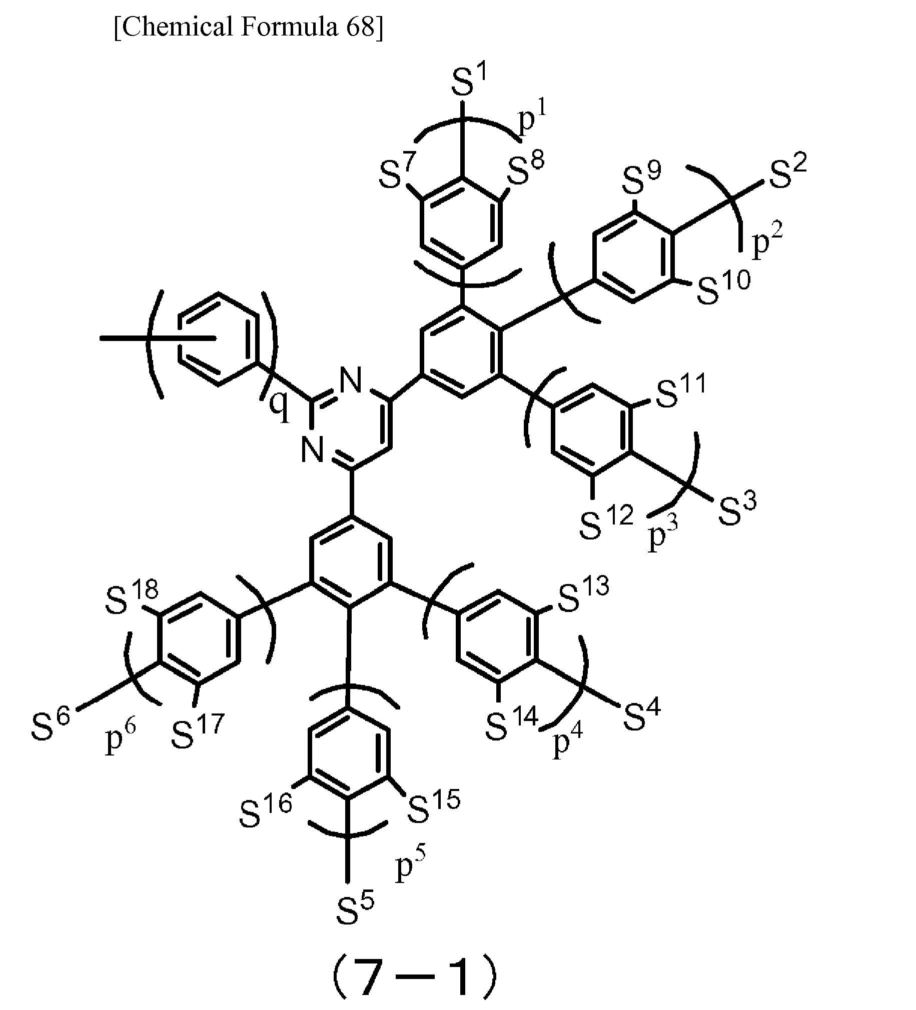

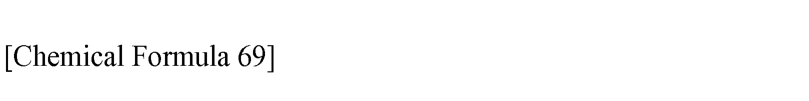

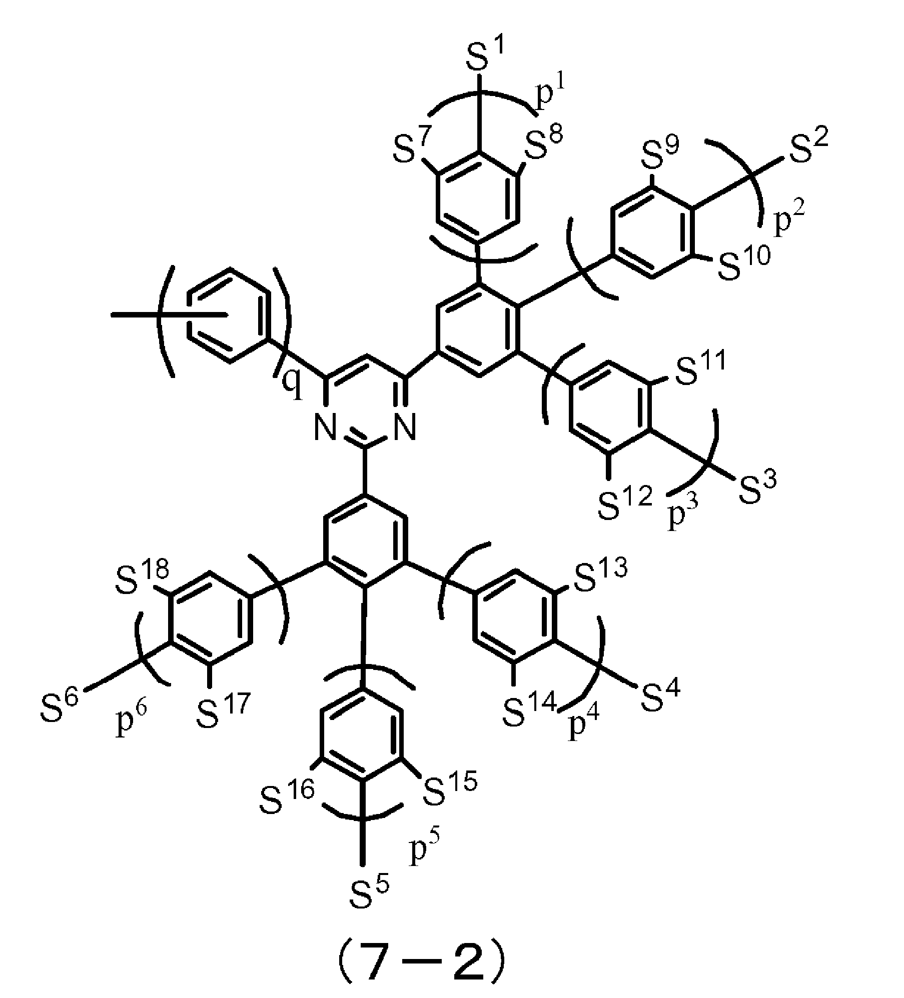

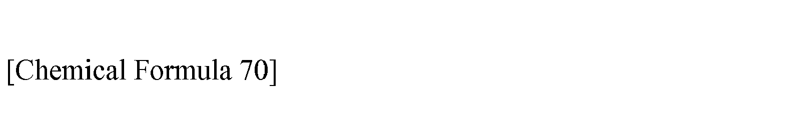

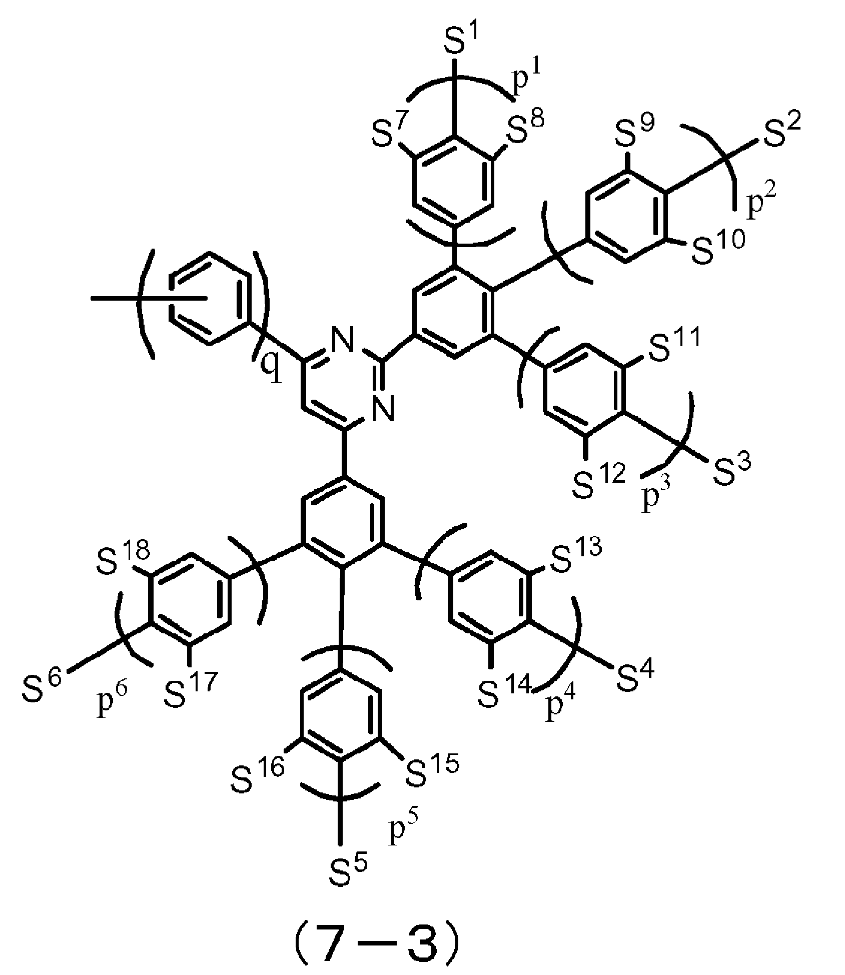

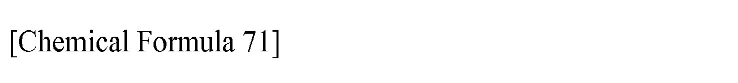

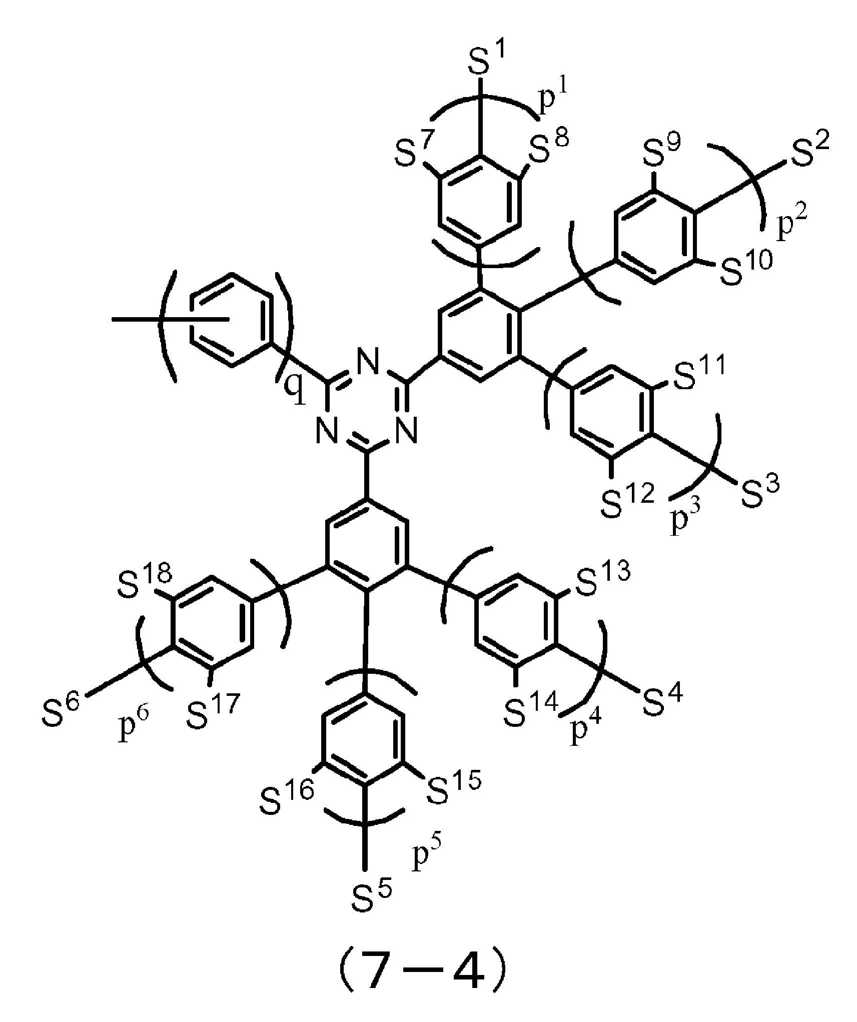

(In formulae (7-1) to (7-4), q, p¹ to p⁶ and S¹ to S¹⁸ represent the same meanings as described above.)

As the groups represented by formulae (7-1) to (7-4), the groups represented by the following formulae (7-5) to (7-19) are preferable.

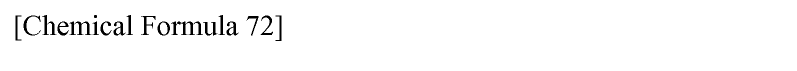

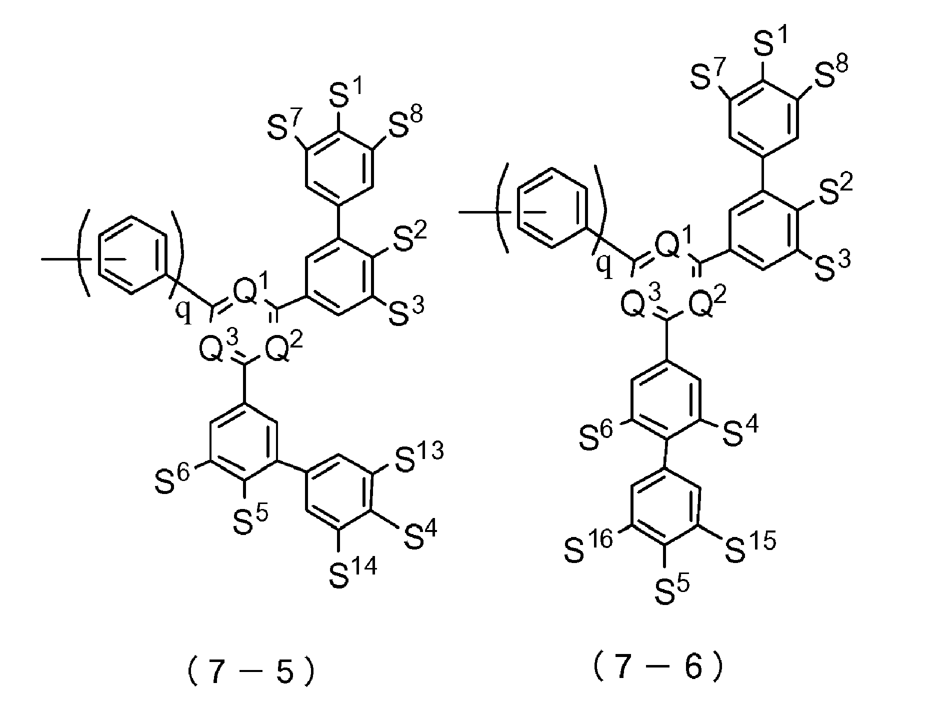

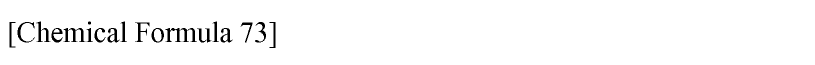

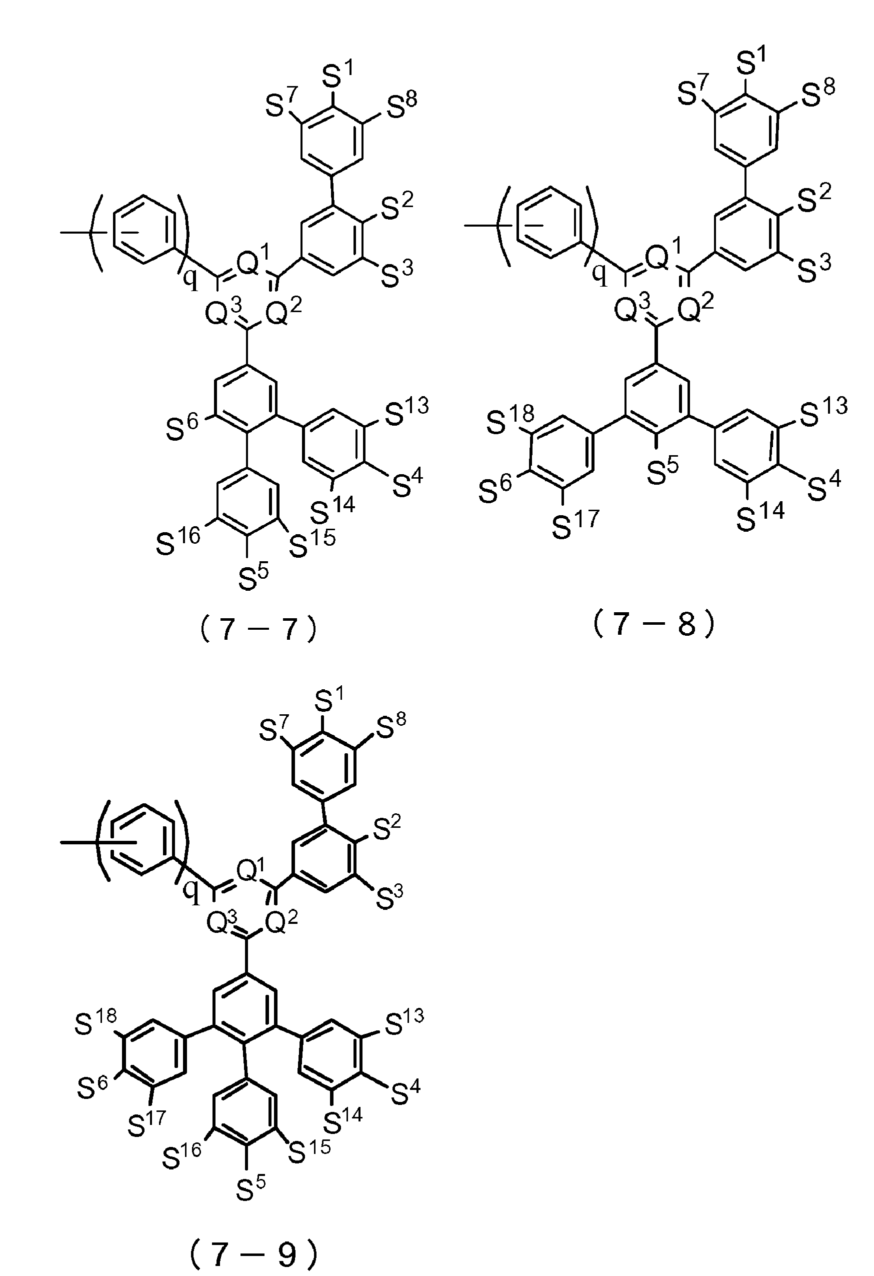

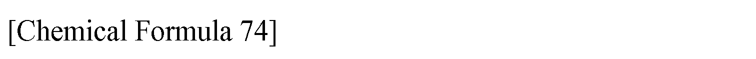

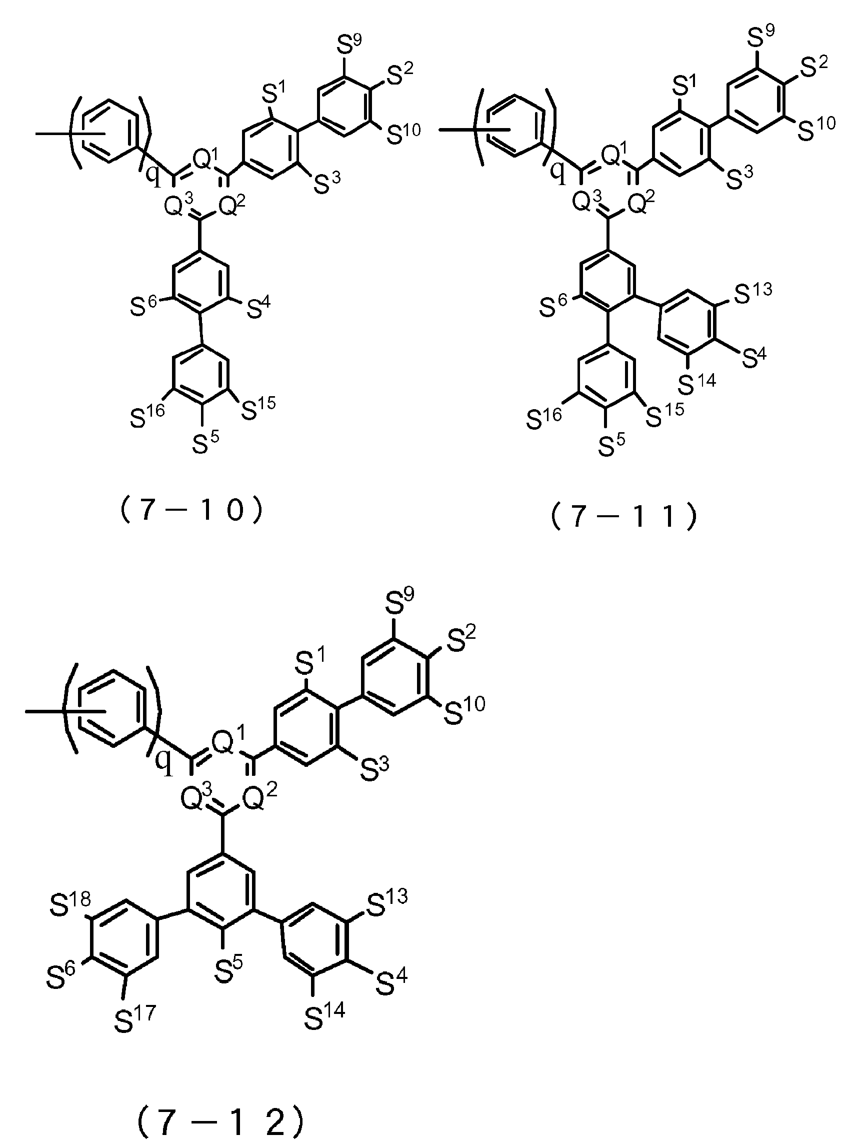

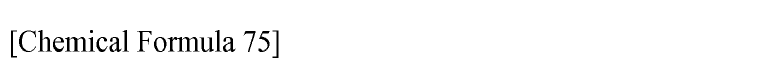

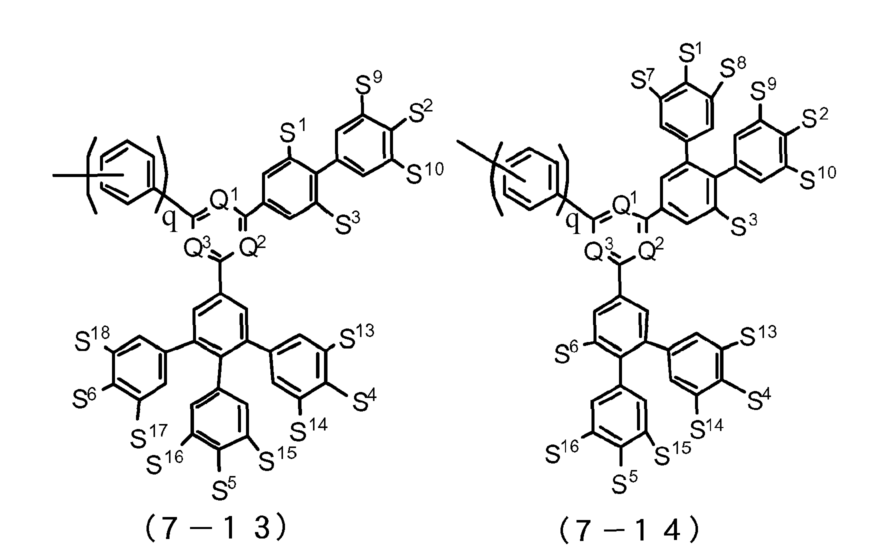

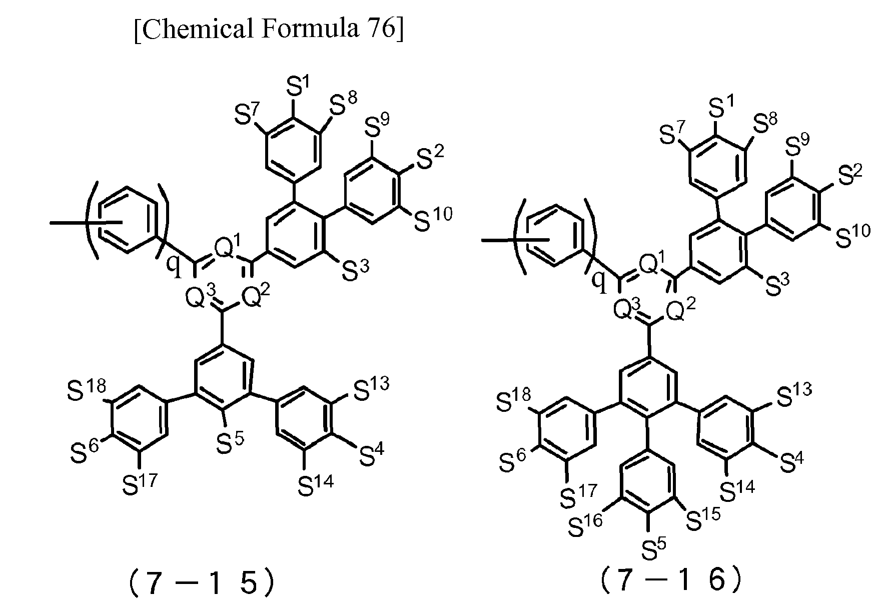

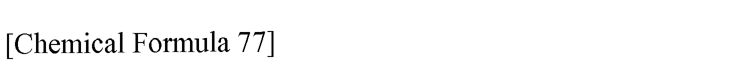

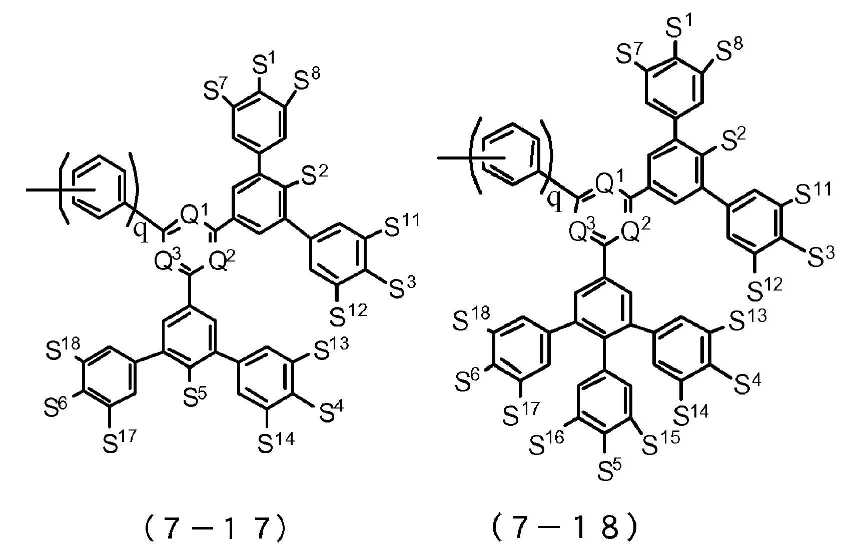

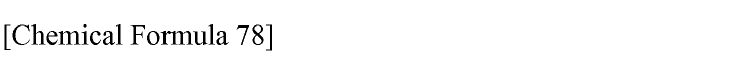

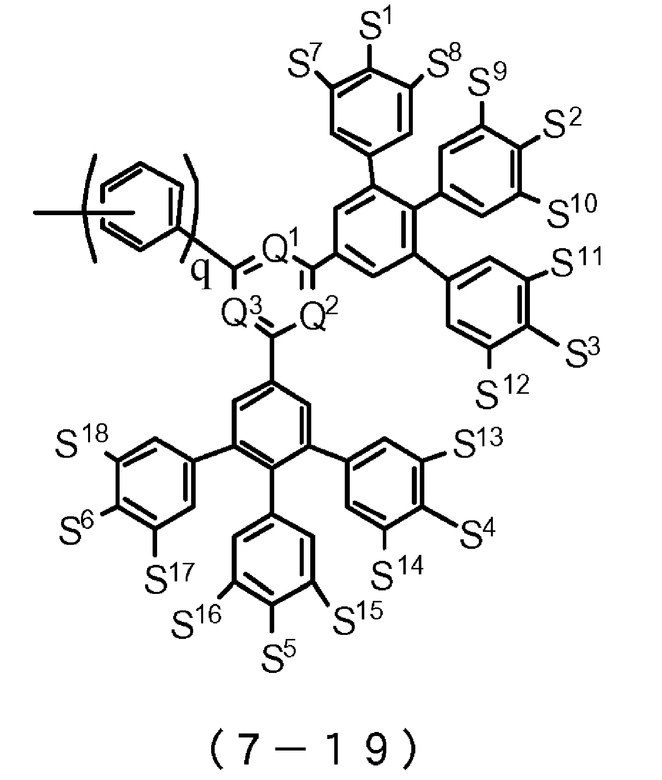

(In formulae (7-5) to (7-19), q, S¹ to S⁶ and Q¹ to Q¹⁸ represent the same meanings as described above.)

In formula (7), S¹ to S⁶ are the same or different and each represents a hydrogen atom, a halogeno group, an alkyl group, an alkenyl group, an alkynyl group, an alkoxy group, a group represented by formula (8), an alkylthio group, or an alkylsilyl group, and are preferably a hydrogen atom, an alkyl group, an alkenyl group, or an alkynyl group, and more preferably a hydrogen atom and an alkyl group.

In formula (7), S⁷ to S¹⁸ are the same or different and each represents a hydrogen atom, a halogeno group, an alkyl group, an alkenyl group, an alkynyl group, an alkoxy group, a group represented by formula (8), an alkylthio group, an aryl group, or an alkylsilyl group, and when a plurality of each S⁷ to S¹⁸ exist, they may be the same or different and are preferably a hydrogen atom, an alkyl group, an alkenyl group, an alkynyl group, or aryl group, and more preferably a hydrogen atom and an alkyl group.

Examples of the halogeno group include a fluoro group, a chloro group, a bromo group, and an iodine group.

Examples of the alkyl group, the aryl group, the alkenyl group, the alkynyl group, the alkoxy group, the alkylthio group, and the alkylsilyl group include the same groups as the examples of the alkyl group, the aryl group, the alkenyl group, the alkynyl group, the alkoxy group, the alkylthio group, and the alkylsilyl group, which are substituents which the above ring A¹ may have.

In formula (8), i represents an integer of 1 to 6, j represents an integer of 1 to 6, and k represents an integer of 0 to 5. From the viewpoint of synthesis of the resulting compound, i is preferably 1 to 3, j is preferably 1 or 2, and k is preferably 0 or 1.

From the viewpoint of light-emitting properties when made into a device, p¹, p³, p⁴ and p⁶ are preferably 0.

Examples of one of the preferred embodiments of the group represented by formula (7) include a group represented by formula (9).

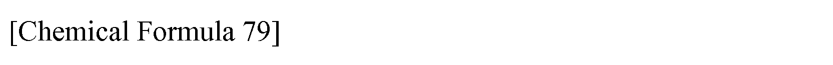

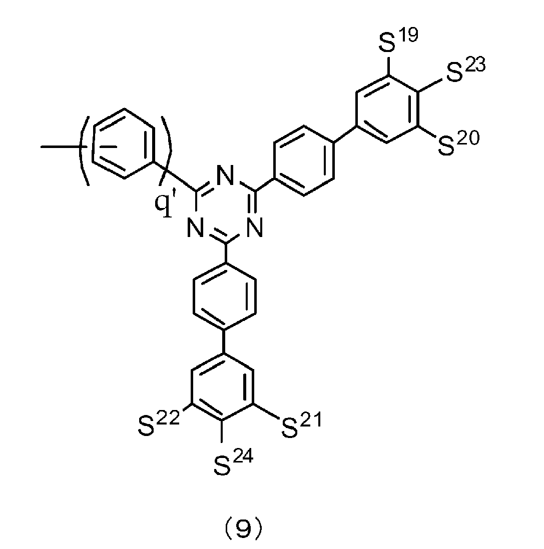

(In formula (9), q' represents 1 or 2; and S¹⁹ to S²⁴ are the same or different and each represents a hydrogen atom, an alkyl group, an alkenyl group, an alkoxy group, or a group represented by formula (8).)

In formula (9), q' represents 1 or 2, and q' is preferably 2.

In formula (9), S¹⁹ to S²⁴ are the same or different and each represents a hydrogen atom, an alkyl group, an alkenyl group, an alkoxy group, or a group represented by formula (3). Examples of the alkyl group, the alkenyl group, and the alkoxy group include the same groups as the examples of the alkyl group, the alkenyl group, and the alkoxy group, which are substituents which the above ring A¹ may have, and are preferably a hydrogen atom, an alkyl group, an alkenyl group, and an alkynyl group, and more preferably a hydrogen atom and an alkyl group.

As the group represented by formula (9), from the viewpoint of ease of synthesis of the resulting compound, groups represented by the following formulae (9-1) to (9-8) are preferable, and the groups represented by formula (9-1), formula (9-2), formula (9-7) and formula (9-8) are more preferable, and the groups represented by formula (9-1) and formula (9-2) are further preferable.

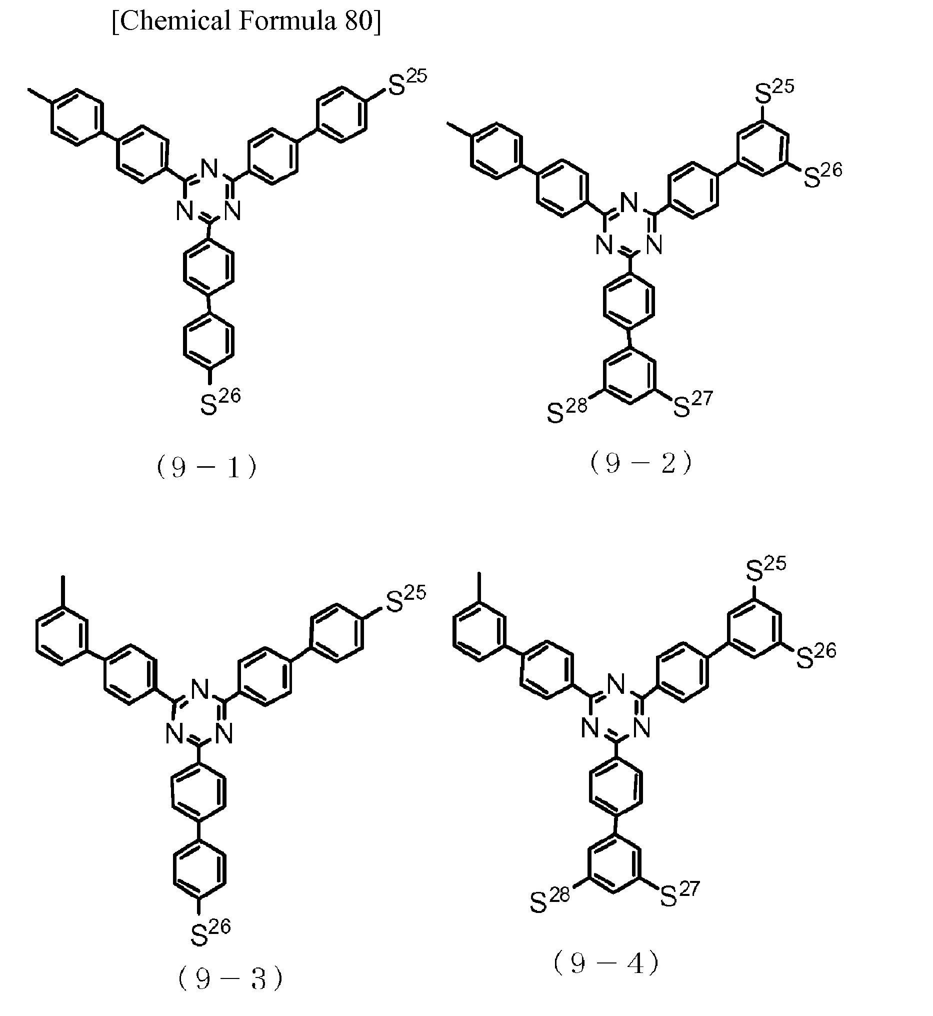

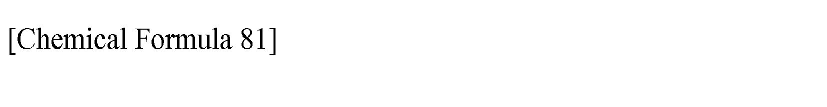

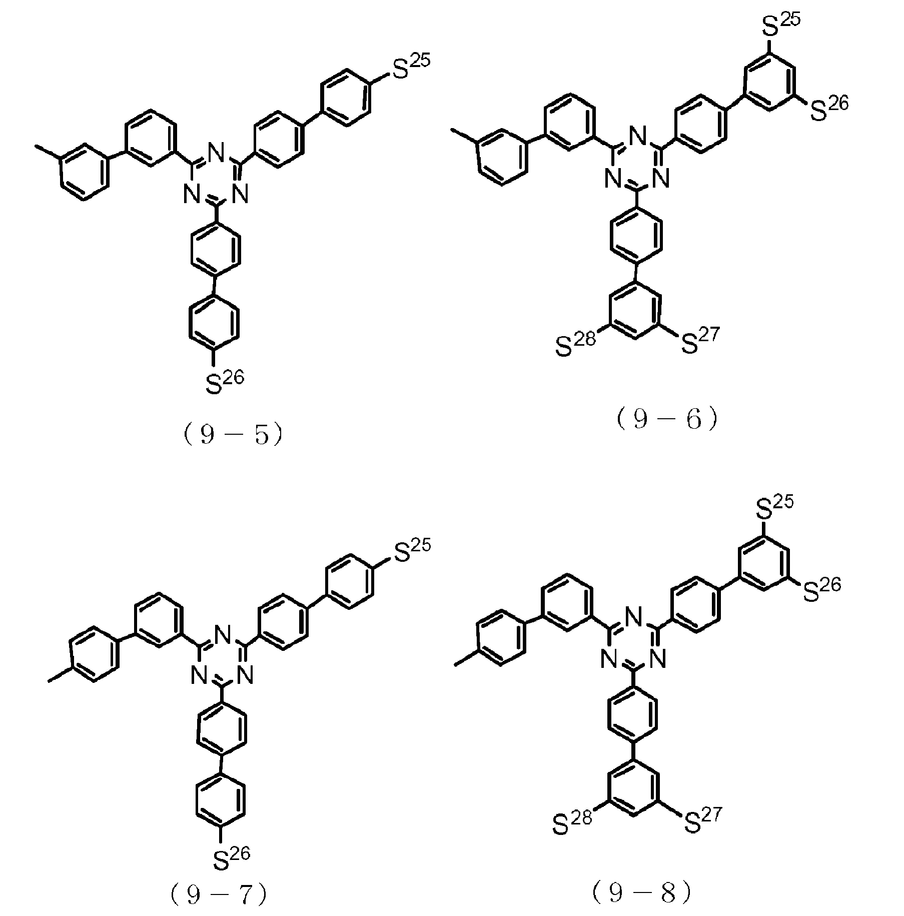

(In formula (9-1) to formula (9-8), S²⁵ to S²⁸ are the same or different and each represents an alkyl group, an alkenyl group, an alkoxy group, or a group represented by formula (8)).

(In formula (9-1) to formula (9-8), S²⁵ to S²⁸ are an alkyl group, an alkenyl group, an alkoxy group, or a group represented by formula (3). Examples of the alkyl group, the alkenyl group, and the alkoxy group include the same groups as the examples of the alkyl group, the alkenyl group, and the alkoxy group, which are substituents which the above ring A¹ may have.

S²⁵ to S²⁸ are preferably an alkyl group, and from the viewpoint of solubility and synthesis, more preferably an i-propyl group, a butyl group, an i-butyl group, an s-butyl group, a t-butyl group, a pentyl group, a hexyl group, a cyclohexyl group, a heptyl group, an octyl group, a 2-ethylhexyl group, a nonyl group, or a decyl group, and particularly preferably an i-propyl group, a butyl group, a t-butyl group, a pentyl group, a hexyl group, a heptyl group, or an octyl group.

Next, applications of the polymer compound of the present invention are described.

In general, the polymer compound of the present invention emits fluorescence or phosphorescence in a solid state, and it can be used as a polymer light emitter (light-emitting material of high molecular weight). In addition, the polymer compound has an excellent charge transporting ability, and can be suitably used as a material for polymer electroluminescent device (hereinafter, may be referred to as "polymer light-emitting device".) or a charge transport material. The polymer light-emitting device using the polymer light emitter is a high performance polymer light-emitting device which can be driven at low voltage with high efficiency. Therefore, the polymer light-emitting device can be preferably used for a backlight of a liquid crystal display, a curved or planar light source for lighting, a segment-type display device, and a device such as a flat panel display of dot matrix. Moreover, the polymer compound of the present invention can also be used as a laser dye, a material for organic solar battery, an organic semiconductor for organic transistor, a conductive thin film, and a material for conductive thin film such as an organic semiconductor thin film. Furthermore, it can also used as a light-emitting thin film material that emits fluorescence or phosphorescence.

Next, the polymer light-emitting device of the present invention is described.

The polymer light-emitting device of the present invention has an organic layer between the electrodes consisting of an anode and a cathode containing the polymer compound of the present invention or the composition of the present invention described later. This organic layer may be any one of a light-emitting layer, a hole transport layer, a hole injection layer, an electron transport layer, an electron injection layer, an interlayer layer, and the like, and is preferably a light-emitting layer. The light-emitting layer herein refers to a layer having the function of emitting light, the hole transport layer refers to a layer having the function of transporting holes, and the electron transport layer refers to a layer having the function of transporting electrons. In addition, the interlayer layer refers to a layer located between the light-emitting layer and the anode and adjacent to the light-emitting layer and playing a role of isolating the light-emitting layer from the anode or the light-emitting layer from the hole injection layer or the hole transport layer. The electron transport layer and the hole transport layer are collectively referred to as a charge transport layer. Moreover, the electron injection layer and the hole injection layer are collectively referred to as a charge injection layer. The light-emitting layer, the hole transport layer, the hole injection layer, the electron transport layer, and the electron injection layer may be used each independently, or two or more layers may be used.

When an organic layer is a light-emitting layer, the light-emitting layer that is the organic layer may further contain at least one material selected from a hole transport material, an electron transport material and a light-emitting material. The light-emitting material herein refers to a material showing fluorescence and/or phosphorescence.

When the polymer compound of the present invention is mixed with a hole transport material, the mixing ratio of the hole transport material relative to the total mixture (equivalent to the compound described later) is 1 to 80% by weight, and preferably 5 to 60% by weight. When the polymer compound of the present invention is mixed with an electron transport material, the mixing ratio of the electron transport material relative to the total mixture is 1 to 80% by weight, and preferably 5 to 60% by weight. Furthermore, when the polymer compound of the present invention is mixed with a light-emitting material, the mixing ratio of the light-emitting material relative to the total mixture is 1 to 80% by weight, and preferably 5 to 60% by weight. When the polymer compound of the present invention is mixed with at least two materials selected from the group consisting of a hole transport material, an electron transport material, and a light-emitting material, the mixing ratio of the light-emitting material relative to the total mixture is 1 to 50% by weight, and preferably 5 to 40% by weight, and the total ratio of the hole transport material and the electron transport material is 1 to 50% by weight, and preferably 5 to 40% by weight.

As the hole transport material, the electron transport material and the light-emitting material to be mixed, a known low-molecular compound, triplet light-emitting complex and high-molecular-weight compound (different from the polymer compound of the present invention) can be used, and it is preferable to use a high-molecular-weight compound. Examples of the hole transport material, electron transport material and light-emitting material that are high-molecular-weight compounds include a polyfluorene, a derivative and fluorene copolymer thereof, a polyarylene, a derivative and arylene copolymer thereof, a polyarylenevinylene, a derivative and arylenevinylene copolymer thereof, and a (co)polymer of an aromatic amine and a derivative thereof, which are disclosed in WO99/13692, WO99/48160, GB2340304A, WO00/53656, WO01/19834, WO00/55927, GB2348316, WO00/46321, WO00/06665, WO99/54943, WO99/54385, US5777070, WO98/06773, WO97/05184, WO00/35987, WO00/53655, WO01/34722, WO99/24526, WO00/22027, WO00/22026, WO98/27136, US573636, WO98/21262, US5741921, WO97/09394, WO96/29356, WO96/10617, EP0707020, WO95/07955, Japanese Patent Application Laid-Open Publication No. 2001-181618, Japanese Patent Application Laid-Open Publication No. 2001-123156, Japanese Patent Application Laid-Open Publication No. 2001-3045, Japanese Patent Application Laid-Open Publication No. 2000-351967, Japanese Patent Application Laid-Open Publication No. 2000-303066, Japanese Patent Application Laid-Open Publication No. 2000-299189, Japanese Patent Application Laid-Open Publication No. 2000-252065, Japanese Patent Application Laid-Open Publication No. 2000-136379, Japanese Patent Application Laid-Open Publication No. 2000-104057, Japanese Patent Application Laid-Open Publication No. 2000-80167, Japanese Patent Application Laid-Open Publication No. 10-324870, Japanese Patent Application Laid-Open Publication No. 10-114891, Japanese Patent Application Laid-Open Publication No. 9-111233, Japanese Patent Application Laid-Open Publication No. 9-45478, and the like.

As a light-emitting material that is a low-molecular compound, for example, a naphthalene derivative, an anthracene and a derivative thereof, a perylene and a derivative thereof, dyes such as polymethine base, xanthene base, coumarin base and cyanine base dye, metallic complexes of 8-hydroxyquinoline and a derivative thereof, aromatic amines, tetraphenylcyclopentadiene and a derivative thereof, and tetraphenylbutadiene and a derivative thereof can be used. Furthermore, compounds described in Japanese Patent Application Laid-Open Publication No. 57-51781 and Japanese Patent Application Laid-Open Publication No. 59-194393 can be also used.

Examples of the triplet light-emitting complex include Ir(ppy)₃, Btp₂Ir(acac) containing iridium as a central metal, ADS066GE commercially available from American Dye Source, Inc., PtOEP containing platinum as a central metal, Eu(TTA)₃phen containing europium as a central metal, and the like.

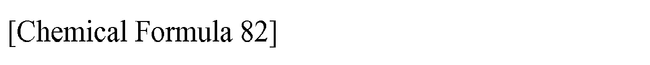

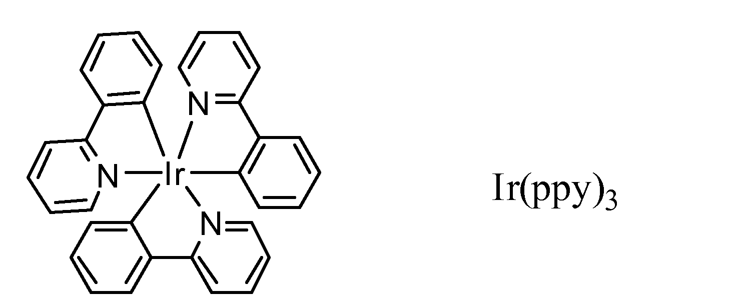

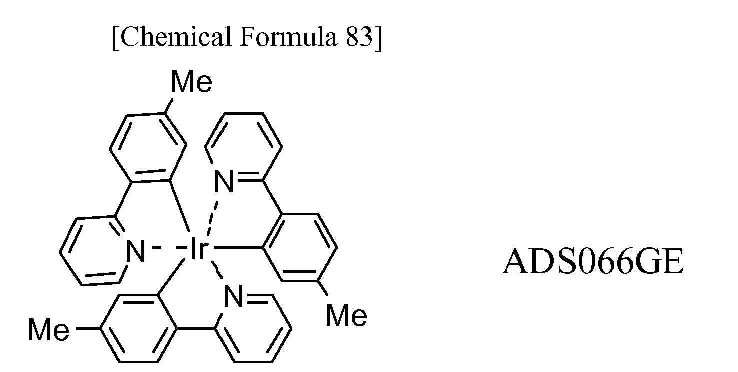

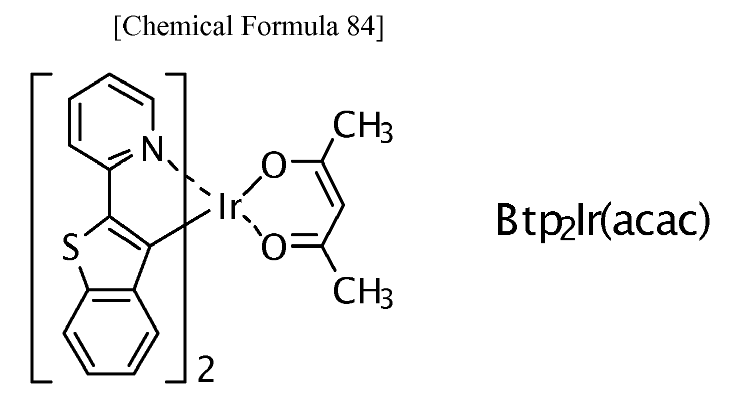

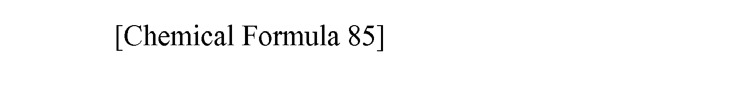

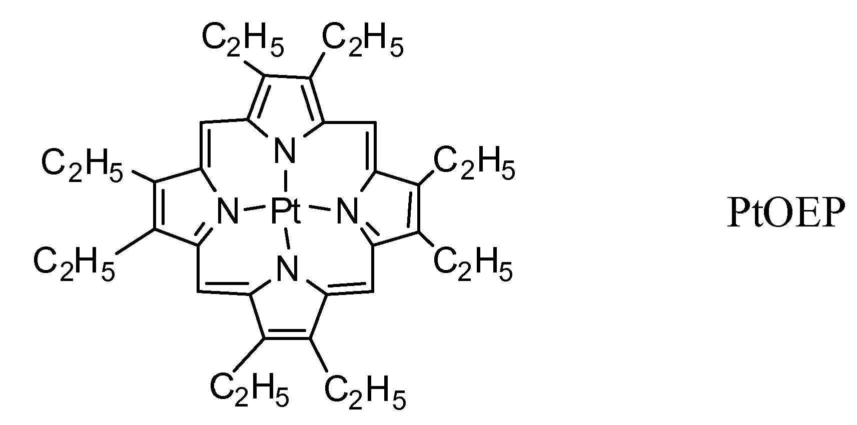

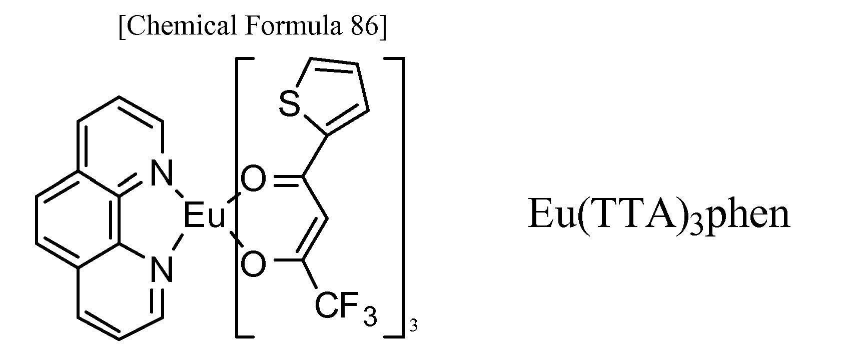

As the triplet light-emitting complex, complexes described in Nature, (1998), 395, 151, Appl. Phys. Lett. (1999), 75(1), 4, Proc. SPIE-Int. Soc. Opt. Eng. (2001), 4105 (Organic Light-Emitting Materials and DevicesV), 119, J. Am. Chem. Soc., (2001), 123, 4304, Appl. Phys. Lett., (1997), 71(18), 2596, Syn. Met., (1998), 94(1), 103, Syn. Met., (1999), 99(2), 1361, Adv. Mater., (1999), 11(10), 852, Jpn. J. Appl. Phys., 34, 1883 (1995), and the like can be also used.

It is possible to prepare a composition by using the polymer compound of the present invention in combination with at least one material selected from hole transport materials, electron transport materials and light-emitting materials. This composition is useful as a light-emitting material and a charge transport material. In this composition, the polymer compound of the present invention may be used singly or in combinations of two or more.

The film thickness of a light-emitting layer which the polymer light-emitting device of the present invention has shows an optimum value varying depending on the material to be used and should be selected so as to give optimum driving voltage and light emission efficiency, and it is generally 1 nm to 1 µm, preferably 2 nm to 500 nm, and further preferably 5 nm to 200 nm.

Examples of a method for forming a light-emitting layer include a method of film formation from a solution. As the film formation method from a solution, application methods such as a spin-coating method, a casting method, a micro gravure coating method, a gravure coating method, a bar coating method, a roll coating method, a wire bar coating method, a dip coating method, a spray coating method, a screen printing method, a flexographic printing method, an offset printing method, and an inkjet printing method can be used. Printing methods such as a screen printing method, a flexographic printing method, an offset printing method, and an inkjet printing method are preferable since pattern formation and multicolor separate painting are easy.

In the above printing method, it is preferable to use a solution prepared by further comprising a solvent to the composition of the present invention. The ratio of the polymer compound of the present invention in the solution is generally 20 to 100% by weight and preferably 40 to 100% by weight, based on the total weight of the solid content excluding the solvent.

The ratio of the solvent contained in the solution is generally 1 to 99.9% by weight, preferably 60 to 99.5% by weight, and further preferably 80 to 99.0 % by weight, based on the total weight of the solution.

While the viscosity of the solution varies depending upon the printing method, in the solution used in the method in which the solution passes through an ejection apparatus, such as an inkjet printing method, the viscosity is preferably in the range of 1 to 20 mPa·s at 25°C in order to prevent clogging and flight bending at the time of ejection.

The solution may contain a stabilizer and an additive for controlling viscosity and/or surface tension. As the additive, a high-molecular-weight polymer compound (thickener) and a poor solvent for increasing viscosity, a low-molecular-weight compound for reducing viscosity, a surfactant for reducing surface tension and the like should be used in combination.

The above high-molecular-weight compound may be any compound as long as it is soluble in the same solvent as that of the polymer compound of the present invention and does not inhibit light emission and charge transport, and polystyrene and polymethyl methacrylate, and the like can be used. The weight average molecular weight of the high-molecular-weight compound is preferably 0.5 million or more, and more preferably 1 million or more.

A poor solvent can be also used as a thickener. More specifically, viscosity can be increased by adding a small amount of poor solvent to the solid content in the solution. When a poor solvent is added for this purpose, the type and addition amount of the solvent should be selected in the range where the solid content in the solution does not precipitate. In consideration of the stability during storage of the solution, the amount of the poor solvent is preferably 50% by weight or less relative to the total amount of the solvent and further preferably 30% by weight or less.

In addition, the solution of the present invention may contain an antioxidant other than the polymer compound of the present invention for improving storage stability. The antioxidant may be any compound as long as it is soluble in the same solvent as that of the polymer compound of the present invention and does not inhibit light emission and charge transport, and examples include a phenol based antioxidant and a phosphorus based antioxidant.

As a solvent used in the solution of the present invention, a solvent capable of dissolving or homogeneously dispersing components that constitute the solution other than the solvent is preferable. Examples of the solvent include chlorine base solvents such as chloroform, methylene chloride, 1,2-dichloroethane, 1,1,2-trichloroethane, chlorobenzene, and o-dichlorobenzene; ether base solvents such as tetrahydrofuran, dioxane, and anisole; aromatic hydrocarbon base solvents such as toluene and xylene; aliphatic hydrocarbon base solvents such as cyclohexane, methylcyclohexane, n-pentane, n-hexane, n-heptane, n-octane, n-nonane, and n-decane; ketone base solvents such as acetone, methyl ethyl ketone, cyclohexanone, benzophenone, and acetophenone; ester solvents such as ethyl acetate, butyl acetate, ethyl cellosolve acetate, methyl benzoate, and phenyl acetate; polyhydric alcohols such as ethylene glycol, ethylene glycol monobutyl ether, ethylene glycol monoethyl ether, ethylene glycol monomethyl ether, dimethoxyethane, 1,2-propanediol, diethoxymethane, triethylene glycol monoethyl ether, glycerin, and 1,2-hexanediol, and derivatives thereof; alcohol base solvents such as methanol, ethanol, propanol, isopropanol, and cyclohexanol; sulfoxide base solvents such as dimethylsulfoxide; and amide base solvents such as N-methyl-2-pyrrolidone and N,N-dimethylformamide. In addition, these solvents can be used singly or in combinations of a plurality of these. Of them, from the viewpoint of solubility, uniformity during film formation and viscosity properties of a polymer compound, aromatic hydrocarbon base solvents, aliphatic hydrocarbon base solvents, ester base solvents and ketone base solvents are preferable, and toluene, xylene, ethylbenzene, diethylbenzene, trimethylbenzene, n-propylbenzene, isopropylbenzene, n-butylbenzene, isobutylbenzene, s-butylbenzene, anisole, ethoxybenzene, 1-methylnaphthalene, cyclohexane, cyclohexanone, cyclohexylbenzene, bicyclohexyl, cyclohexenyl-cyclohexanone, n-heptylcyclohexane, n-hexylcyclohexane, 2-propylcyclohexanone, 2-heptanone, 3-heptanone, 4-heptanone, 2-octanone, 2-nonanone, 2-decanone, dicyclohexyl ketone, acetophenone and benzophenone are more preferable.

From the viewpoint of film formability and from the viewpoint of device properties, and the like, the number of types of solvents in the solution is preferably two or more types, more preferably two to three types, and further preferably two types.

When two types of solvents are contained in the solution, one type of solvent among them may be in a solid state at 25°C. From the viewpoint of film formability, one type of solvent is preferably a solvent having a boiling point of 180°C or higher, and more preferably a solvent having a boiling point of 200°C or higher. Meanwhile, from the viewpoint of viscosity, 1% by weight or more of the polymer compound of the present invention is preferably dissolved in both of the two types of solvents at 60°C, and 1% by weight or more of the polymer compound of the present invention is preferably dissolved in one type of solvent among the two types of solvents at 25°C.

When two or more types of solvents are contained in the solution, from the viewpoint of viscosity and film formability, the solvent having the highest boiling point is contained in an amount of preferably 40 to 90% by weight, more preferably 50 to 90% by weight, and particularly preferably 65 to 85% by weight, based on the weight of all the solvents in the solution.

The polymer compound of the present invention contained in the solution may be one type or two or more types, and a polymer compound other than the polymer compound of the present invention may be contained in the range which would not impair device properties and the like.

The solution of the present invention may contain water and a metal and a salt thereof in the range of 1 to 1000 ppm. Examples of the metal include lithium, sodium, calcium, potassium, iron, copper, nickel, aluminum, zinc, chromium, manganese, cobalt, platinum, iridium, and the like. In addition, the solution of the present invention may contain silicon, phosphorus, fluorine, chlorine, and bromine in the range of 1 to 1000 ppm.

A thin film can be produced using the solution of the present invention in accordance with a spin-coating method, a casting method, a micro gravure coating method, a gravure coating method, a bar coating method, a roll coating method, a wire bar coating method, a dip coating method, a spray coating method, a screen printing method, a flexographic printing method, an offset printing method, an inkjet printing method, or the like. Among them, the solution of the present invention is preferably used to form a film by a screen printing method, a flexographic printing method, an offset printing method, or an inkjet printing method, and more preferably used to form a film by an inkjet printing method.

Examples of the thin film that can be formed by using the solution of the present invention include a light-emitting thin film, an electric conductive thin film, and an organic semiconductor thin film.

The electric conductive thin film of the present invention preferably has a surface resistance of 1 KΩ/sq or lower. The electric conductivity can be increased by doping a thin film with a Lewis acid, an ionic compound, or the like. The surface resistance is more preferably 100 Ω/sq or lower, and further preferably 10 Ω/sq.

In the organic semiconductor thin film of the present invention, the larger of the electron mobility and the hole mobility is preferably 10⁻⁵ cm²/V/sec or more, more preferably 10⁻³ cm²/V/sec or more, and particularly preferably 10⁻¹ cm²/V/sec or more.

An organic transistor can be obtained by forming the organic semiconductor thin film on a Si substrate having an insulating film of SiO₂ or the like and a gate electrode formed thereon, and then forming a source electrode and a drain electrode with Au and the like.

Meanwhile, examples of the polymer light-emitting device of the present invention include a polymer light-emitting device having an electron transport layer located between a cathode and a light-emitting layer; a polymer light-emitting device having a hole transport layer located between an anode and a light-emitting layer; a polymer light-emitting device having an electron transport layer located between a cathode and a light-emitting layer, and a hole transport layer between an anode and a light-emitting layer; and the like.

Examples of structure of the polymer light-emitting device of the present invention include the following structures a) to d).
a) anode/light-emitting layer/cathode
b) anode/hole transport layer/light-emitting layer/cathode
c) anode/light-emitting layer/electron transport layer/cathode
d) anode/hole transport layer/light-emitting layer/electron transport layer/cathode
(here, / indicates that each layer is adjacently laminated. The same applies hereinafter.)

Moreover, examples also include structures having an interlayer layer located between a light-emitting layer and an anode and adjacent to the light-emitting layer in each of these structures (the following a') to d')).
a') anode/interlayer layer/light-emitting layer/cathode
b') anode/hole transport layer/interlayer layer/light-emitting layer/cathode
c') anode/interlayer layer/light-emitting layer/electron transport layer/cathode
d') anode/hole transport layer/interlayer layer/light-emitting layer/electron transport layer/cathode

When the polymer light-emitting device of the present invention has a hole transport layer, among the hole transport layers to be used, examples of a high-molecular-weight hole transport material include polyvinylcarbazole and derivatives thereof, polysilane and derivatives thereof, polysiloxane derivatives having an aromatic amine on a side chain or a main chain, pyrazoline derivatives, arylamine derivatives, stilbene derivatives, triphenyldiamine derivatives, polyaniline and derivatives thereof, polythiophene and derivatives thereof, polypyrrole and derivatives thereof, poly(p-phenylenevinylene) and derivatives thereof, and poly(2,5-thienylene vinylene) and derivatives thereof. Examples of the hole transport material also include materials described in Japanese Patent Application Laid-Open Publication No. 63-70257, Japanese Patent Application Laid-Open Publication No. 63-175860, Japanese Patent Application Laid-Open Publication No. 02-135359, Japanese Patent Application Laid-Open Publication No. 02-135361, Japanese Patent Application Laid-Open Publication No. 02-209988, Japanese Patent Application Laid-Open Publication No. 03-37992, and Japanese Patent Application Laid-Open Publication No. 03-152184. Among these, as the hole transport material used in the hole transport layer, polymer hole transport materials such as polyvinylcarbazole and derivatives thereof, polysilane and derivatives thereof, polysiloxane derivatives having an aromatic amine compound group on a side chain or a main chain, polyaniline and derivatives thereof, polythiophene and derivatives thereof, poly(p-phenylenevinylene) and derivatives thereof, and poly(2,5-thienylene vinylene) and derivatives thereof are preferable, polyvinylcarbazole and derivatives thereof, polysilane and derivatives thereof, and polysiloxane derivatives having an aromatic amine on a side chain or a main chain are further preferable.

In addition, examples of the hole transport material made of a low-molecular compound include pyrazoline derivatives, arylamine derivatives, stilbene derivatives, and triphenyldiamine derivatives. In a case of the low-molecular hole transport material, the material is preferably dispersed in a polymer binder and used.

As the polymer binder to be mixed, a compound that does not extremely inhibit charge transportation is preferable, and a compound whose absorption of visible light is not strong is preferable. Examples of the polymer binder include poly(N-vinylcarbazole), polyaniline and derivatives thereof, polythiophene and derivatives thereof, poly(p-phenylenevinylene) and derivatives thereof, poly(2,5-thienylene vinylene) and derivatives thereof, polycarbonate, polyacrylate, polymethylacrylate, polymethyl methacrylate, polystyrene, polyvinyl chloride, and polysiloxane.

Polyvinylcarbazole and derivatives thereof are obtained, for example, from a vinyl monomer by cation polymerization or radical polymerization.

Examples of polysilane and derivatives thereof include compounds described in Chemical Review (Chem. Rev.), Vol. 89, p. 1359 (1989) and the published specification of GB Patent No. 2300196. As the synthesis method of polysilane and derivatives thereof, methods described in these can be used, and Kipping method is suitably used.

Since polysiloxane and derivatives thereof show little hole transporting property in the siloxane skeleton structures, ones having a structure of the low-molecular hole transport material on a side chain or a main chain are suitably used. As polysiloxane and derivatives thereof, a compound that has a hole transporting aromatic amine on a side chain or a main chain.

Examples of the film formation method of the hole transport layer, in the case of the hole transport material made of a low-molecular compound, include a method of film formation from a mixed solution with a polymer binder, and in the case of the high-molecular-weight hole transport material, include a method of film formation from a solution.

As the solvent used in the film formation from a solution, one that can dissolve or uniformly disperse the hole transport material is preferable. Examples of the solvent include chlorine-based solvents such as chloroform, methylene chloride, 1,2-dichloroethane, 1,1,2-trichloroethane, chlorobenzene, and o-dichlorobenzene; ether-based solvents such as tetrahydrofuran and dioxane; aromatic hydrocarbon-based solvents such as toluene and xylene; aliphatic hydrocarbon-based solvents such as cyclohexane, methylcyclohexane, n-pentane, n-hexane, n-heptane, n-octane, n-nonane, and n-decane; ketone-based solvents such as acetone, methyl ethyl ketone, and cyclohexanone; ester-based solvents such as ethyl acetate, butyl acetate, and ethyl cellosolve acetate; polyhydric alcohols such as ethylene glycol, ethylene glycol monobutyl ether, ethylene glycol monoethyl ether, ethylene glycol monomethyl ether, dimethoxyethane, 1,2-propanediol, diethoxymethane, triethylene glycol monoethyl ether, glycerin, and 1,2-hexanediol, and derivatives thereof; alcohol-based solvents such as methanol, ethanol, propanol, isopropanol, and cyclohexanol; sulfoxide-based solvent such as dimethyl sulfoxide; and amide-based solvents such as N-methyl-2-pyrrolidone and N,N-dimethylformamide. Meanwhile, these organic solvents can be used singly or in combinations of a plurality of kinds.

As the film formation method from a solution, application methods from a solution, such as a spin coating method, a casting method, a micro gravure coating method, a gravure coating method, a bar coating method, a roll coating method, a wire bar coating method, a dip coating method, a spray coating method, a screen printing method, a flexo printing method, an offset printing method, and an inkjet printing method can be used.
The optimum value of the film thickness of the hole transport layer varies depending on the material to be used, and the film thickness should be selected so as to give appropriate values of the driving voltage and luminous efficiency. However, the thickness that does not cause a pin hole is needed, and when the thickness is too large, the driving voltage of the device is likely to increase. Therefore, the film thickness of the hole transport layer is generally from 1 nm to 1 µm, preferably 2 nm to 500 nm, and further preferably 5 nm to 200 nm.

When the polymer light-emitting device of the present invention has an electron transport layer, a known compound can be used as an electron transport material to be used, and examples include oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, or metal complexes of 8-hydroxyquinoline and derivatives thereof, polyquinoline and derivatives thereof, polyquinoxaline and derivatives thereof, and polyfluorene and derivatives thereof, and examples include compounds described in Japanese Patent Application Laid-Open Publication No. 63-70257, Japanese Patent Application Laid-Open Publication No. 63-175860, Japanese Patent Application Laid-Open Publication No. 02-135359, Japanese Patent Application Laid-Open Publication No. 02-135361, Japanese Patent Application Laid-Open Publication No. 02-209988, Japanese Patent Application Laid-Open Publication No. 03-37992, and Japanese Patent Application Laid-Open Publication No. 03-152184. Among these, oxadiazole derivatives, benzoquinone and derivatives thereof, anthraquinone and derivatives thereof, metal complexes of 8-hydroxyquinoline and derivatives thereof, polyquinoline and derivatives thereof, polyquinoxaline and derivatives thereof, and polyfluorene and derivatives thereof are preferable, and 2-(4-biphenylyl)-5-(4-t-butylphenyl)-1,3,4-oxadiazole, benzoquinone, anthraquinone, tris(8-quinolinol)aluminum, and polyquinoline are further preferable.

Examples of the film formation method of the electron transport layer include each, in the case of the electron transport material that is a low-molecular compound, a vacuum evaporation method from powder or a film formation method from a solution or a melted condition, and in the case of a high-molecular-weight electron transport material, a film formation method from a solution or a melted condition. When a film is formed from a solution or a melted condition, the above-described polymer binder may be used together.

As a solvent used in the film formation from a solution, one that can dissolve or uniformly disperse the electron transport material and/or the polymer binder is preferable. Examples of the solvent include chlorine-based solvents such as chloroform, methylene chloride, 1,2-dichloroethane, 1,1,2-trichloroethane, chlorobenzene, and o-dichlorobenzene; ether-based solvents such as tetrahydrofuran and dioxane; aromatic hydrocarbon-based solvents such as toluene and xylene; aliphatic hydrocarbon-based solvents such as cyclohexane, methylcyclohexane, n-pentane, n-hexane, n-heptane, n-octane, n-nonane, and n-decane; ketone-based solvents such as acetone, methyl ethyl ketone, and cyclohexanone; ester-based solvents such as ethyl acetate, butyl acetate, and ethyl cellosolve acetate; polyhydric alcohols such as ethylene glycol, ethylene glycol monobutyl ether, ethylene glycol monoethyl ether, ethylene glycol monomethyl ether, dimethoxyethane, 1,2-propanediol, diethoxymethane, triethylene glycol monoethyl ether, glycerin, and 1,2-hexanediol, and derivatives thereof; alcohol-based solvents such as methanol, ethanol, propanol, isopropanol, and cyclohexanol; sulfoxide-based solvent such as dimethyl sulfoxide; and amide-based solvents such as N-methyl-2-pyrrolidone and N,N-dimethylformamide. Meanwhile, these organic solvents can be used singly or in combinations of a plurality of kinds.

As the film formation method from a solution or a melted condition, application methods, such as a spin coating method, a casting method, a micro gravure coating method, a gravure coating method, a bar coating method, a roll coating method, a wire bar coating method, a dip coating method, a spray coating method, a screen printing method, a flexo printing method, an offset printing method, and an inkjet printing method can be used.

The optimum value of the film thickness of the electron transport layer varies depending on the material to be used, and the film thickness should be selected so as to give appropriate values of the driving voltage and luminous efficiency. However, the thickness that does not cause a pin hole is needed, and when the thickness is too large, the driving voltage of the device is likely to increase. Therefore, the film thickness of the hole transport layer is generally from 1 nm to 1 µm, preferably 2 nm to 500 nm, and further preferably 5 nm to 200 nm.

Additionally, among charge transport layers placed adjacent to the electrodes, those having the function of improving the charge injecting efficiency from the electrode and having an effect of lowering the driving voltage of the device may be, in particular, generally called charge injection layers (hole injection layer, electron injection layer).

Furthermore, for improving close adherence to an electrode and charge injection from an electrode, the above charge injection layer or an insulating layer having a film thickness of 2 nm or less may be placed adjacent to the electrode, and also, for improving close adherence at an interface, preventing mixing, and the like, a thin buffer layer may be inserted into the interface of a charge transport layer and a light-emitting layer.
The order and the number of layers to be laminated and the thickness of each layer can be set in consideration of the luminous efficiency and the lifetime of the device.

In the present invention, examples of the polymer light-emitting device having the charge injection layers (electron injection layer, hole injection layer) include a polymer light-emitting device having a charge injection layer placed adjacent to a cathode, and a polymer light-emitting device having a charge injection layer placed adjacent to an anode.

Examples of the structure of the polymer light-emitting device of the present invention include the following structures e) to p).
e) anode/charge injection layer/light-emitting layer/cathode
f) anode/light-emitting layer/charge injection layer/cathode
g) anode/charge injection layer/light-emitting layer/charge injection layer/cathode
h) anode/charge injection layer/hole transport layer /light-emitting layer/cathode
i) anode/hole transport layer/light-emitting layer/charge injection layer/cathode
j) anode/charge injection layer/hole transport layer/light-emitting layer/charge injection layer/cathode
k) anode/charge injection layer/light-emitting layer/electron transport layer/cathode
1) anode/light-emitting layer/electron transport layer/charge injection layer/cathode
m) anode/charge injection layer/light-emitting layer/electron transport layer/charge injection layer/cathode
n) anode/charge injection layer/hole transport layer/light-emitting layer/electron transport layer/cathode
o) anode/hole transport layer/light-emitting layer/electron transport layer/charge injection layer/cathode
p) anode/charge injection layer/hole transport layer/light-emitting layer/electron transport layer/charge injection layer/cathode.

Moreover, examples also include structures having an interlayer layer located between a light-emitting layer and an anode and adjacent to the light-emitting layer in each of these structures. In this case, an interlayer layer may be served as a hole injection layer and/or a hole transport layer.

Examples of the charge injection layer include a layer containing an electric conductive polymer, a layer located between an anode and a hole transport layer and containing a material having an ionization potential of an intermediate value between an anode material and a hole transport material contained in the hole transport layer, and a layer located between a cathode and an electron transport layer and containing a material having an electron affinity of an intermediate value between a cathode material and an electron transport material contained in the electron transport layer.

When the above charge injection layer is a layer containing an electric conductive polymer, the electric conductivity of the electric conductive polymer is preferably not less than 10⁻⁵ S/cm and not more than 10³ S/cm, and for decreasing the leak current between light emitting picture elements, more preferably not less than 10⁻⁵ S/cm and not more than 10² S/cm, and further preferably not less than 10⁻⁵ S/cm and not more than 10¹ S/cm.

When the above charge injection layer is a layer containing an electric conductive polymer, the electric conductivity of the electric conductive polymer is preferably not less than 10⁻⁵ S/cm and not more than 10³ S/cm, and for decreasing the leak current between light emitting picture elements, more preferably not less than 10⁻⁵ S/cm and not more than 10² S/cm, and further preferably not less than 10⁻⁵ S/cm and not more than 10¹ S/cm.

For making the electric conductivity of the electric conductive polymer to be not less than 10⁻⁵ S/cm and not more than 10³ S/cm, the electric conductive polymer is generally doped with an appropriate amount of ions.

The kind of the ions to be doped with is an anion in the case of the hole injection layer, and is a cation in the case of the electron injection layer. Examples of the anion include a polystyrenesulfonic acid ion, alkylbenzenesulfonic acid ions, and a camphorsulfonic acid ion, examples of the cation include a lithium ion, a sodium ion, a potassium ion, and a tetrabutylammonium ion.

The film thickness of the charge injection layer is generally from 1 nm to 100 nm, and preferably 2 nm to 50 nm.

A material used in the charge injection layer may be selected according to a relation with materials of an electrode and adjacent layer, and examples include polyaniline and derivatives thereof, polythiophene and derivatives thereof, polypyrrole and derivatives thereof, polyphenylenevinylene and derivatives thereof, polythienylenevinylene and derivatives thereof, polyquinoline and derivatives thereof, polyquinoxaline and derivatives thereof, electric conductive polymers such as a polymer having an aromatic amine structure on a main chain or a side chain, metal phthalocyanines (copper phthalocyanine and the like), and carbon.

An insulating layer having a film thickness of 2 nm or less has a function to make charge injection easier. Examples of a material of the above insulating layer include metal fluorides, metal oxides, organic insulating materials, and the like. Examples of the polymer light-emitting device comprising the insulating layer having a film thickness of 2 nm or less include a polymer light-emitting device in which the insulating layer having a film thickness of 2 nm or less is placed adjacent to a cathode, and a polymer light-emitting device in which the insulating layer having a film thickness of 2 nm or less is placed adjacent to an anode.

Examples of the structure of the polymer light-emitting device of the present invention include the following structures q) to ab).
q) anode/insulating layer having a film thickness of 2 nm or less/light-emitting layer/cathode
r) anode/light-emitting layer/insulating layer having a film thickness of 2 nm or less/cathode
s) anode/insulating layer having a film thickness of 2 nm or less/light-emitting layer/insulating layer having a film thickness of 2 nm or less/cathode
t) anode/insulating layer having a film thickness of 2 nm or less/hole transport layer/light-emitting layer/cathode
u) anode/hole transport layer/light-emitting layer/insulating layer having a film thickness of 2 nm or less/cathode
v) anode/insulating layer having a film thickness of 2 nm or less/hole transport layer/light-emitting layer/insulating layer having a film thickness of 2 nm or less/cathode
w) anode/insulating layer having a film thickness of 2 nm or less/light-emitting layer/electron transport layer/cathode x) anode/light-emitting layer/electron transport layer/insulating layer having a film thickness of 2 nm or less/cathode
y) anode/insulating layer having a film thickness of 2 nm or less/light-emitting layer/electron transport layer/insulating layer having a film thickness of 2 nm or less/cathode
z) anode/insulating layer having a film thickness of 2 nm or less/hole transport layer/light-emitting layer/electron transport layer/cathode aa) anode/hole transport layer/light-emitting layer/electron transport layer/insulating layer having a film thickness of 2 nm or less/cathode ab) anode/insulating layer having a film thickness of 2 nm or less/hole transport layer/light-emitting layer/electron transport layer/insulating layer having a film thickness of 2 nm or less/cathode

Moreover, examples also include structures having an interlayer layer located between a light-emitting layer and an anode and adjacent to the light-emitting layer in each of these structures. In this case, an interlayer layer may be served as a hole injection layer and/or a hole transport layer.

When an interlayer layer is applied to the above structures a) to ab), the interlayer layer is preferably located between an anode and a light-emitting layer and a layer having an ionization potential between two layers adjacent thereto or one layer and an anode.

Examples of a material used in the interlayer layer include polymers containing an aromatic amine such as polyvinylcarbazole and derivatives thereof, polyarylene derivatives having an aromatic amine on a side chain or a main chain, arylamine derivatives, and triphenyldiamine derivatives.

Examples of the film formation method of the interlayer layer in the case of using a polymer material include a film formation method from a solution.

A solvent used in the film formation from a solution is preferably one that can dissolve or uniformly disperse the material used in the interlayer layer. Examples of the solvent include chlorine-based solvents such as chloroform, methylene chloride, 1,2-dichloroethane, 1,1,2-trichloroethane, chlorobenzene, and o-dichlorobenzene; ether-based solvents such as tetrahydrofuran and dioxane; aromatic hydrocarbon-based solvents such as toluene and xylene; aliphatic hydrocarbon-based solvents such as cyclohexane, methylcyclohexane, n-pentane, n-hexane, n-heptane, n-octane, n-nonane, and n-decane; ketone-based solvents such as acetone, methyl ethyl ketone, and cyclohexanone; ester-based solvents such as ethyl acetate, butyl acetate, and ethyl cellosolve acetate; polyhydric alcohols such as ethylene glycol, ethylene glycol monobutyl ether, ethylene glycol monoethyl ether, ethylene glycol monomethyl ether, dimethoxyethane, 1,2-propanediol, diethoxymethane, triethylene glycol monoethyl ether, glycerin, and 1,2-hexanediol, and derivatives thereof; alcohol-based solvents such as methanol, ethanol, propanol, isopropanol, and cyclohexanol; sulfoxide-based solvent such as dimethyl sulfoxide; and amide-based solvents such as N-methyl-2-pyrrolidone, and N,N-dimethylformamide. Meanwhile, these organic solvents can be used singly or in combinations of a plurality of kinds.

As the film formation method from a solution, application methods from a solution, such as a spin coating method, a casting method, a micro gravure coating method, a gravure coating method, a bar coating method, a roll coating method, a wire bar coating method, a dip coating method, a spray coating method, a screen printing method, a flexo printing method, an offset printing method, and an inkjet printing method can be used.

The optimum value of the film thickness of the interlayer layer varies depending on the material to be used, and the film thickness may be selected so as to give appropriate values of the driving voltage and luminous efficiency, and is generally from 1 nm to 1 µm, preferably 2 nm to 500 nm, and further preferably 5 nm to 200 nm.

When the interlayer layer is placed adjacent to the light-emitting layer, particularly when both layers are formed by the application method, the materials of the two layers may be mixed and have an unfavorable effect on the device properties and the like. Examples of a method for reducing the mixing of the materials of the two layers, when the interlayer layer is formed by the application method and thereafter the light-emitting layer is formed by the application method, include a method in which an interlayer layer is formed by the application method, thereafter the interlayer layer is insolubilized in a solvent used in the production of a light-emitting layer by heating the interlayer layer, and then a light-emitting layer is formed. The heating temperature is generally from 150° to 300°C, and the heating time is generally from 1 minute to 1 hour. In this case, for removing a component not insolubilized to a solvent by heating, the interlayer layer is rinsed with a solvent used in the formation of a light-emitting layer after heating and before forming the light-emitting layer, whereby the component can be removed. When the solvent insolubilization by heating is sufficiently performed, the rinsing with a solvent can be omitted. For the sufficient solvent insolubilization by heating, it is preferable to use a compound containing at least one polymerizable reactive group in the molecule, as a polymer compound to be used in the interlayer layer. Furthermore, the number of polymerizable reactive groups is preferably 5% or more based on the number of repeating units in the molecule.

A substrate forming the polymer light-emitting device of the present invention may be a substrate which forms an electrode and does not change shape when layers of organic substances are formed, and examples include a glass substrate, a plastic substrate, a polymer film substrate, and a silicon substrate. In the case of an opaque substrate, the electrode opposite thereto is preferably transparent or semi-transparent.

While at least one of the anode and the cathode that the polymer light-emitting device of the present invention comprises is generally transparent or semi-transparent, the anode side is preferably transparent or semi-transparent.

As a material for the anode, an electric conductive metal oxide film, a semi-transparent metal thin film, or the like is used. As the material for the anode, films (NESA or the like) produced using an electric conductive inorganic compound made of indium oxide, zinc oxide, tin oxide, and indium tin oxide (ITO) and indium zinc oxide, which are their composites, and the like; gold, platinum, silver, and copper; and the like, and ITO, indium zinc oxide, and tin oxide are preferable. Examples of the production method include a vacuum evaporation method, a sputtering method, an ion plating method, a plating method, and the like. Moreover, as the anode, an organic transparent electric conductive film such as polyaniline and derivatives thereof and polythiophene and derivatives thereof may be used.

The film thickness of the anode can be selected in consideration of the light transmittance and electric conductivity, and is generally from 10 nm to 10 µm, preferably 20 nm to 1 µm, and further preferably 50 nm to 500 nm.

Moreover, for making charge injection easier, a layer made of a phthalocyanine derivative, an electric conductive polymer, a carbon, or the like, or a layer made of a metal oxide, metal fluoride, organic insulating material, or the like having an average film thickness of 2 nm or less may be formed on the anode.

A material for the cathode used in the polymer light-emitting device of the present invention is preferably a material having a small work function. As the material of the cathode, metals such as lithium, sodium, potassium, rubidium, cesium, beryllium, magnesium, calcium, strontium, barium, aluminum, scandium, vanadium, zinc, yttrium, indium, cerium, samarium, europium, terbium, and ytterbium; alloys of two or more of them; alloys of at least one of them and at least one of gold, silver, platinum, copper, manganese, titanium, cobalt, nickel, tungsten, and tin; and graphite and graphite intercalation compounds are used. Examples of the alloy include a magnesium-silver alloy, a magnesium-indium alloy, a magnesium-aluminum alloy, an indium-silver alloy, a lithium-aluminum alloy, a lithium-magnesium alloy, a lithium-indium alloy, and a calcium-aluminum alloy. The cathode may have a laminated structure of two or more layers.

The film thickness of the cathode can be selected in consideration of the electric conductivity and durability, and is generally from 10 nm to 10 µm, preferably 20 nm to 1 µm, and further preferably 50 nm to 500 nm.

As the forming method of the cathode, a vacuum evaporation method, a sputtering method, a laminating method in which a metal thin film is adhered by heat and pressure, or the like is used. Moreover, a layer made of an electric conductive polymer or a layer made of a metal oxide, a metal fluoride, an organic insulating material, or the like having an average film thickness of 2 nm or less may be located between the cathode and the organic substance layer, and after the cathode is produced, a protective layer for protecting the polymer light-emitting device may be mounted. In order to stably use the polymer light-emitting device for a long term, a protective layer and/or a protective cover are preferably mounted to protect the device from the outside.

As the protective layer, a polymer compound, a metal oxide, a metal fluoride, a metal boride, or the like can be used. Meanwhile, as the protective cover, a glass plate, a plastic plate having a surface subjected to a low permeability treatment, or the like can be used. As a method for placing a protective cover, a method in which the cover is pasted to a device substrate with a thermosetting resin or a photo curable resin to be sealed is suitably used. When a spacer is used to retain a space, it is easy to prevent the device from damage. When the space is filled with an inert gas such as nitrogen or argon, it is possible to prevent oxidization of the cathode, and furthermore, when a desiccant such as barium oxide is placed in the space, damage on the device by moisture adsorbed in the production process is easily suppressed. It is preferable to take at least any one of the measures among these.

The polymer light-emitting device of the present invention can be used as a planar light source, a segment display device, a dot-matrix display device, and a backlight of a liquid crystal display device. In order to obtain light emission in a planar form using the polymer light-emitting device of the present invention, an anode and a cathode in a planar form should be disposed so as to be superposed on each other. Moreover, in order to obtain light emission in a pattern form, there are a method in which a mask having a window in a pattern form is disposed on the surface of the above planar light-emitting device, a method in which a non-light-emitting part of an organic substance layer is formed extremely thick so that substantially no light can be emitted, and a method in which any one of an anode and a cathode or both of the electrodes are formed in a pattern form. By forming a pattern by any of these methods and disposing some electrodes in a way that the electrodes can be turned ON/OFF independently, a display device of segment type is obtained which can display numbers, letters, simple marks, and the like. Furthermore, in order to produce a dot-matrix device, both an anode and a cathode should be formed in a stripe form and disposed so as to cross each other. Partial color display and multi-color display are made possible by a method in which a plurality of types of polymer fluorescent substances that emit different light colors are separately applied, or a method in which a color filter or a fluorescent conversion filter is used. The dot-matrix device can be passively driven, or may be actively driven in combination with TFT and the like. These display devices can be used as display devices of a computer, a television, a portable terminal, a cell phone, a car navigation, a viewfinder of a video camera, and the like. Furthermore, the above planar light-emitting device is a thin self light-emitting type, and can be suitably used as a planar light source for a backlight of a liquid crystal display device, or a planar light source for lighting. In addition, when a flexible substrate is used, the device can be also used as a curved light source or a display device.

### [Examples]

Hereinafter, the present invention will be described more specifically on the basis of Examples. However, the present invention is not limited to the following Examples.

As the number average molecular weight and the weight average molecular weight, the polystyrene-equivalent number average molecular weight and weight average molecular weight were obtained by size exclusion chromatography (SEC). Among SEC, a gel permeation chromatography of which mobile phase is an organic solvent is referred to as gel permeation chromatography (GPC). A polymer to be measured was dissolved in tetrahydrofuran in a concentration of about 0.5% by weight, and 30 µL of the solution was injected into GPC (manufactured by Shimadzu Corporation, trade name: LC-10Avp). Tetrahydrofuran was used as the mobile phase of GPC, and allowed to flow at a flow rate of 0.6 mL/min. As a column, two TSKgel SuperHM-H (manufactured by Tosoh Corporation) and one TSKgel SuperH2000 (manufactured by Tosoh Corporation) were connected in series. As a detector, a differential refractive index detector (manufactured by Shimadzu Corporation, trade name: RID-10A) was used.

In addition, LC-MS was measured by the following method. A measurement sample was dissolved in chloroform or tetrahydrofuran so as to have a concentration of about 2 mg/mL, and 1 µL of the solution was injected into LC-MS (manufactured by Agilent Technologies, Inc., trade name: 1100LCMSD). As the mobile phase of LC-MS, ion-exchanged water to which about 0.1% by weight of acetic acid was added and acetonitrile to which about 0.1% by weight of acetic acid was added were used with shifting the ratio thereof, and allowed to flow at a flow rate of 0.2 mL/min. As a column, L-column 2 ODS (3 µm) (manufactured by Chemicals Evaluation and Research Institute, Japan, inner diameter of 2.1 mm, length of 100 mm, particle diameter of 3 µm) was used.

Moreover, TLC-MS was measured by the following method. A measurement sample was dissolved in chloroform or tetrahydrofuran in any concentration, and a small amount of the obtained solution was applied on the surface of a TLC glass plate (manufactured by Merck KGaA, trade name: Silica gel 60 F₂₅₄) preliminarily cut into a size of about 5 cm in length and about 5 mm in width. This was measured using helium gas heated to 240° to 350°C by TLC-MS (manufactured by JEOL Ltd., trade name: JMS-T100TD).

Furthermore, NMR was measured by the following method. In about 0.5 mL of chloroform-d or tetrahydrofuran-d, 5 to 10 mg of measurement sample was dissolved, and NMR was measured using NMR (manufactured by Varian, Inc., trade name: "MERCURY 300").

<Synthesis Example 1> (Synthesis of Compound M-1)

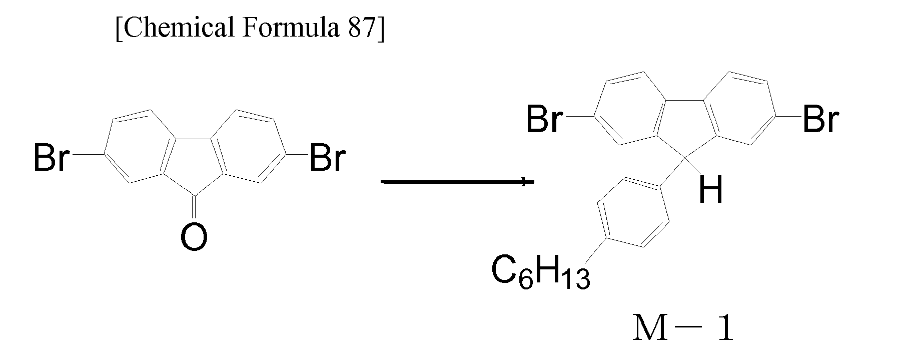

Under nitrogen gas atmosphere, 2,7-dibromofluorenone (75 g, 0.22 mol), hexylbenzene (334 ml, 1.78 mol), and trifluoromethanesulfonic acid (42 ml) were stirred at room temperature, and sodium mercaptosulfonate (8.1 g, 44 mmol) was then added thereto and stirred at 45°C for 9 hours. The obtained solution was cooled to room temperature and then poured into 1 L of hexane. Redundant hexylbenzene was removed by distillation under reduced pressure (105.5°C, 20 hPa), and the obtained residue was diluted with hexane and then poured into methanol, and the precipitated 2,7-dibromofluorenone was removed by filtration. The obtained filtrate was concentrated and then diluted with toluene, and isopropyl alcohol was added thereto, so as to precipitate a solid. The obtained solid was recrystallized with toluene/isopropyl alcohol, to obtain a compound M-1 in the form of a white crystal (53 g, yield: 49%).

¹H-NMR (300 MHz, CDCl₃) δ 0.88 (t, 3H), 1.20-1.45 (m, 6H), 1.54-1.62 (m, 2H), 2.57 (t, 2H), 4.96 (s, 1H), 6.94 (d, 2H), 7.10 (d, 2H), 7.42 (s, 2H), 7.48 (dd, 2H), 7.60 (d, 2H).

<Synthesis Example 2> (Synthesis of Compound M-2)

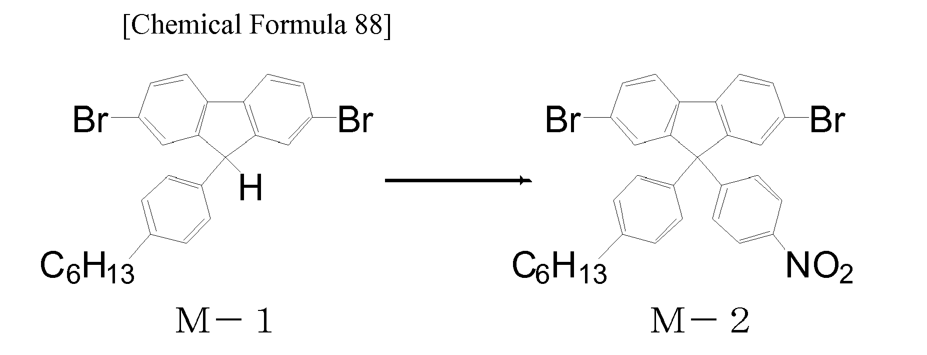

Under nitrogen gas atmosphere, the compound M-1 (10 g, 20.6 mmol), 4-fluoronitrobenzene (3.5 g, 24.8 mmol), and potassium carbonate (4.3 g, 31.0 mmol) were stirred in anhydrous N,N-dimethylformamide (35 ml) under heating to reflux for 6 hours. After cooling to room temperature, while stirring the obtained solution, 300 ml of water was slowly added thereto, and the solution was stirred overnight at room temperature. The precipitated solid was filtered out by filtration under reduced pressure, and the solid on a filter was further washed with water. The obtained solid was subjected to vacuum drying, to obtain a compound M-2 (13.6 g).

¹H-NMR (300 MHz, THF-d8) δ 0.91 (t, 3H), 1.24-1.42 (m, 6H), 1.55-1.61 (m, 2H), 2.59 (t, 2H), 7.07-7.16 (m, 4H), 7.43 (d, 2H), 7.59 (dd, 2H), 7.64 (s, 2H), 7.82 (d, 2H), 8.11 (d, 2H).

<Synthesis Example 3> (Synthesis of Compound M-3)

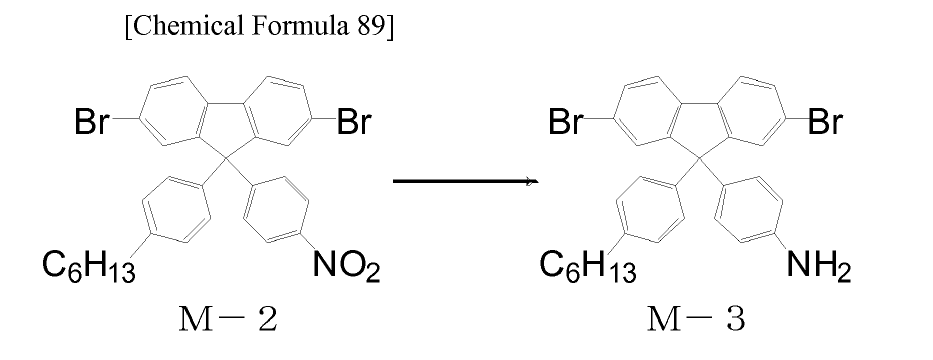

Under nitrogen gas atmosphere, a mixture of the compound M-2 (12.9 g, 21 mmol), ethanol (153 ml) and tin(II) chloride dihydrate (18.6 g, 8 mmol) was stirred under heating to reflux for 6 hours. After cooling to room temperature, the mixture was concentrated under reduced pressure until it became approximately 60 g. The obtained solution was added to ice water (150 g), while stirring. After the ice melted, a 40% by weight sodium hydroxide aqueous solution was added to the obtained aqueous solution, until the pH of the solution exceeded 10, and thereafter the mixture was extracted twice with 200 ml of toluene. The obtained organic layer was dried using anhydrous sodium sulfate and concentrated under reduced pressure, and then recrystallized with toluene-hexane solvent, to obtain a compound M-3 (10 g, yield: 97%).

¹H-NMR (300 MHz, CDCl₃) δ 0.87 (t, 3H), 1.20-1.40 (m, 6H), 1.52-1.57 (m, 2H), 2.54 (t, 2H), 6.54 (d, 2H), 6.91 (d, 2H), 7.02-7.06 (m, 4H), 7.42-7.48 (m, 4H), 7.54 (d, 2H).
LC-MS (APPI, positive) m/z⁺=574 [M+H]⁺.

<Synthesis Example 4> (Synthesis of Compound M-4)

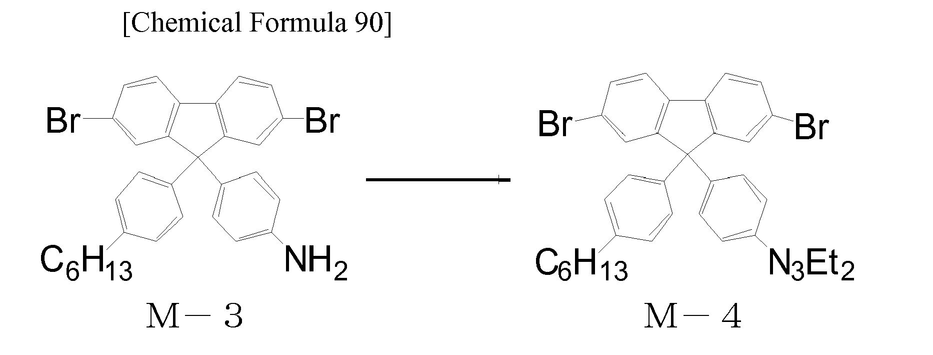

In a 3-L Erlenmeyer flask, 50 g (87 mmol) of the compound M-3 was charged, and 21.7 ml of concentrated hydrochloric acid was slowly added thereto while stirring with a stirring bar. Thereto, 100 ml of water was added, then 2 L of acetonitrile was added, to prepare a solution, and the solution was cooled to 0°C using an ice bath. Thereto, an aqueous solution obtained by dissolving 6.4 g (93 mmol) of sodium nitrate with 20 ml of water was slowly added, and the mixture was stirred at 0°C for 30 minutes (this is defined as "solution a.").

In another 3 L-Erlenmeyer flask, 18.4 g (133 mmol) of potassium carbonate and 12.8 g (174 mmol) of diethylamine were charged, and 128 ml of water was added thereto and stirred at 0°C (this is defined as "solution b.").

The solution a was slowly added to the solution b while stirring, and the mixture was stirred at 0°C for further 30 minutes, then the ice bath was removed, and the mixture was stirred at room temperature for 1 hour. The reaction solution was extracted with 3 L of chloroform and dried with anhydrous sodium sulfate, and then concentrated to remove chloroform by distillation. The obtained mixture was purified by a silica-gel column chromatography (silica gel 1 L, column diameter 6 cm × 60 cm, eluent: hexane:chloroform = 10:1 (volume ratio)), to obtain 51 g of an intended compound M-4 at a yield of 89%.

¹H-NMR (300 MHz, THF-d₈) δ 0.94 (t, J=6.57 Hz, 3H), 1.18-1.32 (m, 6H), 1.32-1.46 (m, 6H), 1.57-1.70 (m, 2H), 2.61 (m, 2H), 3.79 (q, J=7.14 Hz, 4H), 7.08-7.17 (m, 6H), 7.27-7.34 (m, 2H), 7.56 (dd, J=8.10 Hz and 1.74 Hz, 2H), 7.62 (d, J=1.74 Hz, 2H), 7.80 (d, J=8.13 Hz, 2H).

<Synthesis Example 5> (Synthesis of Compound M-5)

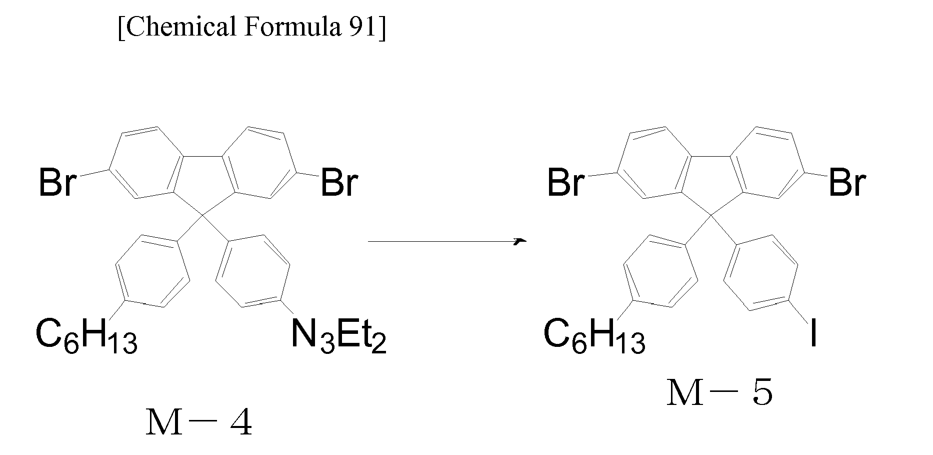

In a 1-L one-necked recovery flask, a stirring bar was charged, and 51 g (77 mmol) of the compound M-4, 39.2 g (154 mmol) of iodine and 500 ml (8 mol) of methyl iodide were added thereto, and argon gas was bubbled into the mixture while stirring for 15 minutes. The obtained solution was stirred for 6 hours under nitrogen atmosphere while heating in an oil bath at 90°C, and thereafter the solvent was distilled away. Thereto, 500 ml of chloroform was added, to make a solution, and the solution was filtered using a glass filter (diameter of 7.5 cm) covered with 250 ml of silica gel, and the filtrate was washed with 1 L of chloroform. The obtained chloroform solution was washed with an aqueous solution of saturated sodium thiosulfate and dried with anhydrous sodium sulfate, and then concentrated. The obtained mixture was purified by a silica-gel column chromatography (silica gel 1 L, column diameter 6 cm × 60 cm, eluent: hexane:chloroform =10:1 (volume ratio)) and further reprecipitated from a hexane-ethanol mixed solvent, to obtain 34.6 g of an intended compound M-5 at a yield of 67% in the form of a white solid.

¹H-NMR (300 MHz, CDCl₃) δ 0.89 (t, J=5.64 Hz, 3H), 1.20-1.40 (br, 6H), 1.58 (br, 2H), 2.56 (t, J=8.01 Hz, 2H), 6.89 (d, J=7.53 Hz, 1H), 6.98-7.10 (m, 4H), 7.13 (d, J=7.41 Hz, 1H), 7.22-7.52 (m, 1H), 7.43-7.52 (m, 4H), 7.54-7.62 (m, 3H).
LC-MS (APPI, positive) m/z⁺=684 [M-]⁺.

<Synthesis Example 6> (Synthesis of Compound M-6)

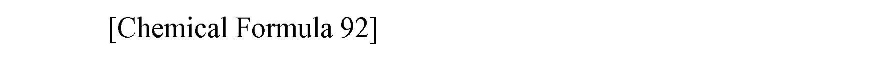

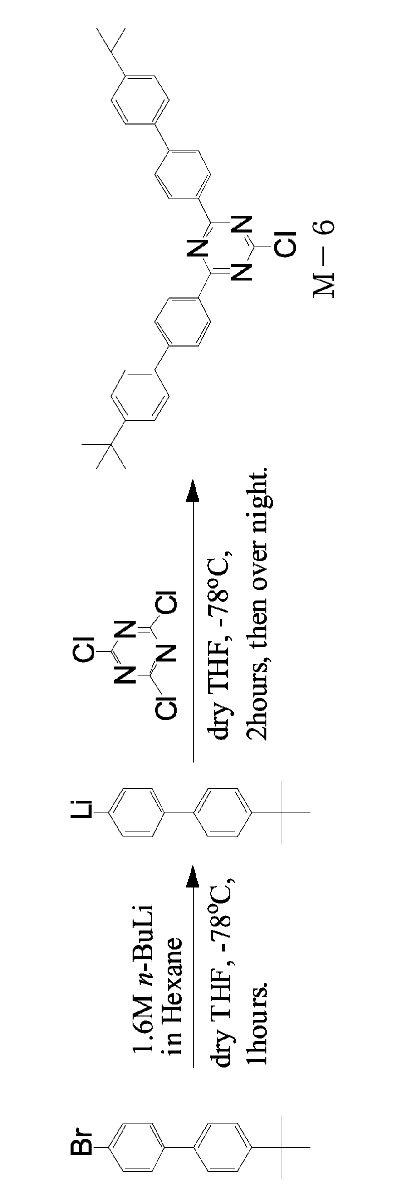

In a 2-L four-necked flask, 25 g (86 mmol) of 4-bromo-4'-butyl-1,1'-biphenyl was placed, and air inside the flask was substituted by argon. Thereto, 1 L of dehydrated tetrahydrofuran was added, and the mixture was cooled down to -78°C using dry ice-acetone as a cryogen while stirring. To the obtained solution, 54 ml (86.4 mmol) of 1.6 M n-butyllithium hexane solution was slowly dropped, and the stirring was continued for 1 hour while maintaining the temperature at -78°C, and the solution became a white slurry (this is referred to as "slurry sample").

In an another 2-L three-necked flask, 7.81 g (42.4 mmol) of cyanuric chloride was placed, and air inside was substituted by argon. Thereafter, 500 ml of dehydrated tetrahydrofuran was added, and the mixture was cooled down to -78°C using dry ice-acetone as a cryogen while stirring. To the solution, the above slurry sample was dropped using a cannula. At this time, the dropping rate was adjusted so that the temperature of the solution would not be -60°C or more. After the dropping, the mixture was stirred at -78°C for further 2 hours. Thereto, 400 mL of aqueous solution of saturated ammonium chloride was added to stop the reaction, and the mixture was heated to room temperature. Extraction and washing with about 5 L of hexane and about 7 L of ion-exchanged water were repeated to the reaction solution. When the obtained organic layer was dehydrated using anhydrous magnesium sulfate, and the solvent was removed by distillation under reduced pressure, a yellow tarry sample was obtained. When this sample was dispersed in 500 ml of acetonitrile and then filtered, a yellow powder was obtained. When this yellow powder was again dispersed in 500 ml of acetonitrile, heated under reflux at 80° to 90°C for 1 hour, and hot-filtered, an intended compound M-6 in the form of a pale yellow powder was obtained. The yield amount was 9.2 g (yield of 41 %).

¹H-NMR (300 MHz, THF-d₈):δ 1.39 (s, 18H), 7.52 (d, J=8.4 Hz, 4H), 7.68 (d, J=8.4 Hz, 4H), 7.83 (d, J=8.4 Hz, 4H), 8.69 (d, J=8.4 Hz, 4H).
LC-MS (APPI, positive) m/z⁺=532 [M]⁺.

<Synthesis Example 7> (Synthesis of Compound M-7)

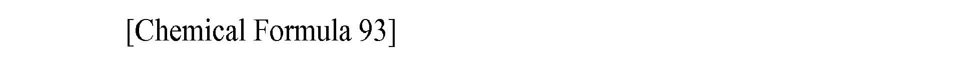

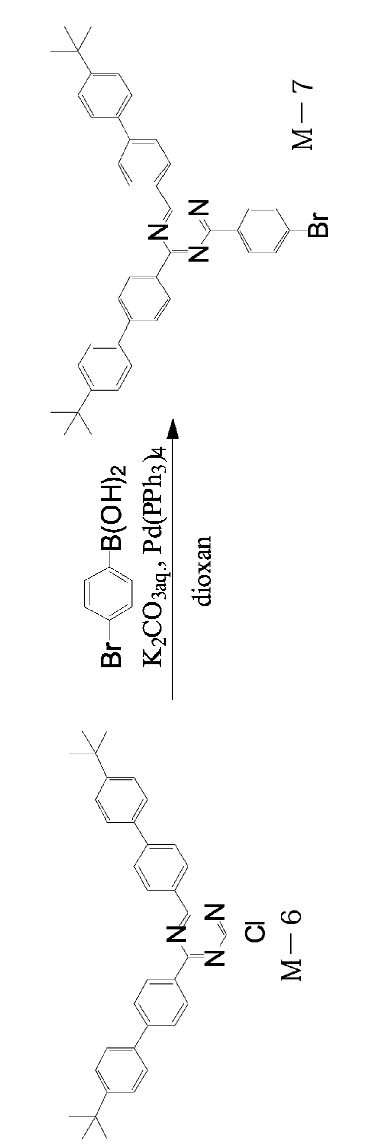

In a 300-mL four-necked recovery flask equipped with a 50-mL pressure equalizing dropping funnel and a 100-mL pressure equalizing dropping funnel, 5.0 g (8.9 mmol) of the compound M-6 and 110 ml of 1,4-dioxane were placed, and the mixture was bubbled with argon gas for 30 minutes. At this time, 3.7 g (27 mmol) of potassium carbonate dissolved in 30 mL of ion-exchanged water was added to the 50-mL pressure equalizing dropping funnel, and 1.7 g (8.5 mmol) of 4-bromophenylboronate dissolved in 50 ml of 1,4-dioxane was added to the 100-mL pressure equalizing dropping funnel, and each mixture was similarly bubbled with argon gas for 30 minutes. Thereafter, the obtained solution was heated up to 70°C while stirring, to completely dissolve the compound M-6 into the solvent. Thereto, 516 mg (0.45 mmol) of tetrakis(triphenylphosphine)palladium was added, and while the reaction solution was heated up to 110°C with stirring, each mixture was slowly dropped thereto from pressure equalizing dropping funnels. The mixture was reacted overnight at 110°C and then cooled down to room temperature, and toluene and ion-exchanged water were added thereto, to wash and extract the mixture. The obtained organic layer was dried using anhydrous magnesium sulfate, and the solvent was removed by distillation under reduced pressure. To the residue, 100 ml of chloroform was added, and the generated white precipitate was removed, and when the solvent was removed by distillation under reduced pressure, a yellow oily residue was obtained. Thereto, a small amount of n-hexane was added, and when the mixture was rubbed with a glass bar, a pale yellow powder was obtained, and thus this was filtered out and washed with n-hexane. When the obtained powder was purified by a medium-pressure preparative column chromatography (carrier: silica gel, eluent: chloroform-hexane =1:3 (volume ratio)), 2 g of an indented compound M-7 was obtained in the form of a white powder (yield: 36%).
TLC-MS (DART, positive) m/z⁺ = 652[M+H]⁺.

<Synthesis Example 8> (Synthesis of Compound M-8) 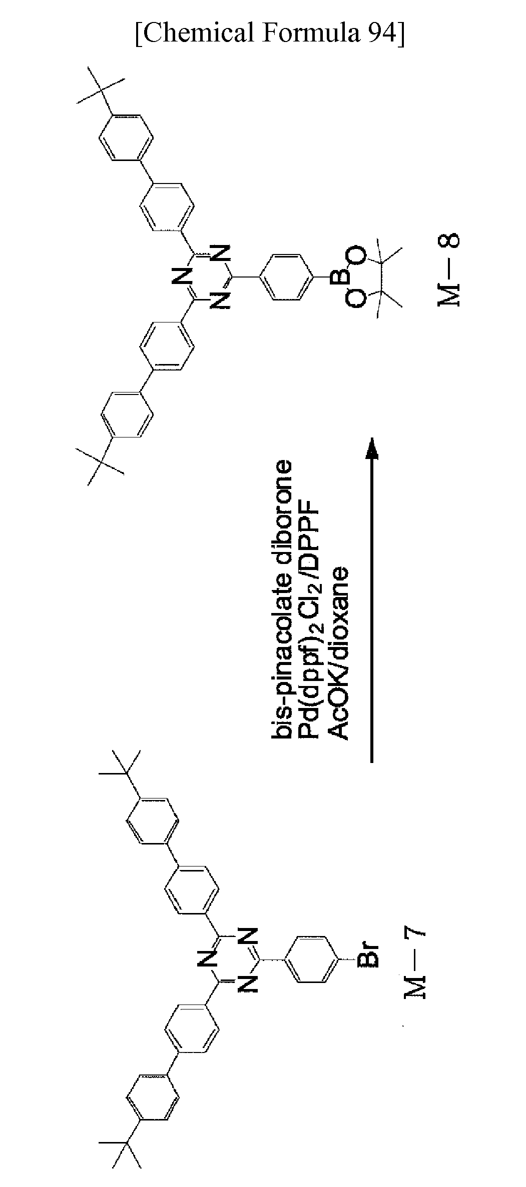 In a 200-mL two-necked round-bottom flask, a stirring bar was charged, and 2 g of the compound M-7 and 1.7 g of bispinacolatodiboran, 0.15 g of bis(diphenylphosphinoferrocene)dichloropalladium(II)dichloromethane complex, 0.10 g of diphenylphosphinoferrocene, and 1.8 g of potassium acetate were charged. Thereto, 26 ml of 1,4-dioxane preliminarily bubbled with argon was added, and thereafter the mixture was heated under reflux for 8 hours. After cooling to room temperature, the reaction solution was concentrated, and dioxane was distilled away. Thereto, 100 ml of toluene was added, and the solution was stirred at room temperature for 15 minutes and filtered using a glass filter (diameter of 5 cm) covered with 100 ml of silica gel, and the filtrate was washed with 200 ml of toluene. The obtained filtrate was concentrated, and 50 ml of hexane was added to the residue and heated under reflux, and 100 ml of ethanol was added thereto and stirred to reach room temperature. The generated crystal was filtered to collect it, to obtain 1.96 g of an intended compound M-8 at a yield of 91% in the form of a white solid.
TLC-MS (DART, positive) m/z⁺ = 700[M]⁺.

<Example 1> (Synthesis of Compound M-9)

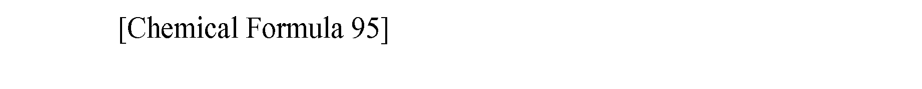

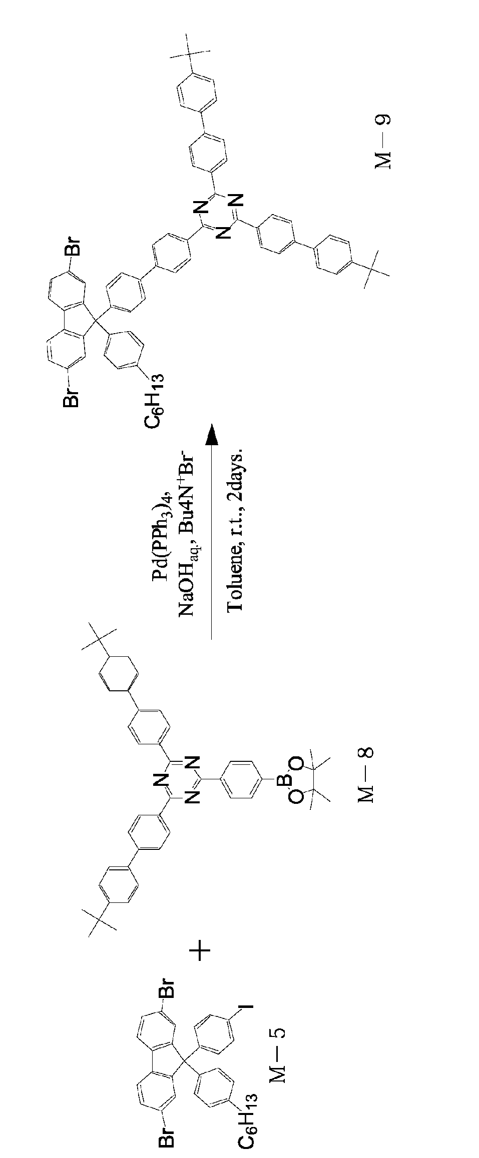

Under nitrogen gas atmosphere, 21.8 g of the compound M-5, 2.2 g of the compound M-8, 0.09 g of tetrakistriphenylphosphinepalladium(0) and a stirring bar were charged into a 100-ml reaction tube, and 14 ml of toluene was added thereto, and thereafter the mixture was bubbled with argon gas for 15 minutes. A 2 M sodium hydroxide aqueous solution was prepared and bubbled with argon gas for 15 minutes, and thereafter 9.4 ml thereof was charged into the 100-ml reaction tube, and 0.4 g of tetrabutylammonium bromide was added thereto and stirred at room temperature for 2 days. The reaction solution was extracted with toluene, and the extract was dried with anhydrous sodium sulfate and then filtered using a glass filter covered with 100 ml of silica gel, and the toluene solution was concentrated. The obtained mixture was purified by a silica-gel column chromatography (silica gel 1 L, column diameter 6 cm × 60 cm, eluent: hexane:chloroform =2:1 (volume ratio)) and reprecipitated by adding hexane, to obtain 1.82 g of an intended compound M-9 at a yield of 61.5% in the form of a white solid.

¹H-NMR (300 MHz, CDCl₃) δ 0.89 (t, J=6.6 Hz, 3H), 1.20-1.45 (m, 24H), 1.55-1.65 (m, 2H), 2.50-2.65 (m, 2H), 7.10 (s, 4H), 7.2-7.3 (m, 2H), 7.42-7.62 (m, 12H), 7.66 (d, J=8.34 Hz, 4H), 7.72-7.85 (m, 6H), 8.83 (d, J=8.18 Hz, 6H).
TLC-MS (DART, positive) m/z⁺=1132 [M]⁺.

<Synthesis Example 9> (Synthesis of Compound M-10)

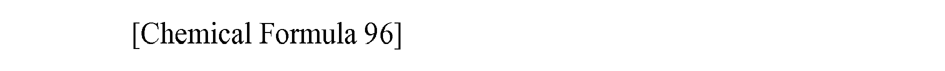

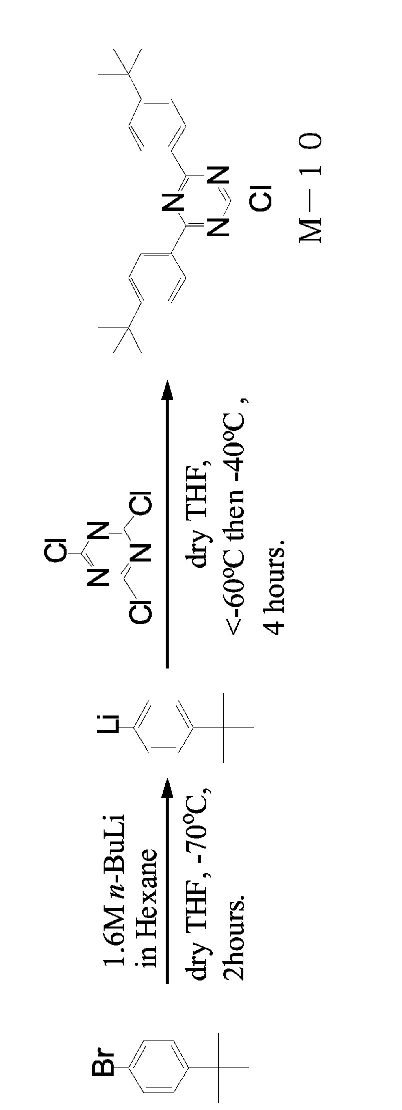

Under a flow of argon, 1-bromo-4-t-butylbenzene (125 g, 587 mmol) and tetrahydrofuran (470 ml) were charged in a reaction vessel and cooled to -70°C. Thereto, n-butyllithium/hexane solution (1.6 M, 367 mL, 587 mmol) was dropped over a period of 90 minutes at -70°C, and after the dropping, the mixture was stirred for 2 hours at -70°C, to obtain a 4-t-butylphenyllithium/tetrahydrofuran (THF) solution. Under a flow of argon, cyanuric chloride (50.8 g, 276 mmol) and tetrahydrofuran (463 mL) were charged into an another reaction vessel and cooled to -70°C. Thereinto, the 4-t-butylphenyllithium/THF solution preliminarily prepared was dropped while cooling so as to have a reaction temperature of -60°C or less. After the dropping, the reaction solution was stirred at -40°C for 4 hours and at room temperature for 4 hours. To the reaction mixture, 50 ml of water was added to terminate the reaction, and the solvent was removed by distillation under reduced pressure. To the residue, 1 L of water and 2 L of chloroform were added, and the organic layer was extracted. Furthermore, the organic layer was washed with 1 L of water, and thereafter the solvent was distilled away. The residue was dissolved in 600 ml of acetonitrile, and an insoluble solid was removed by hot filtration. Thereafter, the filtrate was concentrated to 100 ml or so and cooled to -70°C, and the precipitated solid was collected by filtration. The collected solid was dissolved into a mixed solvent of chloroform (200 mL)/hexane (600 mL), and purified by a silica-gel column chromatography (developing solvent: chloroform/hexane). The solvent was distilled away, and the residue was recrystallized from acetonitrile, to obtain a compound M-10 (41.3 g, 109 mmol).

¹H-NMR (300 MHz, CDCl₃) δ 1.39 (s, 18H), 7.56 (d, J=8.4 Hz, 4H), 8.54 (d, J=8.4 Hz, 4H).
LC-MS (APPI, positive) m/z⁺=380 [M+H]⁺.

<Synthesis Example 10> (Synthesis of Compound M-11)

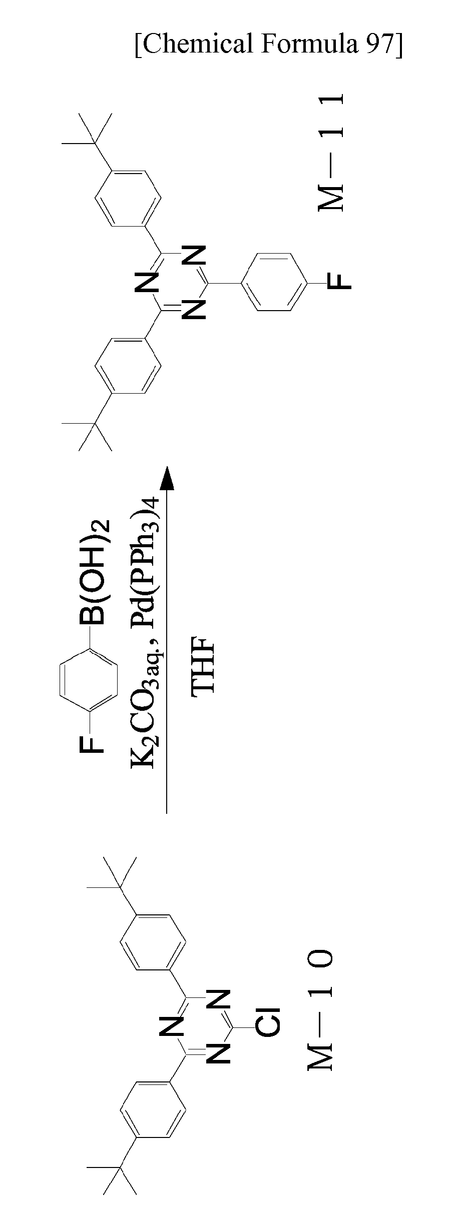

In a 300-mL recovery flask, 0.95 g (2.5 mmol) of the compound M-10 and 0.53 g (3.75 mmol) of 4-fluorophenylboronate were placed, and air inside the recovery flask was substituted by argon. Into the recovery flask, 75 ml of degassed toluene, 65.3 ml of a degassed 20% by weight potassium carbonate aqueous solution were charged, and argon was bubbled for further 10 minutes. Thereto, 14.4 mg (0.013 mmol) of tetrakistriphenylphosphinepalladium was added, and the mixture was heated under reflux for 5 hours. After cooling to room temperature, toluene was charged thereinto, and the mixture was washed twice with an aqueous solution of saturated ammonium chloride and twice with ion-exchanged water and separated. The obtained organic layer was dried using anhydrous sodium sulfate, and then filtered, and the filtrate was concentrated and dried, to obtain 1.12 g of a crude product. The crude product was purified by a silica-gel column (eluent: toluene:hexane =1:10 (volume ratio)), to obtain 0.99 g of a compound M-11 in the form of a white crystal.
LC-MS (APPI, positive) m/z⁺ = 440[M+H]⁺.

<Synthesis Example 11> (Synthesis of Compound M-12)

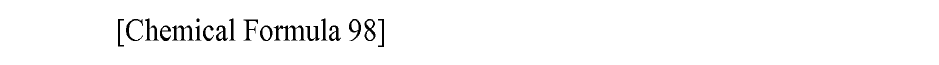

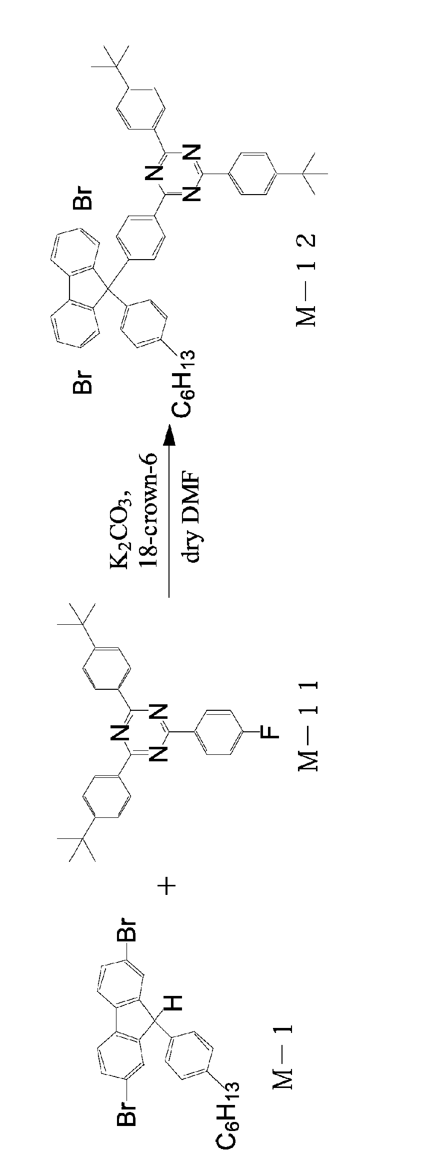

In a 300-mL recovery flask, 13.37 g (27.6 mmol) of the compound M-1, 10.92 g (24.84 mmol) of the compound M-11, 7.63 g (55.2 mmol) of potassium carbonate, and 7.3 g (27.6 mmol) of 18-crown-6-ether were placed, and air inside the recovery flask was substituted by argon. Into the recovery flask, 134 ml of dehydrated dimethylsulfoxide was charged, and heated under reflux for 69 hours. After cooling the reaction solution to room temperature, 200 ml of ion-exchanged water was added to the reaction solution, and this mixture was extracted with chloroform and washed with, in order of an aqueous solution of saturated ammonium chloride, a saturated saline, and ion-exchanged water. The obtained chloroform layer was dried with anhydrous sodium sulfate, and thereafter the solvent was removed by distillation under reduced pressure. When the obtained residue was purified by silica-gel column purification (eluent: toluene/hexane = 1/8 (volume ratio)), 10 g of a compound M-12 in the form of a white crystal was obtained.

¹H-NMR (300 MHz, CDCl₃) δ 0.89 (t, J=5.9 Hz, 3H), 1.35-1.25 (m, 6H), 1.39 (s, 18H), 1.66-1.55 (m, 2H), 1.66-1.55 (m, 2H), 2.58 (t, J=7.5 Hz, 2H), 7.10 (s, 4H), 7.35 (d, J=7.2 Hz, 2H), 7.63-7.49 (m, 10H), 8.64 (d, J=7.2 Hz, 6H).
LC-MS (APCI, positive) m/z⁺=902 [M+H]⁺.

<Synthesis Example 12> (Synthesis of Compound M-13)

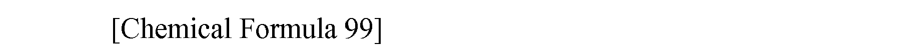

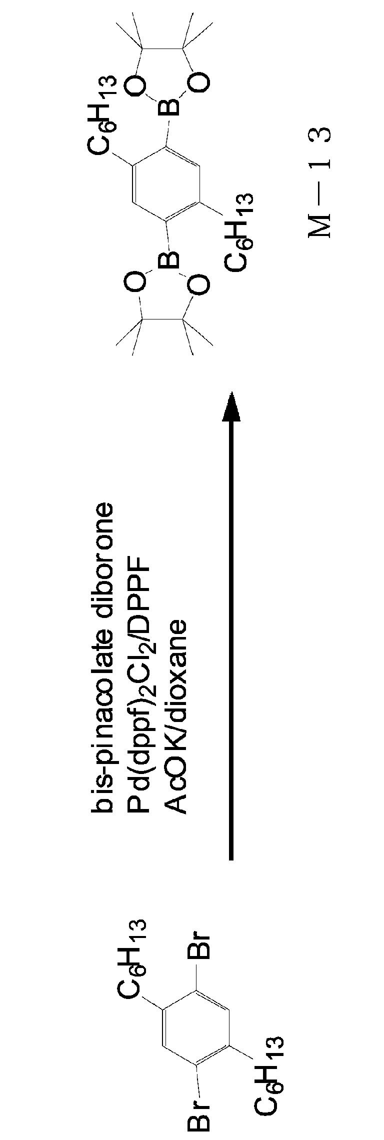

Into a 300-ml four-necked flask, 8.08 g (20 mmol) of 1,4-dihexyl-2,5-dibromobenzene, 12.19 g (48 mmol) of bis(pinacolato)diboron, and 11.78 g (120 mmol) of potassium acetate were charged, air inside the flask was substituted by argon. Thereinto, 100 ml of dehydrated 1,4-dioxane was charged, and the mixture was degassed with argon. Thereinto, 0.98 g (1.2 mmol) of [1,1'-bis(diphenylphosphino)ferrocene]dichloropalladium(II) was charged, and the mixture was further degassed with argon. The mixture was heated under reflux for 6 hours and turned into a dark brown slurry. Toluene and ion-exchanged water were added thereto, the mixture was separated, and the organic layer was washed with ion exchanged water. Anhydrous sodium sulfate and activated carbon were added thereto, and the mixture was filtered with a funnel precoated with celite. The filtrate was concentrated, to obtain 11.94 g of a dark brown crystal. This crystal was recrystallized with n-hexane, and the crystal was washed with methanol. The obtained crystal was dried under reduced pressure, to obtain 4.23 g of a compound M-13 in the form of a white needle-like crystal. Yield: 42%.

¹H-NMR (300 MHz, CDCl₃) δ 0.95 (t, 6H), 1.39-1.42 (bd, 36H), 1.62 (m, 4H), 2.88 (t, 4H), 7.59 (bd, 2H).
LC-MS (ESI, positive) m/z⁺=573 [M+K]⁺.

### <Example 2> (Synthesis of Polymer Compound P-1)

Under inert atmosphere, the compound M-13 (248 mg), 2,7-dibromo-9,9-dioctylfluorene (170 mg), the compound H-9 (226 mg), palladium(II) acetate (0.4 mg), tris(2-methoxyphenyl)phosphine (1.9 mg), and toluene (10 ml) were mixed, and heated to 105°C. To the reaction solution, a 10% by weight tetraethylammonium hydroxide aqueous solution (3.3 ml) was dropped, and the mixture was refluxed for 5 hours. After the reaction, phenylboronic acid (6.4 mg), palladium(II) acetate (0.3 mg), and tris(2-methoxyphenyl)phosphine (1.9 mg) were added thereto, and the solution was further refluxed for 17 hours. Subsequently, a 0.3 M sodium diethyldithiocarbamate aqueous solution (5 ml) was added, and the mixture was stirred for 2 hours at 80°C. After cooling to room temperature, toluene (16 ml) was added thereto, and the mixture was washed twice with water (7 ml), twice with a 3% by weight acetic acid aqueous solution (7 ml), and twice with water (7 ml), and purified by passing through an alumina column and a silica-gel column. The obtained toluene solution was dropped to methanol (80 ml), and the mixture was stirred for 1 hour, thereafter, the obtained precipitation was filtered out and dried. The yield of this precipitation (hereinafter, referred to as "polymer compound P-1") was 3 87 mg.

The polymer compound P-1 has a polystyrene-equivalent number average molecular weight of 1.1 × 10⁵ and a polystyrene-equivalent weight average molecular weight of 2.5 × 10⁵.

The polymer compound P-1 is a copolymer comprising a repeating unit represented by the following formula: 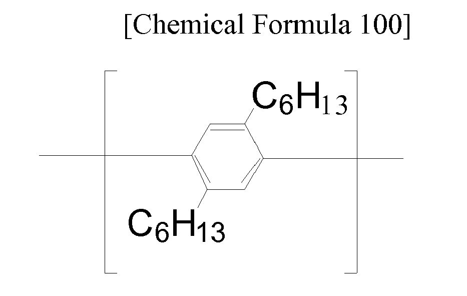 a repeating unit represented by the following formula: 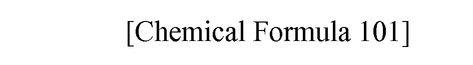 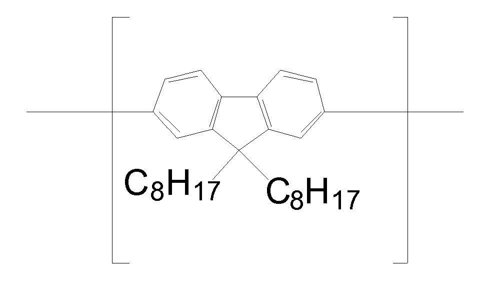 , and a repeating unit represented by the following formula: 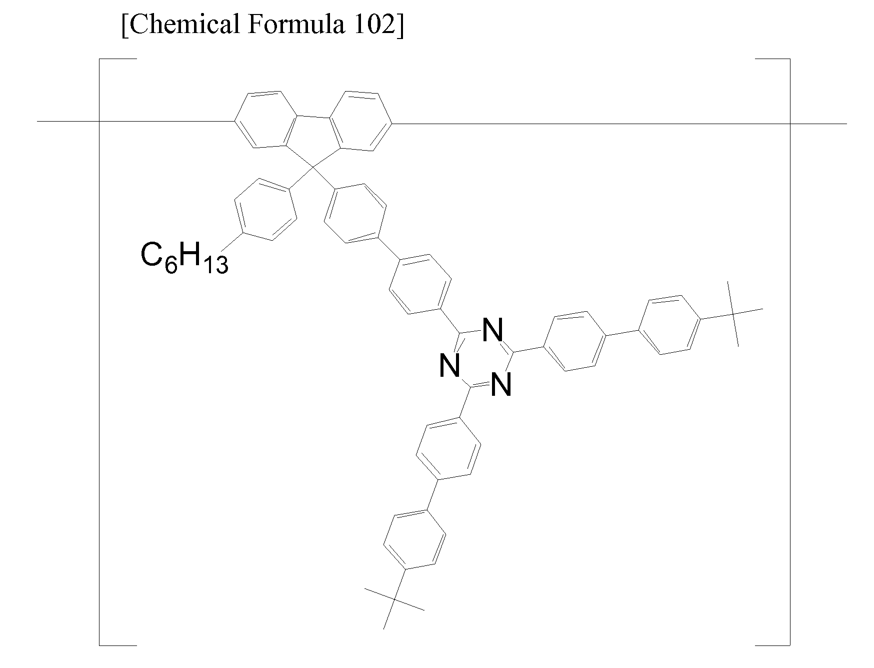 at a molar ratio of 50:30:20 in a theoretical value obtained from the charged raw materials.

### <Example 3> (Synthesis of Polymer Compound P-2)

Under inert atmosphere, the compound M-13 (987 mg), 2,7-dibromo-9,9-dioctylfluorene (679 mg), the compound M-12 (723 mg), palladium(II) acetate (0.8 mg), tris(2-methoxyphenyl)phosphine (4.3 mg), and toluene (20 ml) were mixed, and heated to 105°C. To the reaction solution, a 10% by weight tetraethylammonium hydroxide aqueous solution (6.6 ml) was dropped, and the mixture was refluxed for 5 hours. After the reaction, phenylboronic acid (244 mg), palladium(II) acetate (0.8 mg), and tris(2-methoxyphenyl)phosphine (4.4 mg) were added thereto, and the solution was further refluxed for 17 hours. Subsequently, a 0.3 M sodium diethyldithiocarbamate aqueous solution (12 ml) was added, and the mixture was stirred for 2 hours at 80°C. After cooling to room temperature, the mixture was washed twice with water (26 ml), twice with a 3% by weight acetic acid aqueous solution (26 ml), and twice with water (26 ml), and purified by passing through an alumina column and a silica-gel column. The obtained toluene solution was dropped to methanol (310 ml), and the mixture was stirred for 1 hour, thereafter, the obtained precipitation was filtered out and dried. The yield of this precipitation (hereinafter, referred to as "polymer compound P-2") was 1.19 g.

The polymer compound P-2 has a polystyrene-equivalent number average molecular weight of 3.1 × 10⁵ and a polystyrene-equivalent weight average molecular weight of 7.7 × 10⁵.

The polymer compound P-2 is a copolymer comprising a repeating unit represented by the following formula: 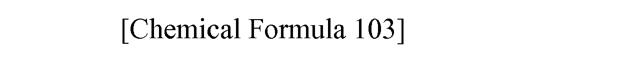 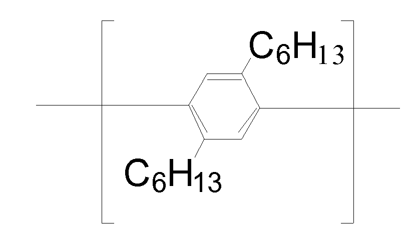 a repeating unit represented by the following formula: 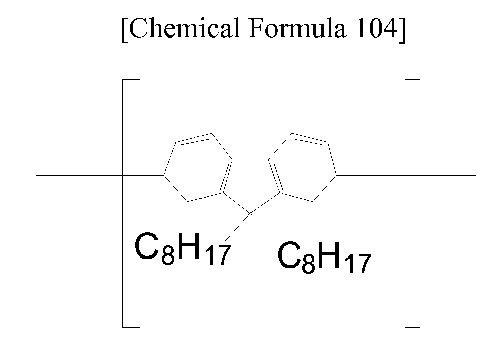 , and a repeating unit represented by the following formula: 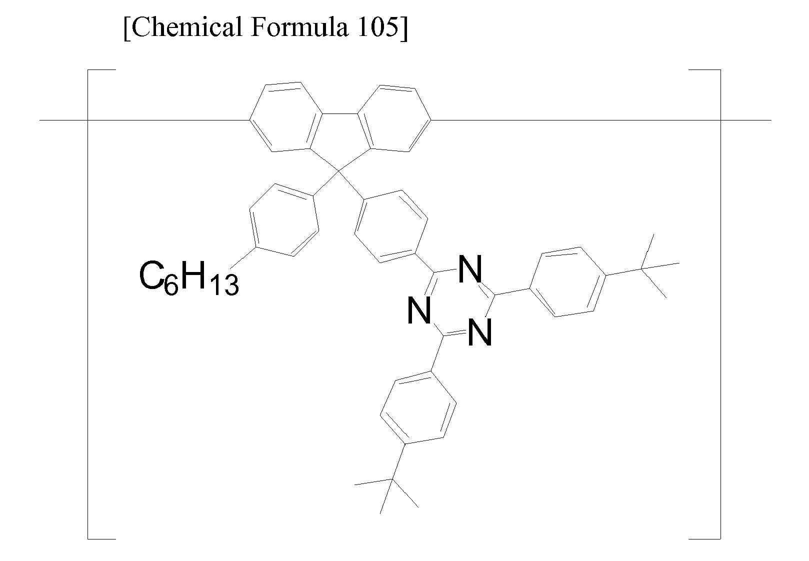 at a molar ratio of 50:30:20 in a theoretical value obtained from the charged raw materials.

### <Synthesis Example 12> (Synthesis of Polymer Compound P-3)

Under inert atmosphere, a compound M-14 (5.20 g) represented by the following formula: 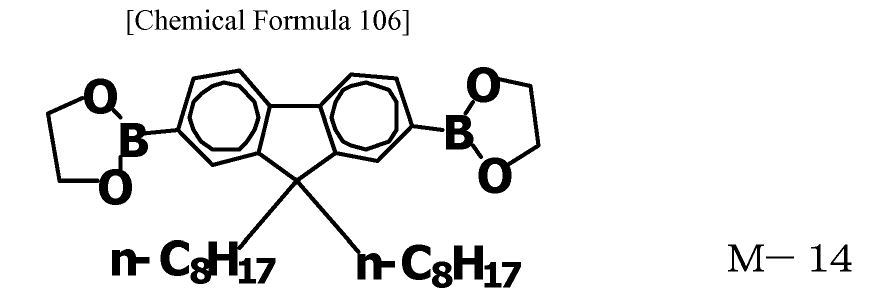 , a compound M-15 (5.42 g) represented by the following formula: 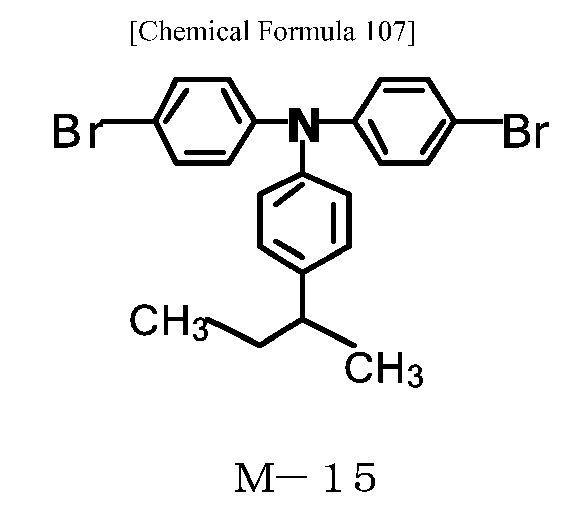 , palladium(II) acetate (2.2 mg), tris(2-methoxyphenyl)phosphine (15.1 mg), trioctylmethylammonium chloride (trade name: "Aliquat 336" (registered trademark), manufactured by Aldrich, 0.91 g) and toluene (70 ml) were mixed, and heated to 105°C. To the reaction solution, a 2 M sodium carbonate aqueous solution (19 ml) was dropped, and the mixture was refluxed for 4 hours. After the reaction, phenylboronic acid (121 mg) was added thereto, and the mixture was further refluxed for 3 hours. Subsequently, a sodium diethyldithiocarbamate aqueous solution was added, and the mixture was stirred for 2 hours at 80°C. After cooling, the obtained reaction solution was washed 3 times with water (60 ml), 4 times with a 3% by weight acetic acid aqueous solution (60 ml), and 3 times with water (60 ml), and the obtained toluene solution was purified by passing through an alumina column and a silica-gel column. The obtained toluene solution was dropped to methanol (3 L) and stirred, and thereafter the obtained precipitation was filtered out and dried. The yield of this precipitation (hereinafter, referred to as "polymer compound P-3 ") was 5.25 g.

The polymer compound P-3 has a polystyrene-equivalent number average molecular weight of 1.2 × 10⁵ and a polystyrene-equivalent weight average molecular weight of 2.6 × 10⁵.

The polymer compound P-3 is an alternating copolymer comprising a repeating unit represented by the following formula: 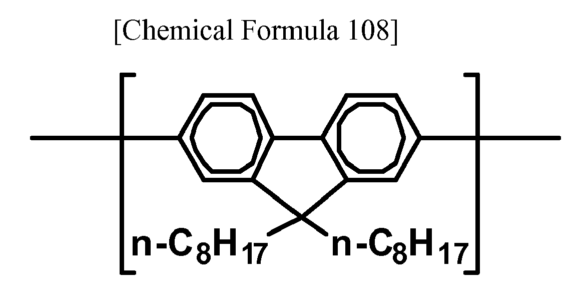 , and a repeating unit represented by the following formula: 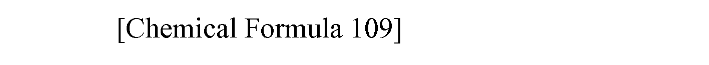 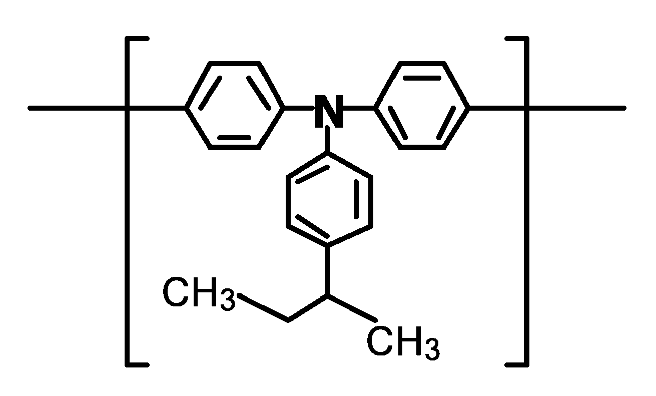 at a molar ratio of 50:50 in a theoretical value obtained from the charged raw materials.

<Comparative Example 1> (Synthesis of Polymer Compound P-4) Under inert atmosphere, the compound M-13 (3.13 g), 2,7-dibromo-9,9-dioctylfluorene (3.47 g), palladium(II) acetate (2.2 mg), tris(2-methoxyphenyl)phosphine (13.4 mg), and 80.0 mL of toluene were mixed, and heated to 100°C. To the reaction solution, a 20% by weight tetramethylammonium hydroxide aqueous solution (22.0 ml) was dropped, and the mixture was refluxed for 4.5 hours. After the reaction, phenylboronic acid (78 mg), palladium(II) acetate (2.2 mg), tris(2-methoxyphenyl)phosphine (13.4 mg), and a 20% by weight tetraethylammonium hydroxide aqueous solution (22.0 ml) were added thereto, and the mixture was further refluxed for 15 hours. Subsequently, a 0.2 M sodium diethyldithiocarbamate aqueous solution (70 ml) was added, and the mixture was stirred for 2 hours at 85°C. After cooling to room temperature, the solution was washed 3 times with water (82 ml), 3 times with a 3% by weight acetic acid aqueous solution (82 ml), and 3 times with water (82 ml), and purified by passing through an alumina column and a silica-gel column. The obtained toluene solution was dropped to methanol (1500 ml), and the obtained precipitation was filtered out and dried. The yield of this precipitation (hereinafter, referred to as "polymer compound P-4") was 3.52 g.
The polymer compound P-4 has a polystyrene-equivalent number average molecular weight of 3.1 × 10⁵ and a polystyrene-equivalent weight average molecular weight of 8.5 × 10⁵.

The polymer compound P-4 is an alternating copolymer comprising a repeating unit represented by the following formula: 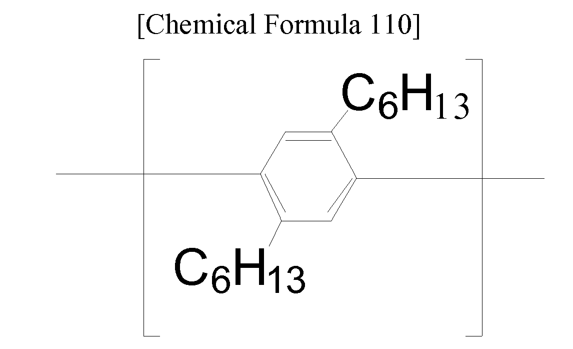 , and a repeating unit represented by the following formula: 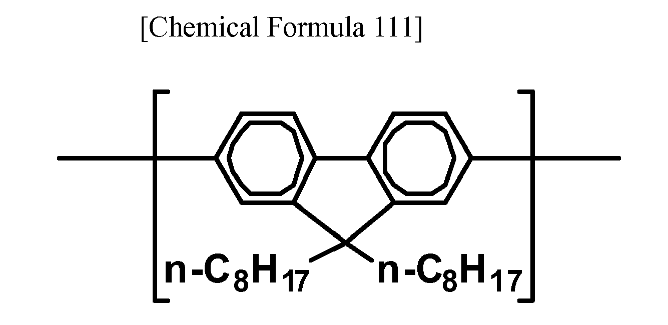 at a molar ratio of 50:50 in a theoretical value obtained from the charged raw materials.

### <Example 4> (Preparation of Polymer Solution S1)

The polymer compound P-1 and an iridium complex (manufactured by American Dye Source, Inc., trade name: ADS066GE, hereinafter, referred to as "ADS066GE".) were dissolved into xylene (manufactured by KANTO CHEMICAL CO., INC., electric industrial grade) so as to have a weight ratio of 95:5, to prepare a solution. At this time, the solution was prepared so that the total weight of the polymer compound P-1 and the iridium complex was 1.5% by weight based on the weight of the whole solution (hereinafter, the solution was referred to as "solution S1").

### <Example 5> (Preparation of Polymer Solution S2)

The polymer compound P-2 and an iridium complex (ADS066GE) were dissolved into xylene (manufactured by KANTO CHEMICAL CO., INC., electric industrial grade) so as to have a weight ratio of 95:5, to prepare a solution. At this time, the solution was prepared so that the total weight of the polymer compound P-2 and the iridium complex was 1.0% by weight based on the weight of the whole solution (hereinafter, the solution was referred to as "solution S2").

### <Preparation Example> (Preparation of Polymer Solution S3)

The polymer compound P-3 was dissolved into xylene (manufactured by KANTO CHEMICAL CO., INC., electric industrial grade), to prepare a solution. At this time, the solution was prepared so that the concentration of the polymer compound P-3 was 0.8% by weight based on the weight of the whole solution (hereinafter, the solution was referred to as "solution S3").

<Comparative Example 2> (Preparation of Polymer Solution S4) The polymer compound P-4 and an iridium complex (ADS066GE) were dissolved into xylene (manufactured by KANTO CHEMICAL CO., INC., electric industrial grade) so as to have a weight ratio of 95:5. At this time, the solution was prepared so that the total weight of the polymer compound P-4 and the iridium complex was 0.9% by weight based on the weight of the whole solution (hereinafter, the solution was referred to as "solution S4").

<Example 6> (Preparation of Polymer Light-Emitting Device P1) On a glass substrate with an ITO film having a thickness of 150 nm being attached thereon by a sputtering method, a film having a thickness of 65 nm was formed by spin-coating using a solution of poly(3,4-ethylenedioxythiophene)/polystyrenesulfonic acid (manufactured by Bayer AG, trade name: BaytronP AI4083), and dried on a hot plate at 200°C for 10 minutes. Subsequently, a film having a thickness of about 20 nm was formed by spin-coating using the polymer solution S3, and dried on a hot plate at 180°C for 60 minutes. Next, the film having a thickness of about 80 nm was formed by spin-coating using the polymer solution S1. This was dried under nitrogen gas atmosphere at 130°C for 10 minutes, and thereafter, as a cathode, barium was vapor-deposited with a film thickness of about 5 nm, and finally, aluminum was vapor-deposited with a film thickness of about 80 nm, so as to prepare a polymer light-emitting device P1. The device was composed of ITO/BaytronP (65 nm)/polymer compound P-3/mixture of polymer compound P-1 and iridium complex (ADS066GE)/Ba/Al. After the degree of vacuum reached 1 × 10⁻⁴ Pa or less, the vapor-deposition of the metals was started.

When voltage was applied to the polymer light-emitting device P1, electroluminescence (EL) was observed. This emission was green light emission having a peak wavelength derived from a film having the polymer compound P-1 at 515 nm. In the polymer light-emitting device P1, the maximum luminous efficiency was 6.4 cd/A, and the voltage at this time was 6.0 V, and the external quantum yield was 2.1%. The emission starting voltage was 3.4 V. The voltage at a luminance of 1000 cd/m² was 7.6 V, the chromaticity coordinate C.I.E. 1931 was (x, y) = (0.362, 0.562), and the luminous efficiency was 5.6 cd/A, and the external quantum yield was 1.8%.

<Example 7> (Preparation of Polymer Light-Emitting Device P2) On a glass substrate with an ITO film having a thickness of 150 nm being attached thereon by a sputtering method, a film having a thickness of 65 nm was formed by spin-coating using a solution of poly(3,4-ethylenedioxythiophene)/polystyrenesulfonic acid (manufactured by Bayer AG, trade name: BaytronP AI4083), and dried on a hot plate at 200°C for 10 minutes. Subsequently, a film having a thickness of about 20 nm was formed by spin-coating using the polymer solution S3, and dried on a hot plate at 180°C for 60 minutes. Next, the film having a thickness of about 80 nm was formed by spin-coating using the polymer solution S2. This was dried under nitrogen gas atmosphere at 130°C for 10 minutes, and thereafter, as a cathode, barium was vapor-deposited with a film thickness of about 5 nm, and finally, aluminum was vapor-deposited with a film thickness of about 80 nm, so as to prepare a polymer light-emitting device P2. The device was composed of ITO/BaytronP (65 nm)/polymer compound P-3/mixture of polymer compound P-2 and iridium complex (ADS066GE)/Ba/Al. After the degree of vacuum reached 1 × 10⁻⁴ Pa or less, the vapor-deposition of the metals was started.

When voltage was applied to the polymer light-emitting device P2, electroluminescence (EL) was observed. This emission was green light emission having a peak wavelength derived from a film having the polymer compound P-2 at 510 nm. In the polymer light-emitting device P2, the maximum luminous efficiency was 6.6 cd/A, and the voltage at this time was 6.6 V, and the external quantum yield was 2.1%. The emission starting voltage was 4.3 V. The voltage at a luminance of 1000 cd/m² was 9.4 V, the chromaticity coordinate C.I.E. 1931 was (x, y) = (0.327, 0.584), and the luminous efficiency was 5.3 cd/A, and the external quantum yield was 1.7%.

<Comparative Example 3> (Preparation of Polymer Light-Emitting Device P3) On a glass substrate with an ITO film having a thickness of 150 nm being attached thereon by a sputtering method, a film having a thickness of 65 nm was formed by spin-coating using a solution of poly(3,4-ethylenedioxythiophene)/polystyrenesulfonic acid (manufactured by H.C. Starck GmbH., trade name: CLEVIOUS P AI4083), and dried on a hot plate at 200°C for 10 minutes. Subsequently, a film having a thickness of about 20 nm was formed by spin-coating using the polymer solution S3, and dried on a hot plate at 180°C for 60 minutes. Next, the film having a thickness of about 80 nm was formed by spin-coating using the polymer solution S4. This was dried under nitrogen gas atmosphere at 130°C for 10 minutes, and thereafter, as a cathode, barium was vapor-deposited with a film thickness of about 5 nm, and finally, aluminum was vapor-deposited with a film thickness of about 80 nm, so as to prepare a polymer light-emitting device P3. The device was composed of ITO/CLEVIOUS P (65 nm)/polymer compound P-3/mixture of polymer compound P-4 and iridium complex (ADS066GE)/Ba/A1. After the degree of vacuum reached 1 × 10⁻⁴ Pa or less, the vapor-deposition of the metals was started.

When voltage was applied to the polymer light-emitting device P3, electroluminescence (EL) was observed. This emission was green light emission having a peak wavelength derived from a film having the polymer compound P-4 at 505 nm. In the polymer light-emitting device P3, the maximum luminous efficiency was 3.0 cd/A, and the voltage at this time was 14.6 V, and the external quantum yield was 0.9%. The emission starting voltage was 9.4 V. The voltage at a luminance of 1000 cd/m² was 16.6 V, the chromaticity coordinate C.I.E. 1931 was (x, y) = (0.302, 0.591), and the luminous efficiency was 2.6 cd/A, and the external quantum yield was 0.8%.

<Results> The polymer light-emitting devices P1 and P2 produced in Examples showed a lower voltage of each 6.0 V and 5.1 V in the emission starting voltage, as compared to the polymer light-emitting devices P3 produced in Comparative Example. It was shown from this result that the present invention is effective for lowering the voltage of the emission starting voltage of the polymer light-emitting device. In addition, in comparison of the polymer light-emitting devices P1 and P2 with the polymer light-emitting device P3, a lower voltage of each 9.0 V and 7.2 V even in the driving voltage at a luminance of 1000 cd/m² was showed.

### Industrial Applicability

An organic electroluminescent device produced using the polymer compound of the present invention has a lower emission starting voltage. Therefore, the polymer compound of the present invention can be suitably used as a material of a light-emitting layer of the organic electroluminescent device and is industrially very useful.

## Claims

1. A polymer compound comprising a repeating unit represented by formula (1-0): 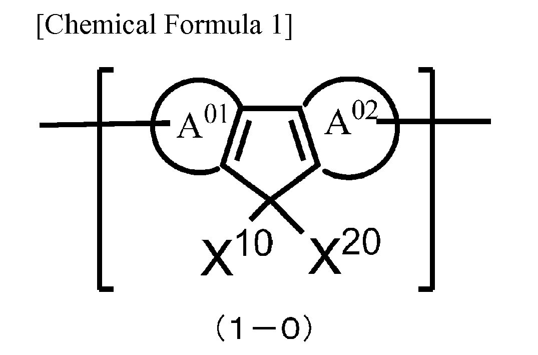 wherein in formula (1-0), ring A⁰¹ and ring A⁰² are the same or different and each represents an aromatic hydrocarbon ring that is optionally substituted; and X¹⁰ and X²⁰ are the same or different and each represents a hydrogen atom or a substituent, provided that at least one of X¹⁰ and X²⁰ is a group represented by formula (2-0): 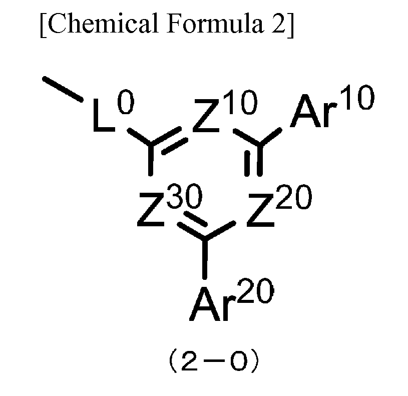 wherein in formula (2-0), Z¹⁰, Z²⁰ and Z³⁰ are the same or different and each represents -N= or -CH=, provided that at least two of Z¹⁰, Z²⁰ and Z³⁰ are -N=; L⁰ represents an arylene group or a single bond; and Ar¹⁰ and Ar²⁰ are the same or different and each represents an aryl group, provided that the total carbon number of the groups represented by Ar¹⁰, Ar²⁰ and L⁰ is 24 or more.

2. The polymer compound according to claim 1, wherein the repeating unit represented by formula (1-0) is a repeating unit represented by formula (1): 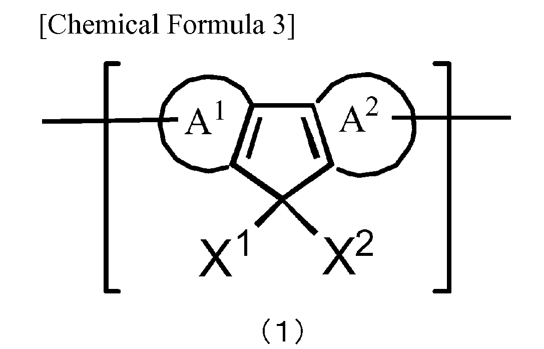 wherein in formula (1), ring A¹ and ring A² are the same or different and each represents an aromatic hydrocarbon ring that is optionally substituted; and X¹ and X² are the same or different and each represents a hydrogen atom or a substituent, provided that at least one of X¹ and X² is a group represented by formula (2): 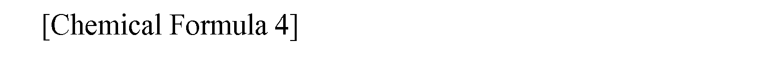 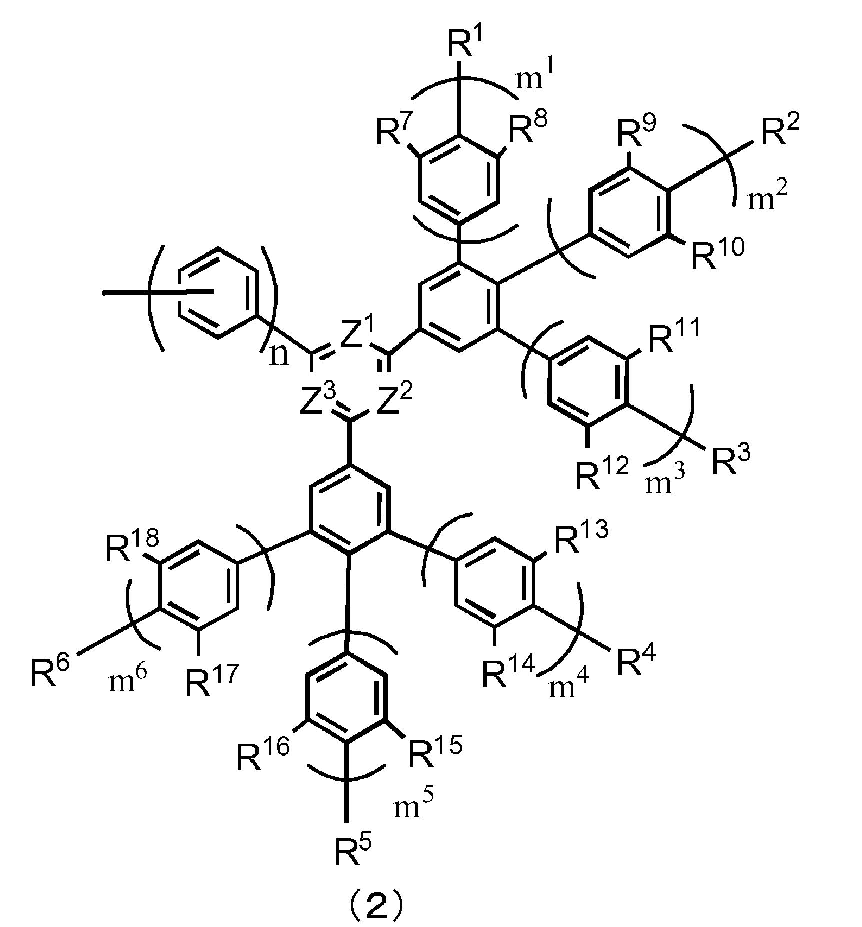 wherein in formula (2), Z¹, Z² and Z³ are the same or different and each represents -N= or -CH=, provided that at least two of Z¹, Z² and Z³ are -N=; n represents an integer of 0 or more; m¹ to m⁶ are the same or different and each represents an integer of 0 or more, provided that m¹ + m² + m³ ≥ 1 and m⁴ + m⁵ + m⁶ ≥ 1; R¹ to R⁶ are the same or different and each represents a hydrogen atom, a halogeno group, an alkyl group, an alkenyl group, an alkynyl group, an alkoxy group, an alkylthio group, an alkylsilyl group, or a group represented by formula (3); R⁷ to R¹⁸ are the same or different and each represents a hydrogen atom, a halogeno group, an alkyl group, an alkenyl group, an alkynyl group, an alkoxy group, an alkylthio group, an aryl group, an alkylsilyl group, or a group represented by formula (3); and when a plurality of each R⁷ to R¹⁸ exist, they may be the same or different: 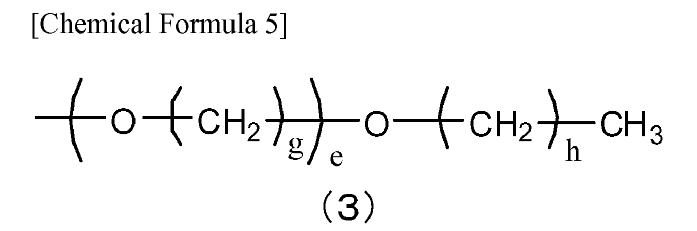 wherein in formula (3), e represents an integer of 1 to 6, g represents an integer of 1 to 6, and h represents an integer of 0 to 5; and when a plurality of g exist, they may be the same or different.

3. The polymer compound according to claim 2, wherein the ring A¹ and ring A² are each a benzene ring that is optionally substituted.

4. The polymer compound according to claim 2 or 3, wherein X¹ is a group represented by formula (2), and X² is an alkyl group, an aryl group, an alkenyl group, an alkynyl group, an alkoxy group, an alkylthio group, an alkylsilyl group, or a group represented by formula (3).

5. The polymer compound according to any one of claims 2 to 4, wherein Z¹, Z² and Z³ are each -N=.

6. The polymer compound according to any one of claims 2 to 5, wherein n is 1 or 2.

7. The polymer compound according to any one of claims 2 to 6, wherein m¹ to m⁶ are each 0 or 1.

8. The polymer compound according to any one of claims 2 to 7, wherein R¹ to R¹⁸ are each a hydrogen atom or an alkyl group.

9. The polymer compound according to any one of claims 2 to 8, wherein m¹, m³, m⁴ and m⁶ are each 0.

10. The polymer compound according to any one of claims 2 to 9, wherein the group represented by formula (2) is a group represented by formula (4): 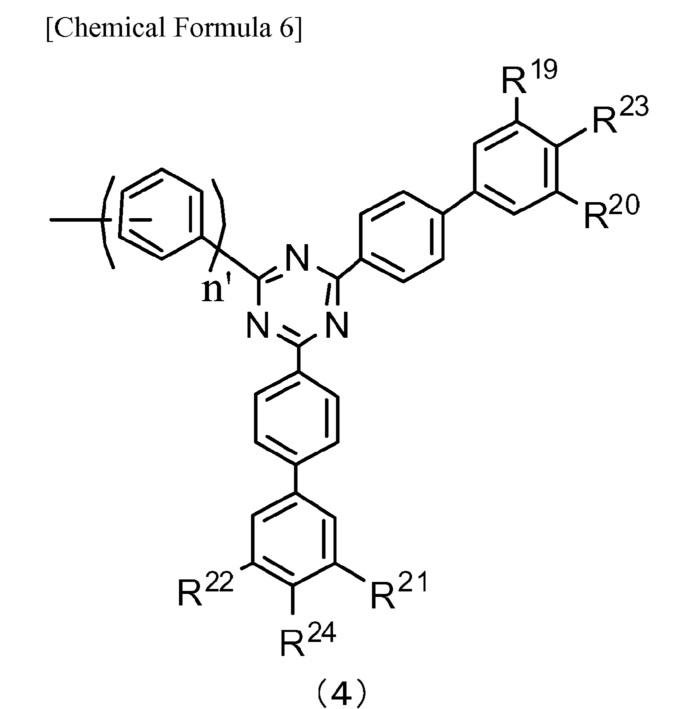 wherein in formula (4), n' represents 1 or 2; and R¹⁹ to R²⁴ are the same or different and each represents a hydrogen atom, an alkyl group, an alkenyl group, an alkoxy group, or a group represented by formula (3).

11. The polymer compound according to claim 10, wherein n' is 2, R¹⁹ to R²² each are a hydrogen atom, and R²³ and R²⁴ are the same or different and are each an alkyl group, an alkenyl group, an alkoxy group, or a group represented by formula (3).

12. The polymer compound according to any one of claims 1 to 11, further comprising a repeating unit represented by formula (5):
-Ar- (5)
wherein in formula (5), Ar represents an arylene group, a divalent heterocyclic group or a divalent aromatic amine residue.

13. The polymer compound according to any one of claims 1 to 12, having a polystyrene-equivalent number average molecular weight of 1 × 10³ to 1 × 10⁸.

14. A compound represented by formula (6): 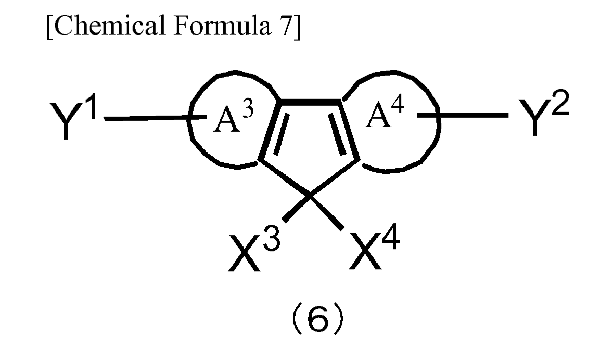 wherein in formula (6), ring A³ and ring A⁴ are the same or different and each represents an aromatic hydrocarbon ring that is optionally substituted; Y¹ and Y² are the same or different and each represents a hydrogen atom or a polymerizable reactive group; and X³ and X⁴ are the same or different and each represents a hydrogen atom or a substituent, provided that at least one of X³ and X⁴ is a group represented by formula (7): 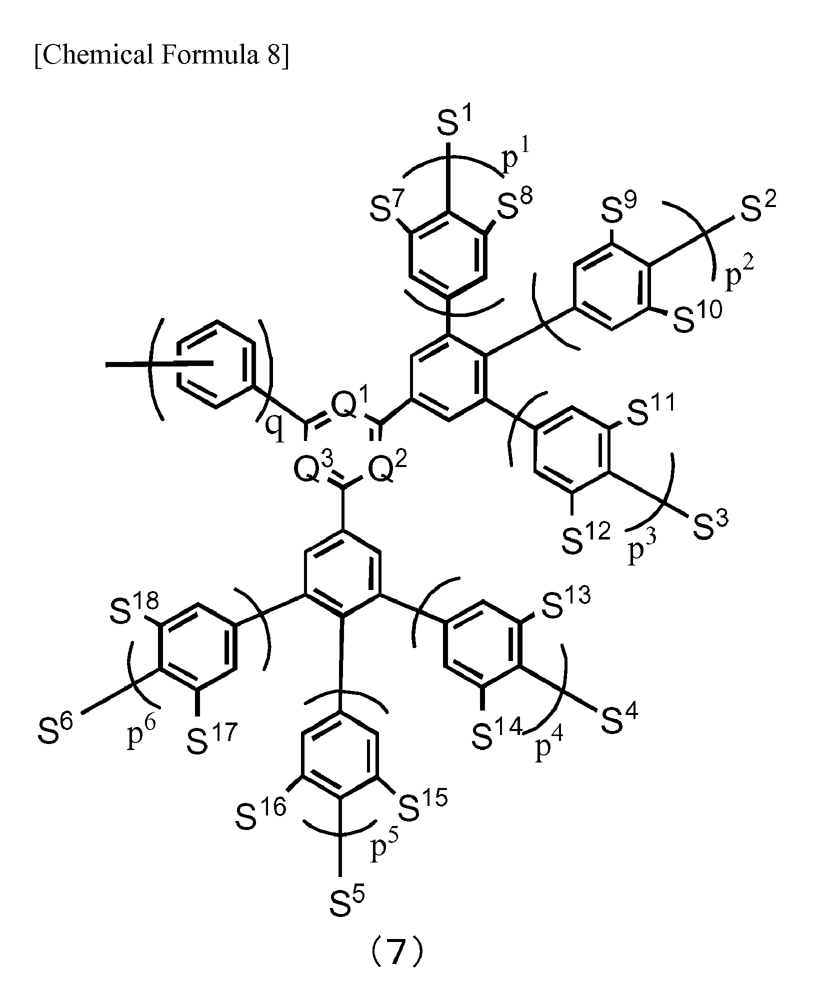 wherein in formula (7), Q¹, Q² and Q³ are the same or different and each represents -N= or -CH=, provided that at least two of Q¹, Q² and Q³ are -N=; q represents an integer of 0 or more; p¹ to p⁶ are the same or different and each represents an integer of 0 or more, provided that p¹ + p² + p³ ≥ 1 and p⁴ + p⁵ + p⁶ ≥ 1; S¹ to S⁶ are the same or different and each represents a hydrogen atom, a halogeno group, an alkyl group, an alkenyl group, an alkynyl group, an alkoxy group, an alkylthio group, an alkylsilyl group, or a group represented by formula (8); S⁷ to S¹⁸ are the same or different and each represents a hydrogen atom, a halogeno group, an alkyl group, an alkenyl group, an alkynyl group, an alkoxy group, an alkylthio group, an aryl group, an alkylsilyl group, or a group represented by formula (8); and when a plurality of each S⁷ to S¹⁸ exist, they may be the same or different: 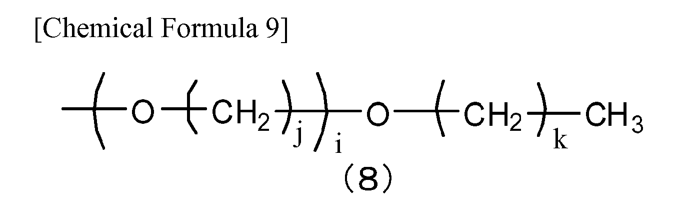 wherein in formula (8), i represents an integer of 1 to 6, j represents an integer of 1 to 6, and k represents an integer of 0 to 5; and when a plurality of j exist, they may be the same or different.

15. A composition comprising at least one material selected from the group consisting of a hole transport material, an electron transport material, and a light-emitting material, and the polymer compound according to any one of claims 1 to 13.

16. A solution comprising the polymer compound according to any one of claims 1 to 13 and a solvent.

17. A solution comprising the composition according to claim 15 and a solvent.

18. A thin film comprising the polymer compound according to any one of claims 1 to 13.

19. A thin film comprising the composition according to claim 15.

20. A polymer light-emitting device having an organic layer between electrodes comprising an anode and a cathode, the organic layer including the polymer compound according to any one of claims 1 to 13 or the composition according to claim 15.

21. The polymer light-emitting device according to claim 20, wherein the organic layer is a light-emitting layer.

22. A polymer light-emitting device having an organic layer, comprising a light-emitting layer and a charge transport layer located between electrodes comprising an anode and a cathode, the charge transport layer including the polymer compound according to any one of claims 1 to 13 or the composition according to claim 15.
